(19) Europäisches Patentamt
European Patent Office
Office européen des brevets

(11) EP 0 729 455 B1

(12) **FASCICULE DE BREVET EUROPEEN**

(45) Date de publication et mention de la délivrance du brevet:
**08.09.2004 Bulletin 2004/37**

(21) Numéro de dépôt: **95901501.7**

(22) Date de dépôt: **17.11.1994**

(51) Int Cl.$^7$: **C07D 209/16**, A61K 31/40, C07D 409/12, C07D 409/14, C07D 413/12

(86) Numéro de dépôt international:
**PCT/FR1994/001343**

(87) Numéro de publication internationale:
**WO 1995/014004 (26.05.1995 Gazette 1995/22)**

(54) **ARYLPIPERAZINES DERIVEES D'INDOLE COMME LIGANDS POUR LES RECEPTEURS 5 HT1-LIKE 5 HT1B ET 5 HT1D**

ARYLPIPERAZINDERIVATE VON INDOL ALS LIGANDEN DER REZEPTOREN 5 HT1-LIKE, 5 HT1B UND 5 HT1D

INDOLE-DERIVED AZYLPIPERAZINES AS LIGANDS FOR 5HT1-LIKE RECEPTORS 5HT1B AND 5HT1D

(84) Etats contractants désignés:
**AT BE CH DE DK ES FR GB GR IE IT LI LU MC NL PT SE**

(30) Priorité: **19.11.1993 FR 9313875**

(43) Date de publication de la demande:
**04.09.1996 Bulletin 1996/36**

(73) Titulaire: **PIERRE FABRE MEDICAMENT**
**92100 Boulogne (FR)**

(72) Inventeurs:
- **HALAZY, Serge**
  **F-81090 Lagarrigue (FR)**
- **PEREZ, Michel**
  **F-81100 Castres (FR)**
- **BRILEY, Michael**
  **F-81650 Gaillac (FR)**
- **PAUWELS, Peter**
  **F-81440 Lautrec (FR)**

(74) Mandataire: **Ahner, Francis et al**
**Cabinet Régimbeau**
**20, rue de Chazelles**
**75847 Paris cedex 17 (FR)**

(56) Documents cités:
**EP-A- 0 500 086**
**WO-A-94/02460**
**WO-A-94/15916**
**GB-A- 2 191 488**

Remarques:
Le dossier contient des informations techniques présentées postérieurement au dépôt de la demande et ne figurant pas dans le présent fascicule.

EP 0 729 455 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention se rapporte à de nouvelles arylpipérazines dérivées d'indole, à des procédés pour leur préparation et à leurs utilisations thérapeutiques.

Les composés selon la présente invention sont des ligands ayant une très haute affinité et une très bonne sélectivité pour les récepteurs communément appelés $5HT_1$-like et plus particulièrement pour les récepteurs appelés $5HT_{1B}$ et $5HT_{1D}$, selon la nouvelle nomenclature récemment proposée par P. Humphrey, P. Hartig et D. Hoyer (TiPS, 14, 233-236, 1993).

Les médicaments incluant (seuls ou en association avec d'autres agents thérapeutiques), les principes actifs de la présente invention trouvent leur emploi dans le traitement tant curatif que préventif des maladies liées au dysfonctionnement des récepteurs $5HT_{1\text{-like}}$ incluant les récepteurs $5HT_{1B}$, $5HT_{1D\alpha}$ et $5HT_{1D\beta}$, à leur dérégulation ou à des modifications de l'activité du ligand endogène (généralement la sérotonine).

Il a d'ailleurs été démontré que la sérotonine pouvait jouer un rôle dans certaines maladies telles que la dépression, la douleur, les désordres convulsifs obsessionnels, les attaques de panique, l'obésité, la schizophrénie; l'anxiété, certains dysfonctionnements sexuels ou encore certaines formes de dégénérescence telles que la maladie de Parkinson ou d'Alzheimer [se référer par exemple à : S. Langer, N. Brunello, G. Racagni, J. Mendlecvicz, "Serotonin receptors subtypes : pharmacological significance and clinical implications" Karger ed. (1992) ; B.E. Leonard, Int. Clin. Psychopharmacology, 7, 13-21 (1992); D.G. Grahame-Smith, Int. Clin. Psychopharmacology, 6, Suppl. 4, 6-13 (1992); E. Zifa, G. Fillion, Pharmacological Reviews, 44, 401-458 (1992) ; R.W. Fuller, J. Clin. Psychiatry, 53, 36-45 (1992)].

Les composés de la présente invention sont des ligands puissants et sélectifs des récepteurs $5HT_{1\text{-like}}$ qui peuvent agir comme agonistes, agonistes partiels ou antagonistes au niveau de ces récepteurs, et peuvent donc trouver une application dans les désordres liés à la sérotonine mentionnés ci-dessus.

La plupart des composés de la présente invention sont en outre des agonistes puissants (tant au niveau de leur affinité qu'au niveau de leur efficacité ou activité intrinsèque) et sélectifs des récepteurs $5HT_{1B}$ et $5HT_{1D}$. Les agonistes des récepteurs $5HT_{1\text{-like}}$ et plus particulièrement des récepteurs $5HT_{1D}$ présentent une activité vasoconstrictrice sélective et trouvent leur utilisation dans le traitement de la migraine et des désordres vasospastiques [(voir par exemple A. Doenicke et al., The Lancet, 1, 1309-1311 (1988) ; M.D. Ferrari, P.R. Saxena, Cephalalgia, 13, 151-165 (1993) ; S.J. Peroutka, Headache, 30, 5-11 (1990) ; M.A. Moskowitz, TiPS, 13, 307-311 (1992) ; W. Feniuk, P.P. Humphrey, M.S. Perren, H.E. Connor, E.T. Whalley, J. Neurol. 238, S57-S61 (1991) ; A.V. Deligonis, S.J. Peroutka, Headache, 31, 228-231 (1991)].

Les composés de la présente invention, qui sont, pour la plupart, des agonistes puissants et sélectifs des récepteurs $5HT_{1\text{-like}}$, trouvent donc plus particulièrement leur emploi dans le traitement curatif et prophylactique des crises de migraine "classique" (avec aura), "commune" (sans aura), l'algie vasculaire de la face, les céphalées chroniques vasculaires et des désordres vasospastiques.

L'état antérieur de la technique dans ce domaine est illustré notamment par:

- les demandes de brevets français F 9215919 (30/12/92) et F 9307982 (30/6/93) qui décrivent de nouveaux composés indoliques dérivés respectivement de pipérazines et d'arylamines comme ligands des récepteurs $5HT_{1B}$ - $5HT_{1D}$.
- la demande de brevet d'invention FR 2671971 qui décrit des dérivés 5-O-carboxyméthylés de la tryptamine qui ont une bonne affinité pour les récepteurs $5HT_{1D}$.
- les demandes de brevet européen 0 313 397, 0 486 666, 0 494 774-A1, 0 497 512-A2, 0 501 568-A1, 0 464 558, 0 548 813-A1 et la demande de brevet WO 92/13856 qui décrivent des dérivés hétérocycliques dérivés de tryptamine comme agonistes des récepteurs $5HT_{1\text{-like}}$.
- les demandes de brevet européen 0 533 266, 0 533 267 et 0 533 268 qui revendiquent des benzamides dérivées d'arylpipérazine comme antagonistes du récepteur $5HT_{1D}$.

Néanmoins, ces demandes de brevet, en aucun cas, ne décrivent ni ne suggèrent les dérivés de pipérazine indolique faisant partie de la présente invention : la présente invention décrit une nouvelle classe d'arylpipérazines dérivées d'indole qui se distingue des dérivés les plus proches de l'art antérieur (et en particulier de la demande de brevet français FR 9 215 919 déposée le 30/12/92) non seulement par leur structure chimique originale et différente, mais aussi par leur profil biologique et leur potentiel thérapeutique, puisque de nombreux composés selon la présente invention présentent l'avantage de conjuguer pour la première fois dans une même molécule une affinité très forte pour les récepteurs $5HT_{1B}$ ou $5HT_{1D}$, une sélectivité importante par rapport aux récepteurs $5HT_{1A}$ et une efficacité agoniste (activité intrinsèque) remarquable.

La présente invention concerne des composés de formule I :

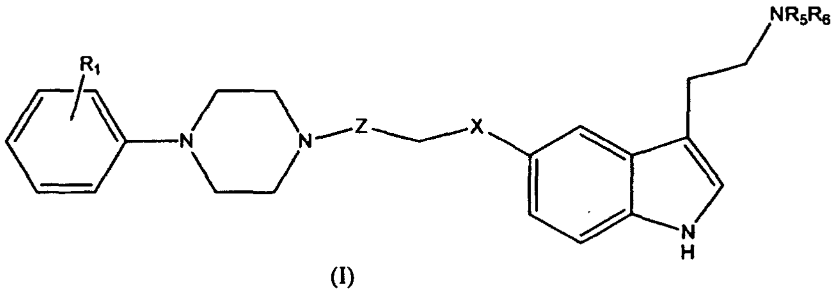

(I)

leur préparation et les médicaments les contenant.

Dans la formule (I),

$R_1$ représente un radical $NH_2$, $NO_2$, $NR_5R_6$, $NHSO_2R_7$ ou CN, pouvant être en position o, m ou p sur le cycle aromatique

Z représente C=O, $(CH_2)_n$ ou encore $CO(CH_2)_n$ ; n étant compris entre 1 et 5

X représente $CH_2$ ou O

$R_5$ et $R_6$ identiques ou différents représentent chacun un atome d'hydrogène, un radical alkyle linéaire ou ramifié, comprenant de 1 à 6 atomes de carbone, de préférence un méthyle, éthyle, n-propyle, isopropyle, n-butyle, s-butyle, ou t-butyle

$R_7$ représente un radical alkyle linéaire ou ramifié comprenant de 1 à 5 atomes de carbone, ou un hétérocycle à 5 atomes contenant un ou plusieurs atome(s) de soufre

ou $R_7$ représente $-NMe_2$ quand $R_5$ et $R_6$ représentent un atome d'hydrogène et Z représente C=O,

leurs sels et solvats acceptables pour l'usage thérapeutique.

Dans les définitions qui précèdent et celles qui seront citées ci-après, sauf mention contraire, les radicaux alkyle, alcoxy, ou alkylthio contiennent 1 à 6 atomes de carbone en chaine droite ou ramifiée et les portions cycloalkyle contiennent 3 à 7 atomes de carbone. Dans la formule (I), les atomes d'halogène sont préférentiellement les atomes de chlore, de fluor ou de brome.

Les composés de formule (I) contenant 1 ou plusieurs centres asymétriques présentent des formes isomères.

Les racémiques et les énantiomères purs de ces composés font également partie de cette invention.

L'invention comprend également les sels et les solvats de ces composés acceptables pour l'usage thérapeutique.

Parmi les sels acceptables pour l'usage thérapeutique des indoles de formule générale (I), on citera des sels formés par addition avec des acides organiques ou minéraux et par exemple les chlorhydrates, les bromhydrates, les sulfates, les fumarates et les maléates. D'autres sels peuvent être utiles dans la préparation des composés de formule (I), par exemple les adduits avec le sulfate de créatinine.

L'invention comprend également la préparation par les procédés décrits ci-dessous des composés de formule générale (I) et de leurs sels ou solvats (tels que les hydrates) acceptables pour l'usage thérapeutique.

D'une manière générale, les composés de formule générale (I) dans lesquels $R_6$ représente un hydrogène sont préparés à partir de dérivés de formule (II).

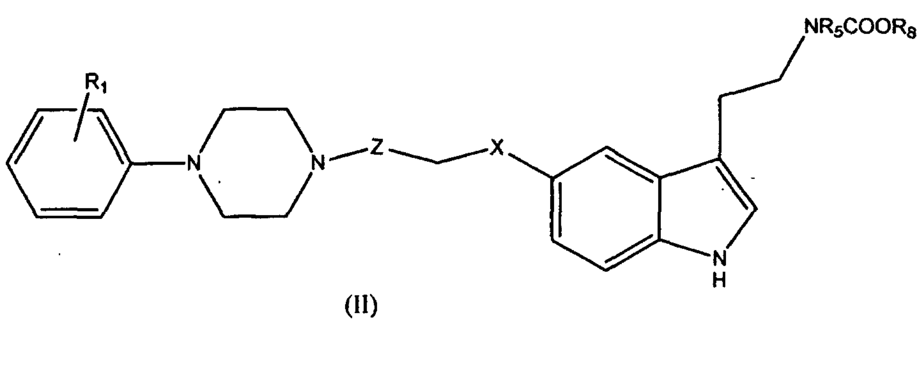

(II)

dans laquelle $R_1$, Z, X et $R_5$ sont décrits comme précédemment, $R_8$ est un radical t-butyle ou benzyle. La transformation des composés de formule (II) dans lesquels $COOR_8$ est un groupe $CO_2{}^tBu$ (BOC) en composés de formule (I) dans lesquels $R_6$ est un hydrogène, est préférentiellement effectuée à l'aide d'un acide tel que l'acide trifluoroacétique ou

l'acide chlorhydrique dans un solvant organique tel que l'éther, le tétrahydrofuranne, le toluène, le dichlorométhane, le chloroforme, le méthanol, l'éthanol ou l'isopropanol à une température comprise entre -15°C et 40°C. La transformation des composés de formule (II) dans lesquels $COOR_8$ est un groupe $CO_2$ $CH_2$ $C_6H_5$ en composé de formule (I) dans lesquels $R_6$ est un hydrogène est préférentiellement effectuée par hydrogénation catalytique en utilisant par exemple du palladium sur charbon comme catalyseur, sous pression atmosphérique d'hydrogène dans un solvant tel que le THF, l'éthanol, l'isopropanol, l'acétate d'éthyle, pouvant contenir jusqu'à 10 % d'acide acétique ou citrique, à une température comprise entre 0° et 60°C.

La préparation des dérivés de formule (I) dans laquelle $R_1$, Z, X, $R_5$ et $R_6$ sont décrits comme précédemment, et X représente un atome d'oxygène sont préparés, d'une manière générale, par condensation d'un dérivé de formule générale (V).

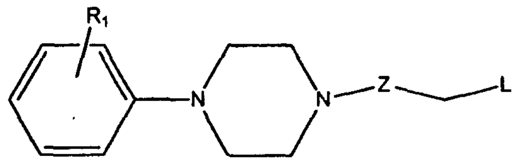

(V)

dans laquelle $R_1$ et Z sont définis comme dans la formule (I) et L représente un groupe partant tel qu'un halogène (de préférence un atome de brome, d'iode ou de chlore) un O-mésylate, O-triflate ou O-tosylate, avec un dérivé de la sérotonine de formule générale (VI):

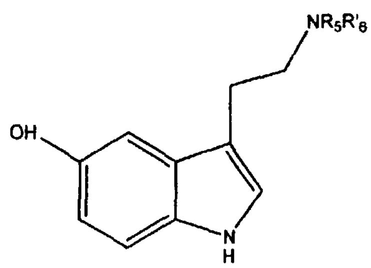

(VI)

dans laquelle le résidu $R_5$ est décrit comme précédemment et $R'_6$ représente $R_6$, $R_6$ étant décrit comme précédemment, ou $R'_6$ représente $COOR_8$, $R_8$ étant défini comme précédemment, suivie si nécessaire d'une transformation du groupe $COOR_8$ en $R_6$ = H selon le procédé décrit précédemment.

La préparation des dérivés de formule (I) par condensation des dérivés de formule (V) avec les dérivés de formule (VI) peut être réalisée, d'une façon générale, en présence d'une base organique (NaH, KH, $Et_3N$, DBU, DBN, TMP, DIPEA, tBuOK)
ou inorganique ($K_2CO_3$, $KHCO_3$, $NaHCO_3$,$CS_2CO_3$, KOH, NaOH, $CaCO_3$...) dans un solvant anhydre tel que le THF, la DMF, le DMSO, l'acétone, la diéthylcétone, la méthyléthylcétone, l'acétonitrile ou la DME à une température comprise entre 20° et 140°C, en présente ou non d'un sel comme catalyseur et qui peut être KI, $Bu_4NI$, LiI, $AgBF_4$, $AgClO_4$, $Ag_2CO_3$, KF, $Bu_4NF$ ou CsF. Le choix des conditions expérimentales pour réaliser la condensation entre les dérivés de formule (V) et (VI) pour obtenir les dérivés de formule (I) est bien évidemment dépendant de la nature des substituants dans les réactifs (V) et (VI) et plus particulièrement de la nature du groupement Z.

A titre d'exemple, lorsque Z est une fonction carbonyle (CO) et L un halogène, la condensation entre (V) et (VI) pour donner (I) est effectuée préférentiellement à 80°C, dans la méthyléthylcétone, en présence d'un excès de $K_2CO_3$ et d'une quantité catalytique de KI. Lorsque le groupe Z est défini comme $(CH_2)_n$ la condensation entre les dérivés (V) et (VI) est effectuée dans un solvant tel que la DMF ou le DMSO, en présence d'une base telle que la DBU ou la DIPEA, à 100°C en présence de KI ou de $Bu_4NI$ en quantité catalytique. Une méthode alternative consiste à condenser les dérivés (V) et (VI), en condition neutre, dans la DMF, en présence d'un large excès d'un fluorure tel que KF, CsF ou $Bu_4NF$.

Les composés de formule générale (V) dans laquelle les substituants $R_1$ et L sont définis comme précédemment, sont préparés par des méthodes qui diffèrent en fonction de la nature du résidu Z. C'est ainsi que les dérivés de formule (V) dans lesquels Z est un groupe carbonyle faisant partie d'une fonction amide sont obtenus par réaction

des pipérazines de formule générale (III).

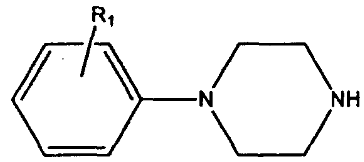

(III)

dans laquelle le résidu $R_1$ est défini comme dans la formule (I), avec un dérivé de formule (IX)

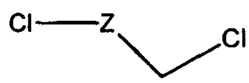

(IX)

dans laquelle Z représente C = O. Cette réaction qui permet de préparer les dérivés de formule (V) dans lesquels Z = CO et L = Cl à partir des arylamines (III) et des chlorures d'acide (IX) est une réaction bien connue de formation d'amide à partir d'une amine et d'un chlorure d'acide, et peut être réalisée dans un solvant tel que le dichlorométhane, le THF, le chloroforme, l'acétone, la méthyléthylcétone, la DME ou l'acétonitrile, à une température comprise entre -20°C et 80°C, en présence d'une base telle qu'une amine tertiaire (DBU, $Et_3N$, DIPEA) ou des bases inorganiques telles que des carbonates ($KHCO_3$, $NaHCO_3$, $K_2CO_2$, $Na_2CO_3$, $Cs_2CO_3$) la soude ou encore la potasse.

Les dérivés de formule (V) dans lesquels Z représente un groupe $-(CH_2)_n-$ sont généralement préparés par condensation d'une arylamine de formule (III) avec un dérivé de formule (X)

$$L - (CH_2)_n - CH_2 - L'$$

(X)

dans laquelle L représente un groupe partant tel qu'un chlore, un brome, un iode, un groupe mésylate, tosylate, triflate et L' peut être soit identique à L soit représente un groupe OR' dans lequel R' est défini comme un groupe protecteur d'un alcool tel qu'un éther silylé ($SiMe_3$, $Si^tBuMe_2$, $SiC_6H_5Me_2$), un tétrahydropyrane ou encore un benzyle ou un trityle. Il est bien entendu que dans le cas où L' est différent de L, la condensation entre la pipérazine de formule (III) et l'intermédiaire (X) est suivie de l'hydrolyse du groupe protecteur OR' pour donner un dérivé alcoolique intermédiaire qui est transformé en groupe partant ce qui conduit aux composés (V) dans lesquels $R_1$ et L sont définis comme précédemment. Dans la procédure mentionnée ci-dessus, l'hydrolyse de la fonction OR' en alcool est réalisée par les méthodes décrites et appropriées en fonction de la nature de R' (se référer à l'ouvrage de T.W. Greene, "Protective groups in organic synthesis", John Wiley & Sons, 1981) et la transformation de l'alcool ainsi obtenu en groupe partant [(de façon à obtenir les composés (V)] est réalisée par les techniques et méthodes bien connues pour ce type de transformation, telles que l'utilisation de $SOCl_2$ ou $POCl_3$ dans le dichlorométhane pour la formation de dérivés de formule (V) dans lesquels L = Cl, l'utilisation de $PBr_3$ ou $Br_2PPh_3$ pour la formation de dérivés de formule (V) dans lesquels L = Br, l'utilisation de $PI_3$ ou $P_2I_4$ pour la formation de dérivés de formule (V) dans lesquels L = I, l'utilisation du chlorure de tosyle pour la formation de dérivés de formule (V) dans lesquels L = OTos, l'utilisation du chlorure de mésyle pour la formation de dérivés de formule (V) dans lesquels L = OMes et enfin l'utilisation d'anhydride triflique pour la formation de dérivés de formule (V) dans lesquels L = OTf.

Les composés de formule générale (I) peuvent également être préparés par un autre procédé qui consiste à traiter une arylpipérazine de formule générale (III) définie comme précédemment avec un dérivé de la sérotonine de formule générale (XI)

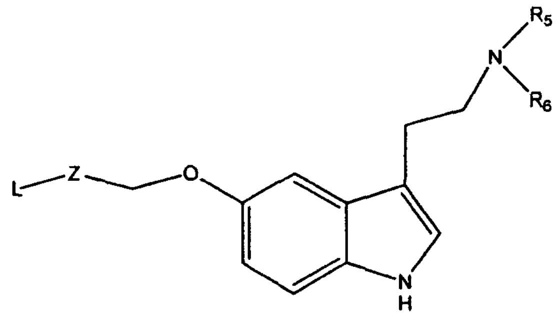

(XI)

dans laquelle les résidus $R_5$ et $R_6$ sont définis comme décrit précédemment dans la formule (I) et L est défini comme un groupe partant tel qu'un halogène (de préférence un atome de brome, d'iode ou de chlore), un mésylate, un tosylate ou un triflate ou le précurseur d'un groupe partant tel qu'un radical hydroxyle.

La préparation des dérivés de formule (I) dans lesquels Z représente un résidu $-(CH_2)_n-$ par ce procédé est réalisée par condensation entre un dérivé de pipérazine de formule (III) et un intermédiaire de formule générale (XI) en présence d'une base organique (NaH, $^tBuOK$, DBU, DIPEA) ou inorganique (KOH, $K_2CO_3$, $NaHCO_3$, $Cs_2CO_3$) dans un solvant anhydre tel que le THF, la DMF, le DMSO, l'acétonitrile ou la méthyléthylcétone à une température comprise entre 20 et 100°C.

Les intermédiaires de formule (XI) peuvent être préparés par condensation d'un dérivé de la sérotonine de formule (VI) dans laquelle $R_5$ et $R_6$ sont définis comme dans la formule (I) avec un dérivé de formule (XII)

$$L' - (CH_2)_n - CH_2 - L''$$

(XII)

dans laquelle L' et L" peuvent être simultanément des halogènes (chlore, brome ou iode) ou L' représente un groupe OR où R est un groupement protecteur classique tel qu'un groupement silylé (triméthylsilyle, triéthylsilyle, $^t$butyldiméthylsilyle), benzyle, tétrahydropyranyl ou trityl et dans ce cas L" représente un groupe partant tel qu'un halogène (de préférence un chlore, un iode ou un brome), un OMes, un OTos ou un OTf. Dans ce cas, après la condensation, L' qui représente OR sera déprotégé et transformé en groupe partant L comme défini dans la formule (XI) par les méthodes décrites précédemment.

Cette réaction de condensation entre les intermédiaires (VI) et (XII) tels que décrits précédemment est effectuée en milieu basique (en présente d'une base telle que NaH, KH, $^tBuOK$, $K_2CO_3$, $Cs_2CO_3$, DIPEA, DBU) dans un solvant anhydre tel que le DMSO, la DMF, le THF, l'acétonitrile, la méthyléthylcétone ou la DME à une température comprise entre 0 et 100°C.

Dans le cas particulier des dérivés de formule (I) dans lesquels $R_1$, $R_5$ et $R_6$ sont décrits comme précédemment mais où Z représente $-(CH_2)-$, une méthode de synthèse préférée consiste à réduire les dérivés correspondants de formule (I) dans lesquels Z représente CO par un agent de réduction qui permet de transformer une amide en amine tel que le borane ($BH_3.Me_2S$) ou $LiAlH_4$ en utilisant les méthodes et techniques bien connues pour ce type de réduction.

Dans le cas des dérivés de formule (I) dans lesquels $R_1$, Z, $R_5$ et $R_6$ sont décrits comme précédemment et dans lesquels X = $CH_2$, une méthode générale de synthèse consiste à condenser un intermédiaire de formule générale (IV)

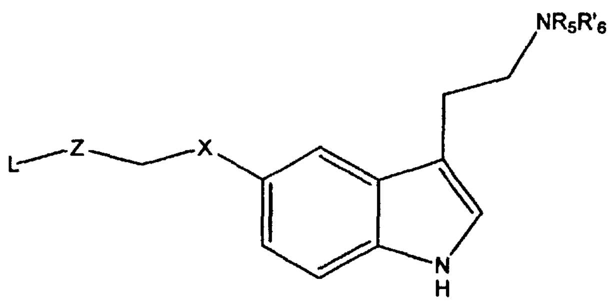

(IV)

dans laquelle Z, X et $R_5$ sont décrits comme précédemment, $R'_6$ représente $R_6$, $R_6$ étant défini comme précédemment ou $COOR_8$, $R_8$ étant défini comme précédemment, L représente un groupe partant avantageusement choisi dans le groupe constitué par un halogène (chlore, brome ou iode) ou un radical O-mésyle, O-trifluorométhoane sulfonyle ou O-tosyle, avec une arylpipérazine de formule générale (III) dans laquelle $R_1$ est défini comme précédemment, puis si nécessaire à transformer le groupe $COOR_8$ en hydrogène selon le procédé décrit précédemment.

Les méthodes utilisées pour obtenir les produits de formule générale (I) dans lesquels X = $CH_2$ par condensation des intermédiaires (IV) et (III) diffèrent en fonction de la nature du résidu Z et peuvent être comparables, en fonction de la variation de Z aux méthodes préalablement décrites pour la synthèse des produits (V) par condensation des intermédiaires (III) et (IX).

Les dérivés indoliques de formule générale (IV) dans lesquels Z = CO, L est un groupe partant tel qu'un halogène, $R_5$ et $R_6$ sont décrits comme précédemment, peuvent être obtenus par condensation d'un dérivé 5-bromo-indolique de formule (VIII)

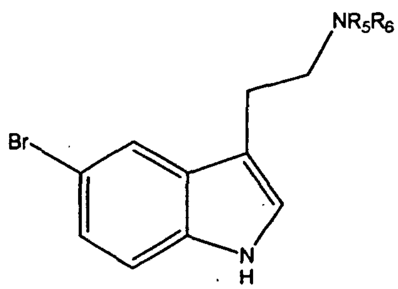

(VIII)

dans lequel $R_5$ et $R_6$ sont définis comme précédemment, avec un acide ou un ester dérivé de cet acide insaturé de formule générale (VII)

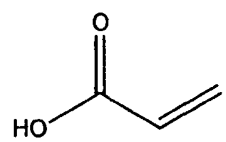

(VII)

en utilisant une catalyse au palladium (0) selon la méthode d'alkylation des aromatiques bien connue comme réaction de Heck, suivi de la réduction de la double liaison par hydrogénation catalytique ($H_2$ atmosphérique Pd/C, méthanol) et de la transformation de l'acide ou de l'ester ainsi formé en dérivé IV par les méthodes et techniques bien connues de l'homme du métier pour transformer un acide carboxylique ou un ester correspondant en chlorure d'acide X (L = Cl, Z = CO) ou en ester activé propre à être condensé avec une amine pour former une amide (en particulier par formation intermédiaire d'un anhydre mixte avec le chloroformate d'éthyle).

Les dérivés de formule générale (I) dans lesquels $R_1$, $R_5$ et $R_6$ sont décrits comme précédemment, Z = X = $CH_2$ sont préparés à partir des produits de formule générale (I) dans lesquels $R_1$, $R_5$ et $R_6$ sont décrits comme précé-

demment, Z = CO, X = $CH_2$ par réduction de la fonction amide avec un réducteur bien connu pour ce type de réaction tel que l'hydrure de lithium et aluminium dans un solvant aprotique tel que l'éther ou le THF.

Il faut également considérer comme partie intégrale de cette invention les méthodes de préparation de dérivés de formule (I) à partir d'autres dérivés de formule (I) dans laquelle au moins un des substituants $R_1$, $R_5$ ou $R_6$ sont différents par les techniques et méthodes bien connues de l'homme du métier.

En particulier, la présente invention revendique aussi une méthode de préparation de nombreux composés de formule générale (I) dans laquelle $R_1$ est un radical $NH_2$, $NR_5R_6$, $NHSO_2R_7$ en position ortho, méta ou para, à partir de composés de formule générale (I) préparés comme décrits précédemment et dans laquelle Z, X, $R_5$ et $R_6$ sont décrits comme précédemment et $R_1$ représente $NO_2$.

C'est ainsi que les composés de formule générale (I), dans laquelle $R_1$ représente $NH_2$, sont préparés par réduction du groupe nitro par les méthodes et techniques bien connues pour ce type de réduction (se référer par exemple à : R.C. Larouk "Comprehensive organic transformation", p. 412 (1989), VCH.), telle que l'hydrogénation atmosphérique catalyzée par du paladium sur charbon, l'utilisation de $SnCl_2$ ou de zinc ou encore, de catalyseur au rhodium en présence d'hydrazine.

Les intermédiaires de synthèse, dans laquelle $R_1$, représente $-N_2^+$, peuvent également être préparés à partir des anilines de formule (I) dans laquelle $R_1$ représente $NH_2$, après réaction avec l'acide nitreux ($HNO_2$) en utilisant les méthodes et techniques bien connues pour ce type de transformation (cfr Patai, "The Chemistry of Diazonium and diazogroups", Wiley, N.Y., 1978). Ces intermédiaires permettent de préparer les produits de formule générale (I) dans lesquels $R_1$ = $SO_2$ $NHR_7$ après réaction avec $SO_2$ en présence de chlorure cuivrique (Gilbert, synthésis, 1-10, 1969) suivi de la condensation du chlorure de sulfonyle intermédiaire avec une amine ($R_7NH_2$) en présence d'une base telle que la pyridine, la triéthylamine ou la DMAP.

Les composés de formule générale (I) dans lesquels le groupe $R_1$ représente $NR_5R_6$ ou $NHSO_2R_7$ en position ortho, méta ou para peuvent également être obtenus à partir des composés de formule (I) dans laquelle $R_1$ représente $NH_2$ par divers réactifs et méthodes qui dépendent de la nature du groupe $R_1$. C'est ainsi que les composés de formule (I) dans laquelle $R_1$ représente un groupe $NR_5R_6$ (dans lequel $R_6$ = H ou $R_5$) sont préparés par réaction en milieu basique (en présence d'une base organique telle que DBU, $^t$BuOK, DMAP, DIPEA ou inorganique telle que $CS_2CO_3$, HaH, KH) avec des agents d'alkylation correspondants ($R_5$ - L dans lequel L représente un atome de chlore, de brome, d'iode ou un radical O-mesyl, O-toxyl ou O-trifluorasulfonyle). Le contrôle de la réaction (temps, solvant, température, nombre d'équivalents de $R_5$-L) permet d'obtenir l'aniline mono ou di-alkylée.

Les composés de formule (I) dans lesquels $R_1$ représente $NHSO_2R_7$ sont accessibles par une réaction similaire qui consiste à condenser l'aniline avec un chlorure de sulfonyle de formule $ClSO_2R_7$.

On comprendra que dans certaines des transformations ci-dessus, il peut être nécessaire ou souhaitable de protéger des groupes sensibles éventuels de la molécule en question afin d'éviter des réactions secondaires indésirables. Ceci peut être réalisé par l'utilisation des groupes protecteurs conventionnels tels que ceux décrits dans "Protective Groups in Organic Synthesis" ed. J.F. McOwie, Plenum Press, 1973 et dans T.W. Greene, Protective Groups in Organic Synthesis, John Wiley & Sons, 1981. Les groupes protecteurs peuvent être enlevés lors de toute étape ultérieure appropriée, en utilisant les méthodes et techniques également décrites dans les références citées précédemment. C'est ainsi que dans certains cas particuliers il peut être nécessaire de protéger l'azote indolique lors de la préparation de composés de formule (I).

Lorsque l'on désire isoler un composé selon l'invention à l'état de sel, par exemple à l'état de sel formé par addition avec un acide, on peut y parvenir en traitant la base libre de formule générale (I) par un acide approprié de préférence en quantité équivalente, ou par le sulfate de créatinine dans un solvant approprié.

Lorsque les procédés décrits ci-dessus pour préparer les composés de l'invention donnent des mélanges de stéréoisomères, ces isomères peuvent être séparés par des méthodes conventionnelles telles que la chromatographie préparative.

Lorsque les nouveaux composés de formule générale (I) possèdent un ou plusieurs centres asymétriques, il peuvent être préparés sous forme de mélange racémique ou sous forme d'énantiomères que ce soit par synthèse énantiosélective ou par résolution. Les composés de formule (I) possédant au moins un centre asymétrique peuvent par exemple être séparés en leurs énantiomères par les techniques habituelles telle que la formation de paires diastéréomériques par formation d'un sel avec un acide optiquement actif tel que l'acide (-) di-p-toluoyl-1-tartrique, l'acide (+) di-p-toluoyl-1-tartrique, l'acide (+) camphor sulfonique, l'acide (-) camphor sulfonique, l'acide (+) phénylpropionique, l'acide (-) phénylpropionique, suivie par cristallisation fractionnée et régénération de la base libre. Les composés de formule (I) dans lesquels $R_6$ est un hydrogène comprenant au moins un centre asymétrique peuvent également être résolus par formation d'amides diastéréomériques qui sont séparés par chromatographie et hydrolysés pour libérer l'auxiliaire chiral.

D'une façon générale, les composés de formule (I) peuvent être purifiés par les méthodes habituelles, par exemple par cristallisation (en particulier lorsque les composés de formule (I) sont isolés sous forme de sel), chromatographie ou extraction.

Les exemples 1, 2, 5, 7, 8, 13, 14, 19, 20, 23, 25, 31, 35, 36, 37, 40, 41, 44, 46, 50, 57, 58, 59, 61, 62 et 63 qui suivent illustrent l'invention sans toutefois en limiter la portée.

Les exemples 3, 4, 6, 9, 10, 11, 12, 15, 16, 17, 18, 21, 22, 24, 26, 27, 28, 29, 30, 32, 33, 34, 38, 39, 42, 43, 45, 47, 48, 49, 51, 52, 53, 54, 55, 56, 60, 64 à 70 qui suivent sont des exemples comparatifs.

**Exemple 1:** Chlorhydrate de la 2-[3-(2-amino-éthyl)-1-H-indol-5-yloxy]-1-[4-(nitro-phényl)-pipérazin-1-yl]-éthanone.

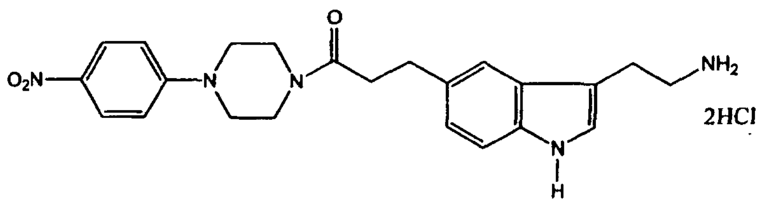

**1A** - 3-(2-N-terbutoxycarbonyl)amino-éthyl)-1-H-indol-5-ol

Le sel créatine sulfate monohydrate de la sérotonine (102 g, 252 mmol) est traité par le diterbutyle dicarbonate (82,6 g, 378 mmol) dans l'eau (2,1 l) en présence de soude 2N (420 ml) à température ambiante. Après 1 heure la réaction est diluée par de l'acétate d'éthyle (3 1) et agitée pendant 10 minutes. Les 2 phases formées sont séparées par décantation ; la phase organique est lavée à l'eau, séchée sur sulfate de sodium, filtrée puis évaporée à sec. Le sirop obtenu est chromatographié sur colonne de gel de silice éluée par un mélange dichlorométhane/méthanol (20: 1 ; v/v). Le composé pur est isolé sous forme de sirop marron (65,9 g ; 95 %). Analyse élémentaire ($C_{15}H_{20}N_2O_3$), % calculés : C 65,20 ; H 7,30 ; N 10,14 ; % trouvés: C64.15 ; H 7.47; N 9.77.

Spectre de résonance magnétique nucléaire du proton, $CDCl_3$ (ppm) : 1,44 s, 9H (tBu) ; 2,86 t, 2H ($CH_2$) ; 3,45 m, 2H ($CH_2$) ; 4,68 s, 1H (NH) ; 5,59 s, 1H (O-H) ; 6,77-7,26 m, 4H (Ar + éthylénique) ; 7,99 s, 1H (NH).

**1B -** 2-chloro-1- [4-nitro-phényl)-pipérazin-1-yl]-éthanone

La 4-nitro-phényl-pipérazine (7 g ; 33,8 mmol) en solution dans la méthyléthylcétone (223 ml), en présence de carbonate de calcium (10,1 g ; 101,4 mmol) est traitée à 0°C et goutte-à-goutte par le chlorure de chloracétyle (3,2 ml ; 40,5 mmol). Après 1 heure à 0°C le milieu est dilué à l'acétate d'éthyle, filtré sur célite, lavé à l'eau puis par une solution de chlorure de sodium. La phase organique est séchée sur sulfate de sodium, filtrée et évaporée. Le solide marron obtenu (7,8 g ; 82 %) est engagé sans autre purification dans l'étape suivante.

**1C** - 2-[3-(2-N-(terbutoxycarbonyl)-amino-éthyl)-1-H-indol-5-yloxy]-1-[4-(4-nitro-phényl)-pipérazin-1-yl]-éthanone.

Un mélange du composé 1A (9,67 g ; 35,01 mmol) et du composé 1B (24,8 g ; 87,5 mmol) dans la méthyléthylcétone (400 ml), en présence du carbonate de potassium (12,1 g; 87,5 mmol) et d'iodure de potassium (581 mg; 3,5 mmol) est chauffé à reflux pendant 5 heures. Le composé 1A (2,0 g ; 7,2 mmol) est à nouveau additionné et la réaction est agitée au reflux 1 heure de plus. Le milieu est ensuite dilué au dichlorométhane, filtré sur célite, lavé à l'eau puis par une solution de chlorure de sodium. La phase organique est séchée sur sulfate de sodium, filtrée puis évaporée à sec. Le sirop obtenu est chromatographié sur colonne de gel de silice éluée par un mélange dichlorométhane/acétone (3:1 ; v/v). Le produit pur est obtenu sous forme de poudre orange (18,2 g ; 83 %).
Analyse élémentaire ($C_{27}H_{33}N_5O_6$), % calculés : C 61,94 ; H 6,35 ; N 13,38 ; % trouvés : C 61,19 ; H 6,22 ; N 13,02.
Spectre de résonance magnétique nucléaire du proton, $CDCl_3$ (ppm) : 1,44 s, 9H (tBu) ; 3,86 t, 4H ($CH_2$) ; 3,83 m, 4H ($CH_2$) ; 4,65 s, 1H (NH) ; 4,79 s, 2H ($COCH_2O$) ; 6,79-7,30 m, 6H (Ar + éthylénique) ; 8,11-8,16 m, 3H (Ar+NH).
Point de fusion: 198°C.

**1** - Chlorhydrate de la 2-[3-(2-amino-éthyl)-1H-indol-5-yloxy]-1-[4-(4-nitro-phényl)-pipérazin-1-yl]-éthanone.

Le produit 1C (930 mg ; 1,77 mmol) en solution dans le toluène (46 ml) est traité par l'acide trifluoroacétique (10 ml). Après 1 h 30 à température ambiante le milieu est dilué au dichlorométhane, lavé à la soude 2N puis à l'eau. La phase organique est séchée sur sulfate de sodium, filtrée et évaporée à sec. Le sirop obtenu est chromatoraphié sur colonne de gel de silice éluée par un mélange chloroforme/méthanol/ammoniaque (80:18,5:1,5 ; v/v). Le produit pur est obtenu sous forme de sirop jaune (317 mg ; 45 %). Ce composé est dilué dans le méthanol et le bis-chlorhydrate est formé par addition de la quantité nécessaire d'acide chlorhydrique dans le méthanol.

Analyse élémentaire ($C_{22}H_{27}N_5O_4Cl_2$), % calculés : C 53,23 ; H 5,48 ; N 14,11; % trouvés : C 53,83 ; H 5,77 ; N 13,80.
Spectre de résonance magnétique nucléaire 1H, DMSO-d6 (ppm) : 2,99 s, 4H ($CH_2$) ; 3,54-3,71 m, 8 H ($CH_2$), 4,84 s, 2H ($COCH_2O$) ; 6-77-6,83 dd, 1H (éthylénique) ; 7,01-7,29 m, 5H (Ar) ; 8,06-8,11 m, 5H (Ar+$NH_3^+$) ; 10,85 d, 1H (NH).
Point de fusion : 206°C.

**Exemple 2:** Chlorhydrate de la 2-[3-(2-amino-éthyl)-1H-indol-5-yloxy]-1-[4-(amino-phényl)pipérazin-1-yl]-éthanone.

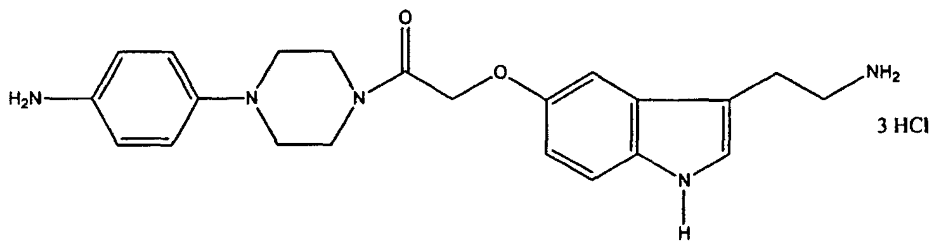

Le produit 1 (5 g ; 9,5 mmol) en suspension dans le méthanol (250 ml) en présence d'une quantité catalytique de palladium sur charbon (505 mg ; 0,47 mmol) est hydrogéné dans un appareil de Parr, sous une pression de 40 psi. Après 12 heures le mélange est filtré sur célite et celle-ci est lavée plusieurs fois au méthanol ce qui permet d'obtenir le produit de réduction pratiquement pur dans le filtrat. Ensuite la célite est lavée au dichlorométhane ce qui permet de récupérer le produit de départ qui n'a pas réagi.

Le filtrat contenant le produit formé est évaporé à sec et le sirop obtenu est chromatographié sur colonne de gel de silice éluée par un mélange dichlorométhane acétone (3:1, v/v). Le produit pur est obtenu sous la forme d'un sirop jaune (2,8 g ; 60 %).
Spectre de résonance magnétique nucléaire, 1H, $CDCl_3$ (ppm) : 1,44 s, 9H (tBu) ; 2,18 s, 2H ($NH_2$) ; 2,87-3,80 m, 12H ($CH_2$) ; 4,66 s, 1H (NH) ; 4,77 s, 2H ($COCH_2O$) ; 6,63-7,28 m, 8H (Ar + éthylénique) ; 8,16 s, 1H (NH).

**2** - Chlorhydrate de la 2-[3-(2-amino-éthyl)-1H-indol-5-yloxy]-1-[4-(4-amino-phényl)-pipérazin-1-yl]-éthanone

Le produit 2A (252 mg ; 0,51 mmol) en solution dans le toluène (13 ml) est traité par l'acide trifluoroacétique (3 ml) à température ambiante. Après 1 heure le milieu est dilué à l'acétate d'éthyle, lavé à la soude 2N, à l'eau puis par une solution saturée de chlorure de sodium. La phase organique est séchée sur sulfate de sodium, filtrée et évaporée. Le sirop obtenu est chromatographié sur colonne de gel de silice éluée par un mélange chloroforme/méthanol/ammoniaque (80:18,5:1,5 ; v/v). Le produit pur est isolé sous forme de sirop jaune (174 mg ; 87 %). Le composé obtenu est dissout dans le méthanol et le chlorhydrate est formé par addition de la quantité nécessaire d'acide chlorhydrique dans le méthanol.
Analyse élémentaire ($C_{22}H_{30}N_5O_2Cl_3.2H_2O$), % calculée : C 49,03 ; H 6,36, N 12,99 ; % trouvés : C 49,37 ; H 6,18 ; N 12,67.
Spectre de résonance magnétique nucléaire, 1H, DMSO-d6 (ppm) : 2,99-3,70 m, 12H ($CH_2$) ; 4,83 s, 2H ($COCH_2O$) ; 6,76-6,82 dd, 1H (éthylénique) ; 7,05-7,28 m, 7H (Ar) ; 8,13 s, 3H ($NH_3^+$) ; 10,86 s, 1H (NH).
Point de fusion : 196°C.

**Exemple 3:** Chlorhydrate de la N-[4-(4-{2-[3-(2-amino-éthyl)-1H-indol-5-yloxy]-acétyl}-pipérazin-1-yl)phényl]acétamide.

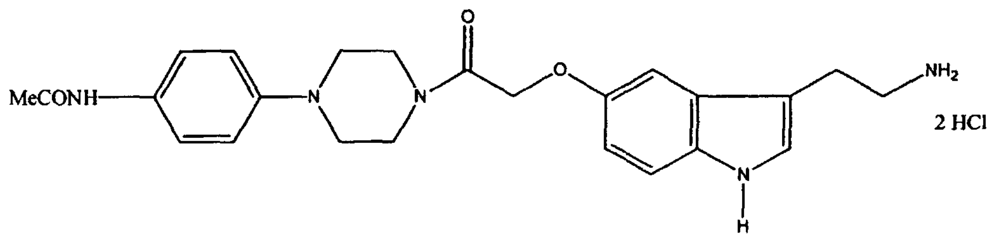

Le produit 2A (300 mg ; 0,61 mmol) en solution dans la pyridine (7,5 ml) est traité à 0°C par l'anhydride acétique (60 ml ; 0,61 mmol). Après 2 heures d'agitation à température ambiante le milieu est dilué à l'acétate d'éthyle et lavé successivement par une solution saturée de sulfate de cuivre, l'eau, et enfin par une solution saturée de chlorure de sodium. La phase organique est séchée sur sulfate de sodium, filtrée et évaporée à sec. Le sirop obtenu est chromatographié sur colonne de gel de silice éluée par un mélange dichlorométhane/acétone (2:1, v/v). Le produit pur est obtenu sous forme de sirop jaune (249 mg ; 77 %). Ce composé est ensuite déprotégé selon la méthode décrite pour la préparation de l'exemple 2.

La purification du produit sous forme de base est effectuée par chromatographie sur colonne de gel de silice éluée par un mélange chloroforme/méthanol/ammoniaque (80:18,5:1,5, v/v). Le produit pur est isolé sous forme de sirop incolore qui conduit, après traitement à l'acide chlorhydrique dans le méthanol, au composé 3 (113 mg ; 36%).
Analyse élémentaire ($C_{24}H_{31}N_5O_3Cl_2$.0,5 $H_2O$), % calculés : C 55,71 ; H 6,23 ; N 13,53 ; % trouvés : C 55,70 ; H 6,40 ; N 13,46.
Spectre de résonance magnétique nucléaire 1H, DMSO-d6 (ppm) : 2,00 s, 3H ($CH_3$); 2,76-3,16 m, 8H ($CH_2$) ; 3,68 m, 6H ($CH_2$ + $H_2O$) ; 4,82 s, 2H (CO$CH_2$O) ; 6,77-6,82 dd, 1H (éthylénique) ; 6,97-7,50 m, 7H (Ar) ; 8,01 s, 3H ($NH_3$+) ; 9,85 s, 1H (NH) ; 10,86 s, 1H (NH).
Point de fusion : 169°C.

**Exemple 4:** Chlorhydrate de la N-[4-(4-{2-[3-(2-amino-éthyl)-1H-indol-5-yloxy]-acétyl}-pipérazin-1-yl)phényl]-benzamide.

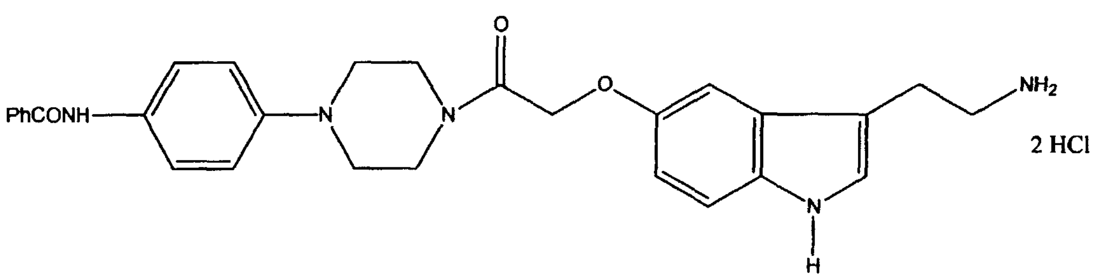

Le composé 4 est obtenu à partir du produit 2A (700 mg ; 1,42 mmol) et de chlorure de benzoyle (0,16 ml ; 1,42 mmol) selon la procédure décrite pour la préparation de l'exemple 3.

La purification du produit sous forme de base est effectuée par chromatographie sur colonne de gel de silice éluée par un mélange chloroforme/méthanol/ammoniaque (80:18,5:1,5 ; v/v). Le produit pur est isolé sous forme de sirop jaune qui conduit, après traitement à l'acide chlorhydrique dans le méthanol, au composé 4 (560 mg, 66%).
Analyse élémentaire ($C_{29}H_{33}N_5O_3Cl_2$.2$H_2O$), % calculés : C 57,43 ; H 6,15 ; N 11,55 ; % trouvés : C 57,32 ; H 5,97 ; N 11,53.
RMN 1H, DMSO-d6 (ppm) : 3,00 s, 4H ($CH_2$) ; 3,35-3,42 d, 4H ($CH_2$) ; 3,89 s, 4H ($CH_2$) ; 4,87 s, 2H (CO$CH_2$O) ; 6,78-6,84 dd, 1H (éthylénique) ; 7,21-7,99 m, 12H (Ar) ; 8,10 s, 3H ($NH_3^+$); 10,35 s, 1H (NH) ; 10,86 s, 1H (NH).
Point de fusion : 184-185°C.

**Exemple 5:** Chlorhydrate de la N-[4-(4-{2-[3-(2-amino-éthyl)-1H-indol-5-yloxy]-acétyl}-pipérazin-1-yl)phényl]-méthanesulfonamide.

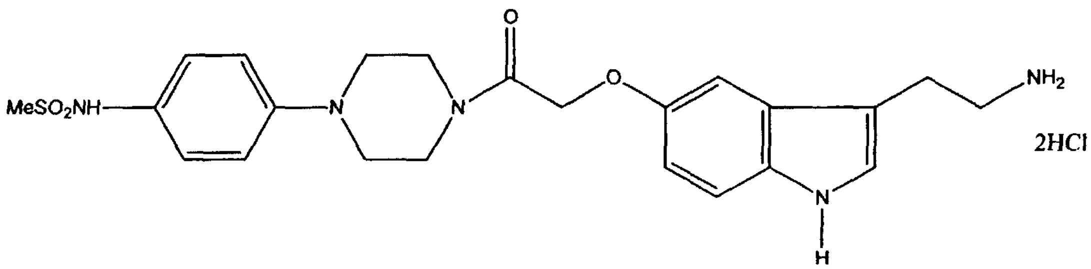

Le composé 5 est obtenu à partir du produit 2A (201 mg ; 0,407 mmol) et de chlorure de méhanesulfonic (31 µl; 0,407 mmol) selon la procédure décrite pour la préparation de l'exemple 3.

La purification du produit sous forme de base est effectuée par chromatographie sur colonne de gel de silice éluée par un mélange chloroforme/méthanol/ammoniaque (80:18,5:1,5 ; v/v). Le produit pur est isolé sous forme de sirop incolore qui conduit, après traitement à l'acide chlorhydrique dans le méthanol, au composé 5 (151 mg, 66%).
Analyse élémentaire ($C_{23}H_{31}N_5O_5SCl_2.2H_2O$), % calculés : C 49,11 ; H 5,91 ; N 12,45 ; % trouvés : C 49,88 ; H 5,73 ; N 11,97.
RMN 1H, DMSO-d6 (ppm) : 2,89 s, 3H (MeSO$_2$) ; 2,99-3,19 m, 8H (CH$_2$) ; 3,68 s, 4H (CH$_2$) ; 4,82 s, 2H (COCH$_2$O) ; 6,77-6,82 dd, 1H (éthylénique) ; 7,01-7,29 m, 7H (Ar) ; 8,02 s, 3H ($NH_3^+$) ; 9,41 s, 1H (NH) ; 10,85 s, 1H (NH).
Point de fusion: 150°C.

**Exemple 6:** Chlorhydrate de la N-[4-(4-{2-[3-(2-amino-éthyl)-1H-indol-5-yloxy]-acétyl}-pipérazin-1-yl)-phényl]-méthanesulfonyl-méthanesulfonamide

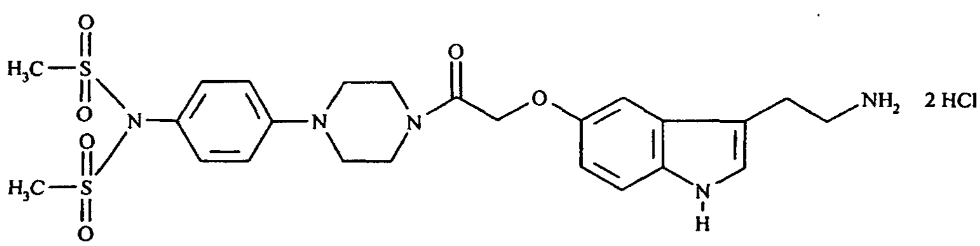

Le produit 2A (500 mg; 1,01 mmol) en solution dans le dichlorométhane (12,5 ml) en présence de triéthylamine (0,56 ml ; 4,04 mmol) est traité par le chlorure de mésyle (0,17 ml ; 2,03 mmol) à 0°C. Après une nuit à température ambiante le milieu est dilué au dichlorométhane et lavé à l'eau. La phase organique est séchée sur sulfate de sodium, filtrée et évaporée à sec. Le sirop obtenu est chromatographié sur colonne de gel de silice éluée par un mélange acétate d'éthyle/dichlorométhane (2:1, v/v). Le produit pur est obtenu sous forme de sirop incolore (127 mg ; 20 %). Ce composé est ensuite déprotégé selon la méthode décrite pour la préparation de l'exemple 2.

La purification du produit sous forme de base est effectuée par chromatoraphie sur colonne de gel de silice éluée par un mélange chloroforme/méthanol/ammoniaque (80:18,5:15, v/v). Le produit pur est isolé sous forme de sirop incolore qui conduit, après traitement à l'acide chlorhydrique dans le mathanol, au composé 6 (109 mg ; 90%).
Analyse élémentaire ($C_{24}H_{33}N_5O_6S_2Cl_2$), % calculés : C 46,30 ; H 5,34 ; N 11,25; % trouvés : C 46,47 ; H 5,47 ; N 10,96.
RMN 1H, DMSO-d6 (ppm) : 2,99-3,90 m, 18H (MeSO$_2$-+CH$_2$) ; 4,83 s, 2H (COCH$_2$O) ; 6,78-6,83 dd, 1H (éthylénique) ; 6,97-7,33 m, 7H (Ar) ; 7,96 s, 3H ($NH_3^+$); 10,85 s, 1H (NH).
Point de fusion : 202 °C.

**Exemple 7:** Chlorhydrate de la N-[4-(4-{2-[3-(2-amino-éthyl)-1H-indol-5-yloxy]-acétyl}-pipérazin-1-yl)phényl]-éthane-sulfonamide.

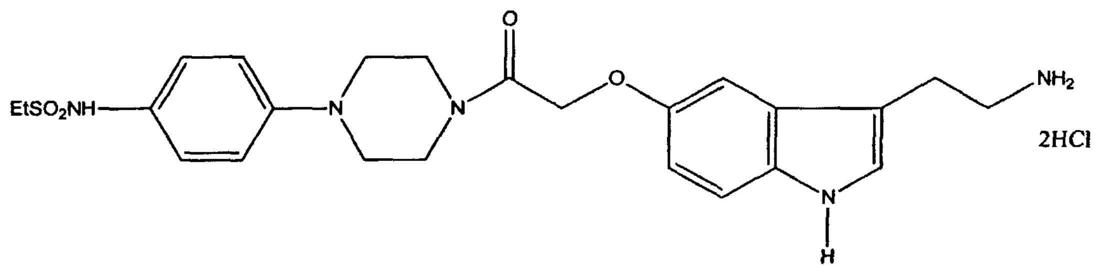

Le composé 7 est obtenu à partir du produit 2A (800 mg ; 1,6 mmol) et de chlorure d'éthanesulfonic (0,15 ml ; 1,6 mmol) selon la procédure décrite pour la préparation de l'exemple 3.

La purification du produit sous forme de base est effectuée par chromatoraphie sur colonne de gel de silice éluée par un mélange chloroforme/méthanol/ammoniaque (80:18,5:15, v/v). Le produit pur est isolé sous forme de sirop orange qui conduit, après traitement à l'acide chlorhydrique dans le méthanol, au composé 7 (730 mg ; 78%).
Analyse élémentaire ($C_{24}H_{33}N_5O_4S_1Cl_2.H_2O$), % calculés : C 50,00 ; H5,95 ; N 12,54 ; % trouvés : C 50,00 ; H 5,75 ; N 12,54.
RMN 1H, DMSO-d6 (ppm) : 1,15-1,22 t, 3H ($CH_3$) ; 2,94-3,16 m, 10H (CH2) ; 3,73 m, 4H ($CH_2$) ; 4,82 s, 2H ($COCH_2O$) ; 6,77-6,82 dd, 1H (éthylénique) ; 7,13-7,28 m, 7H (Ar) ; 7,99 s, 3H ($NH_3^+$) ; 9,55 s, 1H (NH) ; 10,84 s, 1H (NH).
Point de fusion : 222 °C.

**Exemple 8:** Chlorhydrate du thiophène-2-{N-[4-(4-{2-[3-(2-amino-éthyl)-1H-indol-5-yloxy]-acétyl}-pipérazin-1-yl)phényl]-sulfonamide.

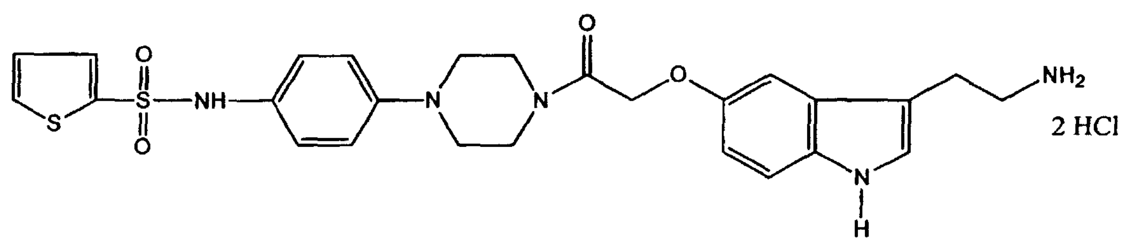

Le composé 8 est obtenu à partir du produit 2A (800 mg ; 1,62 mmol) et de chlorure de thiophène sulfonic (296 mg ; 1,62 mmol) selon la procédure décrite pour la préparation de l'exemple 3.
La purification du produit sous forme de base est effectuée par chromatoraphie sur colonne de gel de silice éluée par un mélange chloroforme/méthanol/ammoniaque (80:18,5:15 ; v/v). Le produit pur est isolé sous forme de poudre jaune qui conduit, après traitement à l'acide chlorhydrique dans le méthanol, au composé 8 (575 mg ; 57 %).
Analyse élémentaire ($C_{26}H_{31}N_5O_4S_2Cl_2.H_2O$), % calculés : C 49,52 ; H5,27 ; N 11,11 ; % trouvés : C 50,44 ; H 5,49 ; N 10,86.
RMN 1H, DMSO-d6 (ppm) : 2,99-3,16 m, 8H ($CH_2$) ; 3,67 s, 4H ($CH_2$), 4,81 s, 2H ($COCH_2O$) ; 6,76-6,81 dd, 1H (éthylénique indole) ; 7,00-7,28 m, 8H (Ar + éthylénique thiophène) ; 7,48 dd, 1H (éthylénique thiophène) ; 7,88 dd, 1H (éthylénique thiophène) ; 8,06 s, 3H ($NH_3^+$) ; 10,13 s, 1 H (NH) ; 10,86 s, 1H (NH).
Point de fusion: 185 °C.

**Exemple 9:** Chlorhydrate de N-[4-(4-{2-[3-(2-amino-éthyl)-1H-indol-5-yloxy]-acétyl}-pipérazin-1-yl)-phényl]-thiophène-2-sulfonyl-thiophène-2-sulfonamide.

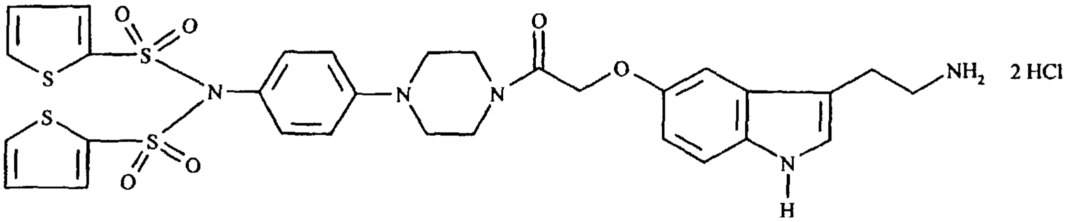

Le composé 9 est obtenu à partir du produit 2A (800 mg ; 1,62 mmol) et de chlorure de thiophène sulfonic (335 mg ; 1,94 mmol) selon la procédure décrite pour la préparation de l'exemple 6.

La purification du produit sous forme de base est effectuée par chromatographie sur colonne de gel de silice éluée par un mélange chloroforme/méthanol/ammoniaque (80:16,5:15, v/v). Le produit pur est isolé sous forme de sirop jaune qui conduit, après traitement à l'acide chlorhydrique dans le méthanol, au composé 9 (380 mg ; 32%).
Analyse élémentaire ($C_{30}H_{33}N_5O_6S_4Cl_2$), % calculés : C 47,49 ; H4,35, N 9,23; % trouvés : C 47,62 ; H 4,40 ; N 9,20.
RMN 1H, DMSO-d6 (ppm) : 3,00 s, 4H ($CH_2$) ; 3,27 s, 4H ($CH_2$), 3,63 s, 4H ($CH_2$); 4,82 s, 2H ($COCH_2O$) ; 6,77-7,30 m, 10H, (Ar + éthylénique) ; 7,70 d, 2H (éthyléniques) ; 8,05 s, 3H ($NH_3^+$) ; 8,20 d, 2H (éthyléniques) ; 10,85 s, 1H (NH).
Point de fusion: 180 °C.

**Exemple 10:** Chlorhydrate du 3,5-diméthyl-isoxazole-4-{N-[4-(4-{2-[3-(2-amino-éthyl)-1H-indol-5-yloxy]-acétyl}-pipérazin-1-yl)phényl]-sulfonamide.

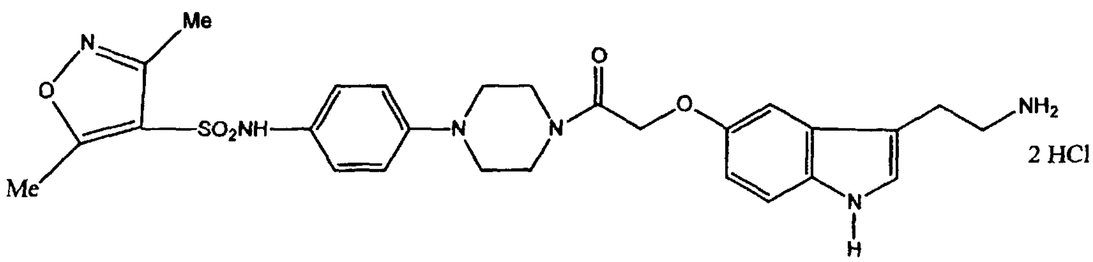

Le composé 10 est obtenu à partir du produit 2A (800 mg ; 1,62 mmol) et de chlorure de 3,5-dimméthyl-isoxazole-4-sulfonic (317 mg ; 1,62 mmol) selon la procédure décrite pour la préparation de l'exemple 3.

La purification du produit sous forme de base est effectuée par chromatoraphie sur colonne de gel de silice éluée par un mélange chloroforme/méthanol/ammoniaque (80:18,5:1,5, v/v). Le produit pur est isolé sous forme de sirop jaune qui conduit, après traitement à l'acide chlorhydrique dans l'éther, au composé 10 (576 mg ; 55%).
Analyse élémentaire ($C_{27}H_{34}N_6O_5S_1Cl_2.H_2O$), % calculés : C 50,11 ; H5,67, N 12,99 ; % trouvés : C 50,21 ; H 5,22 ; N 12,52.
RMN 1H, DMSO-d6 (ppm) : 2,15 s, 3H (Me) ; 2,33 s, 3H (Me), 2,92-3,07 m, 8H ($CH_2$) ; 3,64 s, 4H ($CH_2$) ; 4,80 s, 3H ($COCH_2O$) ; 6,77-6,82 dd, 1H (éthylénique); 6,95-7,28 m, 7H (Ar) ; 7,88 s, 3H ($NH_3^+$) ; 9,98 s, 1H (NH); 10,84 s, 1H (NH).
Point de fusion: 193°C.

**Exemple 11**: Chlorhydrate de la 2-[3-(2-amino-éthyl)-1H-indol-5-yloxy]-1-[4-(4-N-{ éthoxy-carbonyl}-amino-phényl)-pipérazin-1-yl]-éthanone.

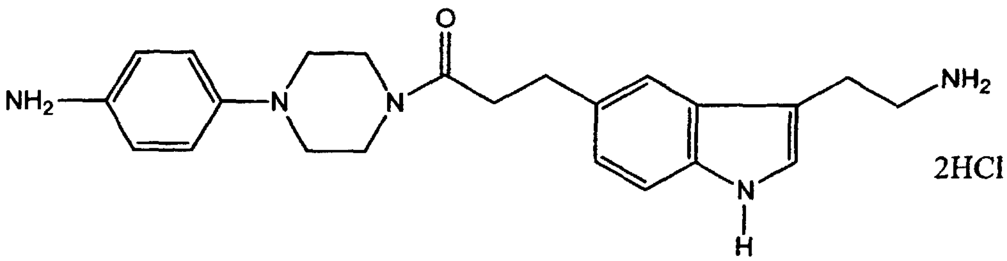

2HCl

Le composé 11 est obtenu à partir du produit 2A (600 mg ; 1,21 mmol) et de chloroformate d'éthyle (0,13 ml ; 1,33 mmol) selon la procédure décrite pour la préparation de l'exemple 6.
La purification du produit sous forme de base est effectuée par chromatoraphie sur colonne de gel de silice éluée par un mélange chloroforme/méthanol/ammoniaque (80:18,5:1,5 ; v/v). Le produit pur est isolé sous forme de sirop incolore qui conduit, après traitement à l'acide chlorhydrique dans l'éther, au composé 11 (230 mg ; 38%).
Analyse élémentaire ($C_{25}H_{32}N_5O_4Cl$), % calculés : C 59,81 ; H 6,42, N 13,95 ; % trouvés : C 60,26 ; H 6,52 ; N 13,49.
RMN 1H, DMSO-d6 (ppm) : 1,22 t, 3H ($CH_3$) ; 2,81-3,03 m, 8H ($CH_2$) ; 3,64 s, 4H ($CH_2$); 4,03-4,14 q, 2H ($CH_2$); 4,80 s, 2H ($CO\underline{CH_2}O$); 6,75-6,80 dd, 1H (éthylénique); 6,80-7,33 m, 7H (Ar); 9,34 s, 1H (NH); 10,74 s, 1H (NH).
Point de fusion: 153°C.

**Exemple 12:** Chlorhydrate de la 2,2,2-trifluoro-éthane [4-(4-{2-[3-(2-amino-éthyl)-1H-indol-5-yloxy]-acétyl}-pipérazin-1-yl)phényl]-sulfonamide.

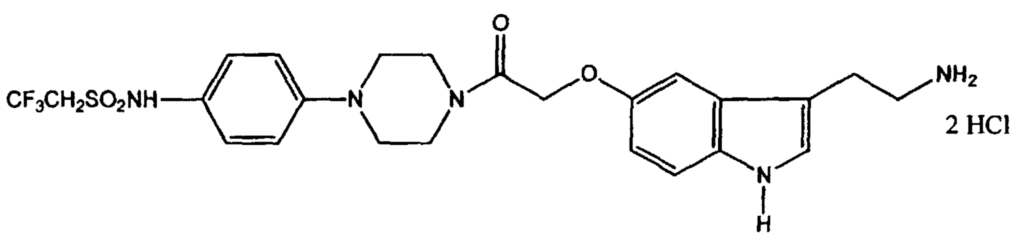

2 HCl

Le composé 12 est obtenu à partir du produit 2A (800 mg ; 1,62 mmol) et de chlorure de trifluoroéthane sulfonic (0,36 ml ; 3,24 mmol) selon la procédure décrite pour la préparation de l'exemple 3.

La purification du produit sous forme de base est effectuée par chromatoraphie sur colonne de gel de silice éluée par un mélange chloroforme/méthanol/ammoniaque (80:18,5:1,5 ; v/v). Le produit pur est obtenu sous forme de sirop jaune-vert qui conduit, après traitement à l'acide chlorhydrique dans le méthanol, au composé 12 (643 mg ; 65 %).
RMN 1H, DMSO-d6 (ppm) : 2,99-3,16 m, 8H ($CH_2$) ; 3,67 s, 4H ($CH_2$), 4,82 s, 2H ($CO\underline{CH_2}O$) ; 6,76-6,82 dd, 1H (éthylénique) ; 6,99-7,55 m, 7H (Ar) ; 7,55 s, 2H ($CF_3\underline{CH_2}SO_2$) ; 8,07 s, 3H ($NH_3^+$) ; 10,12 s, 1H (NH) ; 10,85 s, 1H (NH).

**Exemple 13:** Chlorhydrate de la N-[4-(4-{2-[3-(2-amino-éthyl)-1H-indol-5-yloxy]-acétyl}-pipérazin-1-yl)phényl]-isopropanesulfonamide.

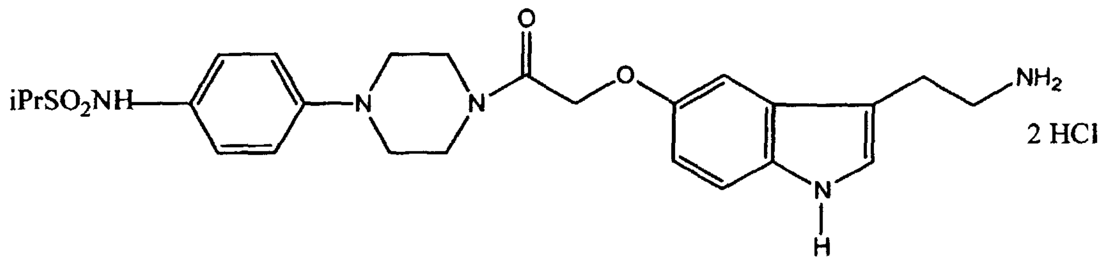

Le composé 13 est obtenu à partir du produit 2A (800 mg ; 1,62 mmol) et de chlorure d'isopropylsulfonic (0,18 ml ; 1,62 mmol) selon la procédure décrite pour la préparation de l'exemple 3.
(80:18,5:1,5 ; v/v). Le produit pur est isolé sous forme de sirop qui conduit, après traitement à l'acide chlorhydrique dans l'éther, au composé 13 (381 mg ; 41 %).
RMN 1H, DMSO-d6 (ppm) : 11,22 d, 6H (Me) ; 2,99-3,17 m, 9H ($CH_2$ + CH) ; 3,73 m, 4H ($CH_2$) ; 4,83 s, 2H ($COCH_2O$) ; 6,78-7,03 dd, 1 H (éthylénique) ; 7,16-7,28 m, 7H (Ar) ; 8,07 s, 3H ($NH_3^+$) ; 9,59 s, 1H (NH) ; 10,86 s, 1H (NH).

**Exemple 14:** Chlorhydrate de la 2-[3-(2-amino-éthyl)-1H-indol-5-yloxy]-1-[4-(2-nitro-phényl)-pipérazin-1-yl)éthanone.

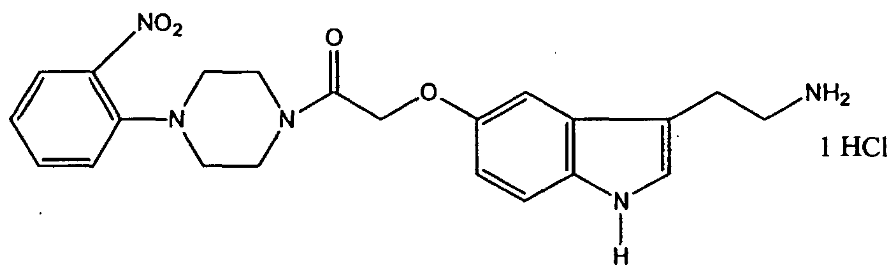

Le composé 14 est obtenu à partir de la 2-nitrophényl-pipérazine (15 g ; 72,5 mmol), de chlorure de chloracétyle (5,78 ml ; 72,56 mmol) et du composé 1A (10,9 g ; 39,6 mmol) selon la procédure décrite pour la préparation de l'exemple 1.
La purification du produit sous forme de base est effectuée par chromatoraphie sur colonne de gel de silice éluée par un mélange chloroforme/méthanol/ammoniaque (80:19:1, v/v). Le produit pur est isolé sous forme de sirop jaune qui conduit, après traitement à l'acide chlorhydrique dans l'éther, au composé 14 (22 g ; 67 %).
Analyse élémentaire ($C_{22}H_{26}N_5O_4Cl$,13 $H_2O$), % calculés : C 54,67 ; H 5,96 ; N 14,49 ; Cl 7,33; % trouvés : C 54,63 ; H 5,74 ; N 14,25 ; Cl 7,65
RMN 1H, DMSO-d6 (ppm): 3,00 m, 8H ($CH_2$); 3,63 s, 4H ($CH_2$), 4,81 s, 2H ($COCH_2O$); 6,78-6,83 dd, 1H (éthylénique); 7,15-7,87 m, 7H (Ar); 8,00 s, 3H ($NH_3^+$) ; 10,85 s, 1H (NH).
Point de fusion : 130 °C.

**Exemple 15:** Chlorhydrate de la 2-[3-(2-amino-éthyl)-1H-indol-5-yloxy]-1-[4-(4-nitro-phényl)-pipérazin-1-yl)éthanethione.

**Exemple 15:** Chlorhydrate de la 2-[3-(2-amino-éthyl)-1H-indol-5-yloxy]-1-[4-(4-nitro-phényl)-pipérazin-1-yl)éthane-thione.

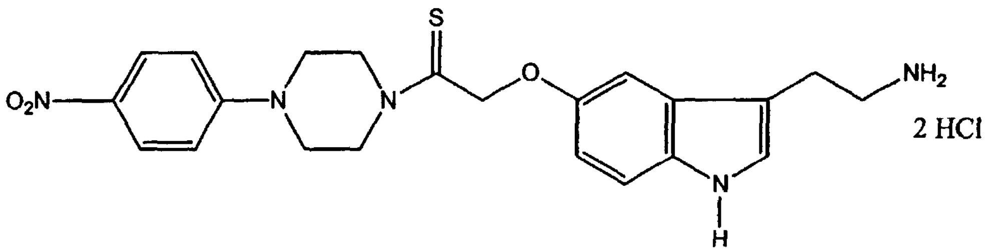

2 HCl

Le composé 1, sous forme de base, (600 mg ; 1,42 mmol) en solution dans le toluène (12 ml) en présence du réactif de Lawesson (401 mg ; 0,99 mmol) est chauffé à reflux pendant 3 heures. Le mélange est ramené à température ambiante, dilué au dichlorométhane et lavé à l'eau (2 fois). La phase organique est séchée sur sulfate de sodium, filtrée et évaporée à sec. Le sirop obtenu est chromatoraphié sur colonne de gel de silice éluée par un mélange chloroforme/méthanol/ammoniaque (80:18,5:1,5, v/v). Le produit pur est isolé sous forme de sirop qui conduit, après traitement à l'acide chlorhydrique dans l'éther, au composé 15 (378 mg ; 52 %).

**Exemple 16:** Chlorhydrate de la 2-[3-(2-amino-éthyl)-1H-indol-5-yloxy]-1-[4-(4-hydroxyamino-phényl)-pipérazin-1-yl) éthanone.

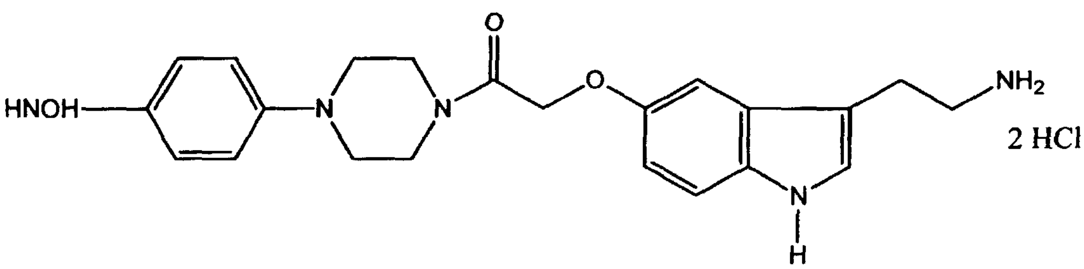

2 HCl

Le composé 1C, (5 g ; 9,54 mmol) en solution dans le THF (120 ml) en présence d'une quantité calatytique de Rhodium sur alumine (490 mg ; 0,48 mmol) est traité à 0°C par l'hydrate d'hydrazine (1,16 ml ; 23,8 mmol). Le mélange est agité à température ambiante pendant 4 heures puis le catalyseur est filtré sur célite ; le milieu est dilué au dichlorométhane, et lavé à l'eau. La phase organique est séchée au sulfate de sodium, filtrée et évaporée à sec. Le sirop obtenu est chromatographié sur colonne de gel de silice éluée par un mélange dichlorométhane/acétate d'éthyle (5:1 puis 2:1 ; v/v). Le produit pur est obtenu sous forme de sirop jaune-vert.

La purification du produit sous forme de base est effectuée par chromatographie sur colonne de gel de silice éluée par un mélange chloroforme/méthanol/ammoniaque (90:9,5:0,5, v/v). Le produit pur est isolé sous forme de sirop qui conduit, après traitement à l'acide chlorhydrique dans l'éther, au composé 16 (780 mg ; 21 %).

**Exemple 17:** Chlorhydrate de la 2-{5-[4-(4-nitro-phényl-pipérazine-1-sulfonylméthoxy]-1H-indol-3-yl}-éthylamine.

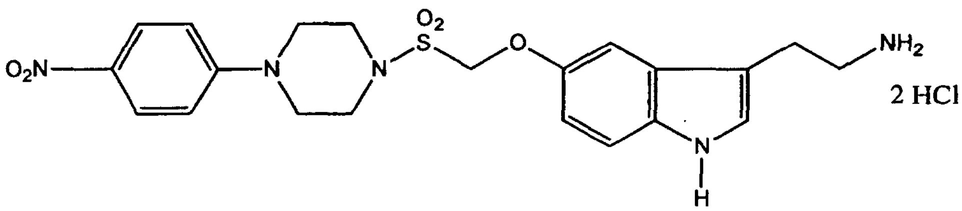

Le composé 17 est obtenu à partir de la 4-nitrophényl-pipérazine (1 g ; 4,82 mmol), de chlorure de chlorométhanesulfonyl (719 mg ; 4,82 mmol) et du composé 1A (739 mg ; 2,63 mmol) selon la procédure décrite pour la préparation de l'exemple 1. Le produit pur est isolé sous forme de sirop qui conduit, après traitement à l'acide chlorhydrique dans l'éther, au composé 17 (420 mg ; 30 %).

**Exemple 18:** Chlorhydrate de la 2-[3-(2-amino-éthyl)-1H-indol-5-yloxy]-1-[4-(4-nitro-phényl)-pipérazin-1-yl]-propane-1-one.

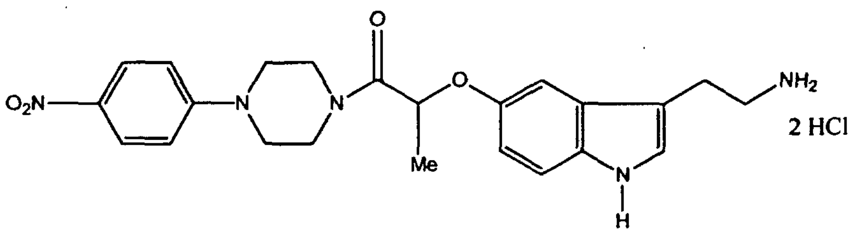

Le composé 18 est obtenu à partir de la 4-nitrophényl-pipérazine (1 g ; 4,82 mmol), de chlorure d' α-méthyl-chloroacétyle (0,46 ml ; 4,82 mmol) et du composé 1A (739 mg ; 2,63 mmol) selon la procédure décrite pour la préparation de l'exemple 1. Le produit pur est isolé sous forme de sirop qui conduit, après traitement à l'acide chlorhydrique dans l'éther, au composé 18 (470 mg ; 35 %).

**Exemple 19:** Chlorhydrate de la 2-[3-(2-diméthylamino-éthyl)-1H-indol-5-yloxy]-1-[ 4-(4-nitro-phényl)-pipérazin-1-yl]-éthanone.

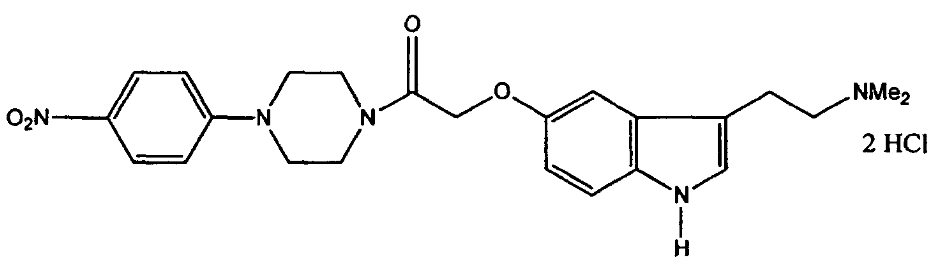

Le composé 19 est obtenu à partir de la 4-nitrophényl-pipérazine (200 mg ; 0,96 mmol), de chlorure de chloroacétyle (76 ml ; 0,96 mmol) et de bufoténine (108 mg; 0,53 mmol) selon la procédure décrite pour la préparation de l'exemple 1. Le produit pur est isolé sous forme de sirop qui conduit, après traitement à l'acide chlorhydrique dans l'éther, au composé 19 (69 mg ; 25 %).

**Exemple 20:** chlorhydrate de la 2-[3-(2-amino-éthyl)-1H-indol-5-yloxy]-1-[4-(4-diméthylamino-phényl)-pipérazin-1-yl]-éthanone.

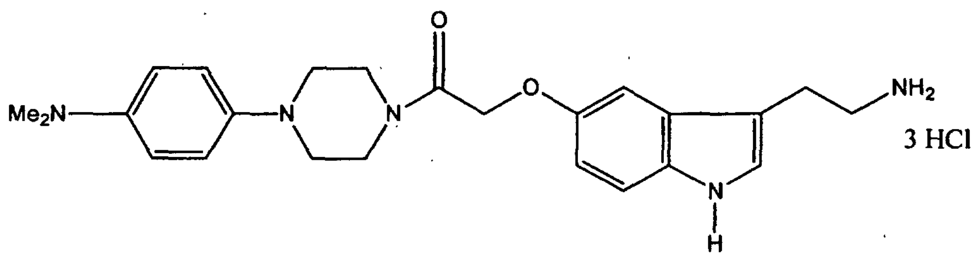

Le produit 2A (800 mg ; 1,62 mmol) en solution dans le tétrahydrofuranne (27 ml) en présence de bromure de tétrabutylammonium (157 mg ; 0,486 mmol) et de soude (6N) (2,7 ml ; 16,2 mmol) est traité par le diméthylsulfate (0,61 ml ; 6,48 mmol). Après 2 h 30 d'agitation à température ambiante le milieu est dilué au dichlorométhane et lavé à l'eau. La phase organique est séchée sur sulfate de sodium, filtrée et évaporée à sec.

Le sirop obtenu est chromatoraphié sur colonne de gel de silice éluée par un mélange dichlorométhane/acétone (4:1 ; v/v).

Ce produit est ensuite déprotégé selon la méthode décrite pour la préparation de l'exemple 2.

Le produit pur est isolé sous forme de sirop qui conduit, après traitement à l'acide chlorhydrique dans l'éther, au composé 20 (344 mg ; 40 %).

**Exemple 21 :** Chlorhydrate de la N-[4-(4-{2-[3-(2-amino-éthyl)-1H-indol-5-yloxy]-acétyl}-pipérazin-1-yl)-phényl]-benzénesulfonamide.

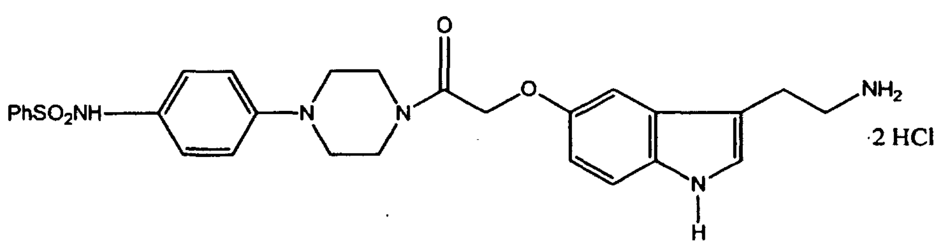

Le composé 21 est obtenu à partir du produit 2A (800 mg ; 1,62 mmol) et de chlorure de benzènesulfonic (0,21 ml ; 1,62 mmol) selon la procédure décrite pour la préparation de l'exemple 3.

La purification du produit sous forme de base est effectuée par chromatoraphie sur colonne de gel de silice éluée par un mélange chloroforme/méthanol/ammoniaque. (80:18,5:1,5 ; v/v). Le produit pur est isolé sous forme de sirop qui conduit, après traitement à l'acide chlorhydrique dans l'éther, au composé 21 ( 450 mg ; 47 % ).

Analyse élémentaire : ($C_{28}H_{32}N_5O_4S$ Cl, 1,2 $H_2O$), % calculés : C 56,83 ; H 5,86; N 11,84 ; % trouvés : C 56,66 ; H 5,56 ; N 11,67

RMN 1H, DMSO -d6 (ppm) : 2,98-3,02 m, 8H ($CH_2$) ; 3,60 m, 4H (CH2) ; 4,79s, 2H ($COCH_2O$) ; 6,75-7,72 m, 13 H (Ar + éthylénique) ; 7,99 s, 3H ($NH3^+$) ; 9,90 s, 1H (NH) ; 10,84 s, 1H (NH)

Point de fusion : 263°C

**Exemple 22** : Chlorhydrate de la 2-[3-(2-amino-éthyl)-1H-indol-5-yloxy]-1-[4-(2-méthoxy-4-nitro-phényl)-pipérazin-1-yl]-éthanone.

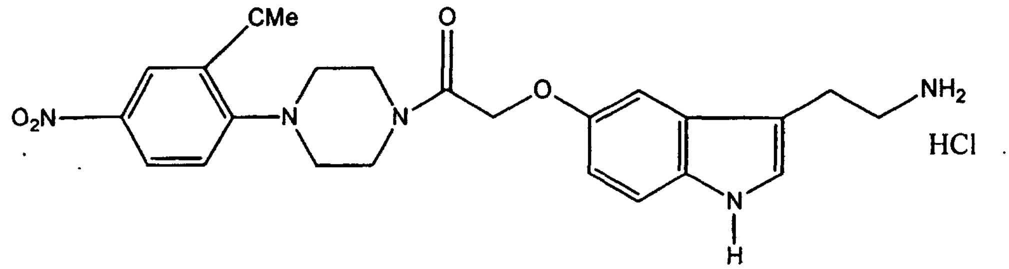

Le composé 22 est obtenu à partir de la 2-méthoxy-4-nitrophényl-pipérazine (1,64 g ; 6,92 mmol), de chlorure de chloracétyle (0,55 ml ; 6,92 mmol) et du composé 1A (1,06 g ; 3,8 mmol) selon la procédure décrite pour la préparation de l'exemple 1. La purification du produit sous forme de base est effectuée par chromatoraphie sur colonne de gel de silice éluée par un mélange chloroforme/méthanol/ammoniaque (80:19:1 ; v/v). Le produit pur est isolé sous forme de sirop jaune qui conduit, après traitement à l'acide chlorhydrique dans l'éther, au composé 22 ( 747 mg, 71 %).
Analyse élémentaire
RMN 1H, DMSO -d6 (ppm)

**Exemple 23 :** Chlorhydrate de la 2-(5-{2-[4-(4-nitro-phényl)pipérazin-1-yl]-éthoxy}-1 H-indol-3-yl)-éthylamine.

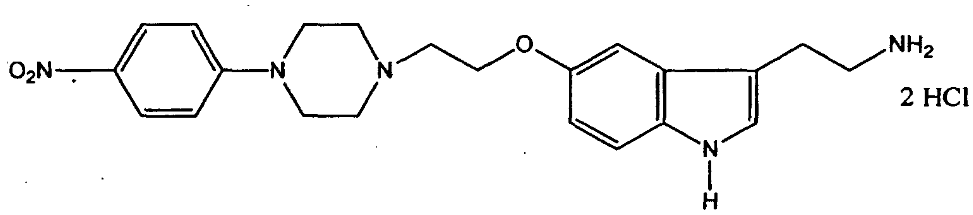

**Exemple 23A :** 2-[5-(2-chloro-éthoxy)-1H-indol-3-yl]-N-(terbutoxycarbonyl)amino-éthyle.

Le produit 1A (5 g ; 18,09 mmol) en solution dans la méthyléthylcétone (25 ml), en présence de carbonate de potassium (15 g ; 108,5 mmol), est traité par le 1-bromo-2-chloro-éthane. Après 24 heures à reflux le milieu est dilué au dichlorométhane, filtré sur célite et évaporé à sec. Le sirop brun obtenu est chromatographié sur colonne de gel de silice éluée par un mélange dichlorométhane/acétone (30:1, v/v). Le produit pur est obtenu sous forme de cristaux blancs (5,2 g ; 84 %).
Analyse élémentaire ($C_{17}H_{23}N_2O_3Cl$), % calculée : C 60,26 ; H 6,84 ; N 8,27. % trouvés : C 60,37 ; H 6,98 ; N 8,21.
RMN 1H, $CDCl_3$ (ppm) : 1,46 s, 9H (tBu) ; 2,88-2,95 t, 2H ($CH_2$) ; 3,45 m, 2H ($CH_2$) ; 3,81-3,87 t, 2H ($CH_2$) ; 4,26-4,32 t, 2H ($CH_2$) ; 4,65 s, 1H (NH) ; 6,87-6,93 dd, 1H (éthylénique) ; 7,01-7,29 m, 3H (Ar) ; 8,16 s, 1H (NH).
Point de fusion : 129°C.

**Exemple 23B** : Chlorhydrate de la 2-(5-{2-[4-(4-amino-phényl)pipérazin-1-yl]-éthox }-1H-indol-3-yl)-éthylamine.

Un mélange du produit 23A (1,03 g ; 3,07 mmol) et de 4-nitrophényl-pipérazine (636 mg ; 3,07 mmol) dans le diméthylformamide (1,5 ml) en présence de carbonate de potassium (1,27 g ; 9,3 mmol) et d'iodure de potassium (166 mg ; 0,3 mmol) est chauffé à 80°C pendant 31 heures. Le milieu est alors dilué à l'acétate d'éthyle, lavé à l'eau puis par une solution saturée de chlorure de sodium. La phase organique est séchée sur sulfate de sodium, filtrée et évaporée à sec. Le solide jaune obtenu est purifié sur colonne de gel de silice éluée par un mélange dichlorométhane/méthanol (30:1, v/v). Le produit pur est isolé sous forme de solide jaune (1,46 g ; 94 %).
Ce produit est déprotégé selon la méthode décrite pour la préparation de l'exemple 2.

Le produit est chromatographié sur colonne de gel de silice éluée par un mélange dichlorométhane/méthanol/ammoniaque (80:18,5:1,5 ; v/v).

Le produit pur est isolé sous forme de sirop jaune qui conduit, après traitement à l'acide chlorhydrique dans l'éther, au composé 23 (1,17 g ; 78 %).

Analyse élémentaire ($C_{22}H_{29}N_5O_3Cl_2$ ; $1,5H_2O$), % calculés : C 51,87 ; H 6,33; N 13,75. % trouvés : C 52,41 ; H 6,18 ; N 13,68.
RMN 1H, DMSO-d6 (ppm) : 3,01-3,58 m, 12H ($CH_2$) ; 4,19 m, 2H ($CH_2$) ; 4,48 m, 2H ($CH_2$) ; 6,79-6,84 dd, 1H (éthylénique) ; 7,11-7,31 m, 5H (Ar) ; 8,09-8,13 m, 5H (Ar + $NH_3^+$) ; 10,91 s, 1H (NH) ; 11,74 s, 1H ($NH^+$).
Point de fusion: 148°C.

**Exemple 24 :** chlorhydrate de la 3-[3-(2-amino-éthyl)-1H-indol-5-yl]-1-[4-(4-nitro-phényl)-pipérazin-1-yl]-prop-2-en-1-one.

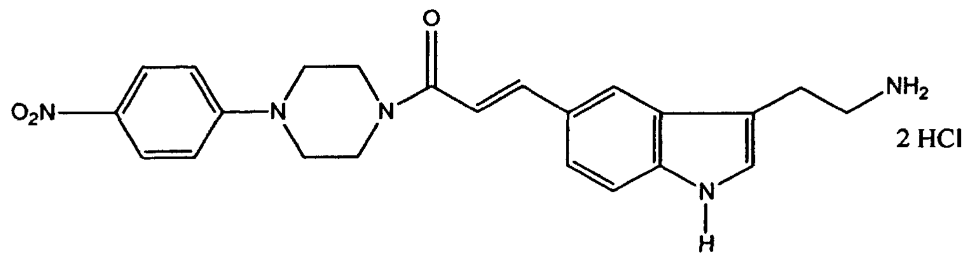

**Exemple 24A :** 2-(5-bromo-1H-indol-3-yl)-N-(terbutoxycarbonyl)-éthylamine.

La 5-bromo-tryptamine (43 g ; 180 mmol) en solution dans le tétrahydrofuranne (500 ml) en présence de soude (2N) (260 ml) est traité par le diterbutyle dicarbonate (60 g; 275 mmol) à température ambiante.

Après une nuit d'agitation à température ambiante, le milieu est dilué à l'acétate d'éthyle, lavé à l'eau puis par une solution saturée de chlorure de sodium. La phase organique est séchée sur sulfate de sodium, filtrée et évaporée à sec. Le sirop obtenu est chromatographié sur colonne de gel de silice éluée par un mélange toluène/acétate d'éthyle (80:20 ; v/v). Le produit pur est obtenu sous forme de sirop qui cristallise dans l'éther de pétrole pour donner une poudre beige (27 g ; 44%).
Point de fusion: 103°C.

**Exemple 24B :** 3-[3-(2-N-terbutoxycarbonyl amino-éthyl)-1H-indol-5-yl]-acrylate de méthyle.

Le produit 24A (5 g ; 14,7 mmol) est chauffé en présence d'acrylate de méthyle (2 ml; 22,1 mmol), de tréthylamine (10 ml), de tri-o-tolylphosphine (90 mg ; 0,29 mmol) et d'acétate de palladium (33 mg ; 1,47 mmol) à 100°C, dans un tube à vis.
Après une nuit d'agitation les mêmes quantités de tri-o-tolylphosphine et d'acétate de palladium sont ajoutées. Après 5 heures le milieu est dilué à l'acétate d'éthyle et le précipité formé est filtré sur célite. Le filtrat est lavé à l'eau puis par une solution saturée de chlorure de sodium. La phase organique est séchée sur sulfate de sodium, filtrée et évaporée à sec. Le sirop obtenu est chromatographié sur colonne de gel de silice éluée par un mélange dichlorométhane/acétate d'éthyle (15:1 puis 10:1 ; v/v). Le produit pur est obtenu sous la forme d'un sirop jaune pâle (4,36 g ; 86 %).
Analyse élémentaire ($C_{19}H_{24}N_2O_4$) ; % calculés : C 66,26 ; H 7,02 ; N 8,13 ; % trouvés : C 65,40 ; H 6,74 ; N 7,79.
RMN 1H, $CDCl_3$ (ppm) : 1,44 s, 9H (tBu) ; 2,92-2,99 m, 2H ($CH_2$) ; 3,45-3,48 m, 2H ($CH_2$) ; 3,82 s, 3H (COOMe) ; 4,64 s, 1H (NH) ; 6,39-6,47 d, 1H (éthylénique); 7,05-7,89 m, 5H (Ar + éthyléniques) ; 8,31 s, 1H (NH).

**Exemple 24C** : 3-[3-(2-N-{terbutoxycarbonyl}-amino-éthyl)-1H-indol-5-yl]-acrylic acide.

Le produit 24B (2 g ; 5,80 mmol) en solution dans l'éthanol (20 ml) et l'eau (0,2 ml) est traité par la potasse en pastilles (650 mg ; 11,6 mmol). Après 3 heures à reflux le milieu est dilué à l'acétate d'éthyle, lavé par une solution normale d'acide chlorhydrique, à l'eau puis par une solution saturée de chlorure de sodium. La phase organique est séchée sur sulfate de sodium, filtrée et évaporée à sec. Le produit 24C est obtenu pur sous la forme d'une poudre blanche (1,85 g ; 97 %).
Point de fusion : 179-181°C.

**Exemple 24:** chlorhydrate de la 3-[3-(2-amino-éthyl)-1H-indol-5-yl]-1-[4-(4-nitro-phényl)-pipérazin-1-yl]-prop-2-en-1-one.

Le produit 24C (500 mg ; 1,51 mmol) en solution dans le dichlorométhane anhydre (18 ml) en présence de N-méthylmorpholine (0,18 ml ; 1,66 mmol) est traité à -15°C, sous azote, par le chloroformiate d'éthyle (0,16 ml ; 1,66 mmol). Après 15 minutes d'agitation à -10°C, la 4-nitrophényl-pipérazine (781 mg ; 3,77 mmol) est additionnée et le mélange est agité de -10°C à température ambiante pendant 1 heure. Le milieu est alors dilué au dichlorométhane, lavé par une solution saturée de bicarbonate de sodium, à l'eau puis par une solution saturée de chlorure de sodium. La phase organique est séchée sur sulfate de sodium, filtrée et évaporée à sec. Le sirop obtenu est chromatographié sur colonne de gel de silice éluée par un mélange dichlorométhane/méthanol (40:1 ; v/v). Le produit pur est isolé sous forme de sirop (564 mg ; 72 %). Ce produit est ensuite déprotégé selon la méthode décrite pour la préparation de l'exemple 2.

Le produit pur est isolé sous forme de sirop qui conduit, après traitement à l'acide chlorhydrique dans l'éther, au composé 24 (277 mg ; 50 %).

**Exemple 25:** Chlorhydrate de la 3-[3-(2-amino-éthyl)-1H-indol-5-yl]-1-[4-(4-nitro-phényl)-pipérazin-1-yl]-propan-1-one.

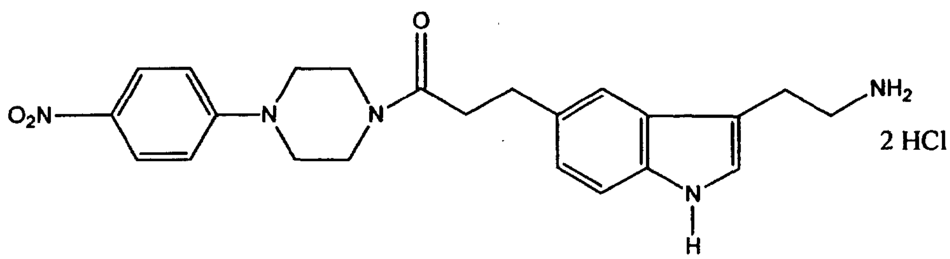

**Exemple 25A:** 3-[3-(2-N-{terbutoxycarbonyl}-amino-éthyl)-1H-indol-5-yl]-propionic acide.

Le produit 24C (500 mg ; 1,51 mmol) en solution dans le méthanol (4 ml) en présence d'une quantité catalytique de palladium sur charbon (30 mg) est hydrogéné par l'hydrogène gazeux. Après 1 h 30 à température ambiante, le milieu est dilué au dichlorométhane, filtré sur célite et évaporé à sec. Le produit pur est obtenu par recristallisation dans l'éther pour donner une poudre beige (483 mg ; 96%).
Analyse élémentaire ($C_{18}H_{24}N_2O_4$) ; % calculés : C 65,04 ; H 7,28 ; N 8,43 ; % trouvés : C 64,57 ; H 7,35 ; N 8,25.
RMN 1H, DMSO-d6 (ppm) : 1,38 s, 9H (tBu) ; 2,50-3,40 m, 8H ($CH_2$) ; 6,87-7,32 m, 4H (Ar + éthylénique) ; 10,68 s, 1H (NH) ; 12,05 s, 1H (NH).

**Exemple 25:** Chlorhydrate de la 3-[3-(2-amino-éthyl)-1H-indol-5-yl]-1-[4-(4-nitro-phényl)-pipérazin-1-yl]-propan-1-one.

Le composé 25 est obtenu à partir du composé 25A (640 mg ; 1,92 mmol) et de 4-nitrophénylpipérazine (981 mg ; 4,73 mmol) selon la méthode décrite pour la préparation de l'exemple 24 à partir de 24C. Le produit pur est obtenu sous forme de sirop qui conduit, après traitement à l'acide chlorhydrique dans l'éther, au composé 25 (531 mg ; 56 %).
Analyse élementaire ($C_{23}H_{29}Cl_2N_5O_3$, 1,7$H_2O$), % calculés: C 52,62 ; H 6,22 ; N 13,34 ; Cl 13,50 ; % trouvés : C 52,56 ; H 5,93 ; N 13,16 ; Cl 14,72
RMN 1H, DMSO-d6 (ppm) : 2,69 m, 2H ; 2,88-3,00 m, 6H ; 3,42 m, 4H ; 3,60 large s, 4H ; 6,94-7,42 m, 6H ; 8,05 d, 2H ; 8,14 s, 3H ; 10,88 s, 1H
Point de fusion: 145°C

**Exemple 26:** Chlorhydrate de la N-[4-(4-{2-[3-(2-amino-éthyl)-1H-indol-5-yloxy]-acétyl}-pipérazin-1-yl)-phényl]-4-nitrophénylsulfonamide.

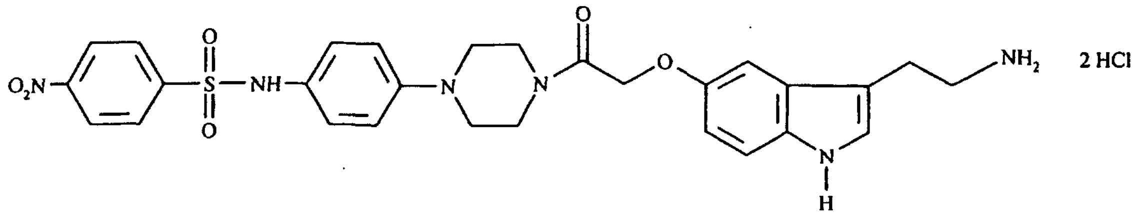

**Méthode A :**

Le composé **26** est obtenu à partir du produit **2A** (768 mg ; 1,55 mmol) et de chlorure de 4-nitrobenzènesulfonyle (689 mg ; 3,10 mmol) selon la procédure décrite pour la préparation de l'exemple **3.**

La purification du produit, sous forme de base, est effectuée par chromatographie sur colonne de gel de silice éluée par un mélange dichlorométhane/méthanol/ammoniaque (80; 18,5; 1,5 ; v/v). Le produit pur est isolé sous forme de sirop jaune pâle qui conduit, après traitement à l'acide chlorhydrique dans l'éther, au compose **26** (391 mg; 44 %).
Analyse élémentaire ($C_{28}H_{32}N_6O_6S_1$-2,5$H_2O$), % calculés: C 48,28 ; H 5,35 ; N 12,06 ; Cl 10,18, % trouvés : C 48,09 ; H 5,05 ; N 11, 84 ; Cl 9,85
RMN 1H, DMSO-d6 (ppm) : 2,96-3,14 m, 8H; 3,62 m, 4H; 4,78 s, 2H; 6,73-6,79 dd, 1H; 6,95-7,26 m, 7H; 7,89-7,94 m, 5H ; 8,33-8,37 d, 2H ; 10,31 s, 1H ; 10,83 s, 1H
Point de fusion : 190°C
Spectre de masse (DCI/$NH_3$): m/z 579 (M+H)

**Méthode B**

**26A** -4-(4-terbutoxycarbonyl-pipérazin-1-yl)-phénylamine

La 1-(4-nitrophényl)-pipérazine (50 g ; 241,2 mmol) en solution dans le dichlorométhane (11) en présence de triéthylamine (50,3 ml ; 361,8 mmol) est traité par le diterbutyle dicarbonate (63,2 g ; 289,4 mmol) à température ambiante, pendant 1 heure. Le milieu est ensuite dilué au dichlorométhane et lavé à l'eau. La phase organique est séchée sur sulfate de sodium, filtrée et évaporée à sec.

Le produit brut obtenu est repris dans le méthanol et est hydrogéné sous pression atmosphérique en présence d'une quantité catalytique de palladium sur charbon (à 5%) (2 g ; 0,94 mmol).

Après 28 h d'agitation à température ambiante, le mélange est filtré sur célite et évaporé à sec. Le sirop obtenu est chromatographié sur colonne de gel de silice éluée par un mélange hexane/acétate d'éthyle (1;1 ; v/v). Le produit pur est isolé sous forme de poudre rose pâle (57 g ; 85 %).
Analyse élémentaire ($C_{15}H_{25}N_3O_2$), % calculés: C 64,95 ; H 8,35 ; N 15,14 , % trouvés : C 64,95 ; H 8,31 ; N 14,89
RMN 1H, DMSO-d6 (ppm) : 1,42 s, 9H ; 2,83 t, 4H ; 3,42 t, 4H ; 4,62 s, 2H ; 6,49 d, 2H ; 6,70 d, 2H
Point de fusion : 96°C

**26B -** N-[4-(4-terbutoxycarbonyl-pipérazin-1-yl-phényl)-4-nitro-benzènesulfonamide

Le composé **26A** (37,6 g ; 135,5 mmol) en solution dans le dichlorométhane (1 l) en présence de triéthylamine (20,7 ml ; 149,1 mmol) est traité à 0°C par le chlorure de 4-nitrobenzènesulfonyle (30,04 g ; 135,5 mmol). Après 3h30 d'agitation de 0°C à température ambiante du chlorure de 4-nitrobenzènesulfonyle (9,1 g ; 40,6 mmol) est à nouveau additionné. Après 2h30 la réaction est diluée au dichlorométhane, lavée à l'eau, séchée sur sulfate de sodium, filtrée et évaporée à sec. Le sirop vert foncé obtenu est repris dans le méthanol à chaud puis refroidi afin d'obtenir une cristallisation du produit attendu, que l'on isole par filtration sur büchner et lavage à l'éther. Obtention d'une poudre jaune (50 g; 80%) engagée directement dans la réaction suivante.

**26C** - 4-nitro-N-(4-pipérazin-1-yl-phényl)-benzènesulfonamide

Le produit précédent (50 g ; 108,1 mmol) en solution dans le toluène (1,3 1) est traité, à température ambiante,

par l'acide trifluoroacétique (180 ml). Après 3 h 30 le milieu est évaporé à sec et coévaporé 5 fois au toluène. Le sirop obtenu est purifié sur colonne de silice éluée par un mélange dichlorométhane/méthanol/ammoniaque (90:9,5:0,5 ; v/v) puis (85:14:1 , v/v). Le produit **26C** et isolé sous forme de poudre jaune pâle (29 g ; 75%).
RMN 1H, DMSO-d6 (ppm) : 3,20 m, 8H ; 6,88 m, 4H ; 7,93 d, 2H ; 8,37 d, 2H ; 9,29 s, 1H
Point de fusion : 240°C

**26D -** N-[4-(4-{2-[3-{2-{N-terbutoxycarbonyl}-amino-éthyl)-1H-indol-5-yloxy]-acétyl}-pipérazin-1-yl)-phényl]-4-nitrophénylsulfonamide

L'acide [3-(2-{N-terbutoxycarbonyl}-amino-éthyl)-1H-indol-5-yloxy]-acétique, préparé selon une méthode décrite précédemment (demande de brevet d'invention FR2699918-A), (1 g ; 2,99 mmol) en solution dans le dichlorométhane anhydre (50 ml) en présence de N-méthylmorpholine (0,45 ml ; 4,5 mmol) est traité, à -15°C et sous azote, par le chloroformate d'éthyle (0,37 ml ; 3,9 mmol).

Après 30 minutes d'agitation le composé **26C** (2,2 g , 6,0 mmol) est additionné et le mélange est agité de -15°C à température ambiante pendant 4 heures. Le milieu est dilué à l'acétate d'éthyle et lavé par une solution saturée de bicarbonate de sodium, puis à l'eau et enfin par une solution saturée de chlorure de sodium. La phase organique est séchée sur sulfate de sodium, filtrée et évaporée à sec.

Le sirop obtenu est chromatographié sur colonne de gel de silice éluée par un mélange dichlorométhane/acétate d'éthyle (2:1 puis 1:1, v/v). Le produit **26D** est isolé sous forme de poudre jaune pâle (1,3 g ; 65 %).
Analyse élémentaire ($C_{33}H_{38}N_6O_8$), % calculés: C 58,39 ; H 5,64 ; N 12,38 , % trouvés : C 58,49 ; H 5,61 ; N 12,03
RMN 1H, DMSO-d6 (ppm) : 1,34 s, 9H ; 2,70 t, 2H ; 3,09 m, 6H ; 3,57 s, 4H ; 4,74 s, 2H ; 6,71-7,21 m, 9H ; 7,89 d, 2H ; 8,34 d, 2H ; 10,16 s, 1H ; 10,63 s, 1H.
Point de fusion : 237°C

**26** - Chlorhydrate de la N-[4-(4-{2-[3-(2-amino-éthyl)-1H-indol-5-yloxy]-acétyl}-pipérazin-1-yl)-phényl]-4-nitrophénylsulfonamide

Le composé **26D** (2,76 g ; 4,06 mmol) en suspension dans le toluène (82 ml) est traité, à température ambiante, par l'acide trifluoroacétique (11 ml). Après 3 h d'agitation, le mélange est évaporé à sec et coévaporé 4 fois au toluène. Le sirop obtenu est chromatographié sur colonne de gel de silice éluée par un mélange dichlorométhane/méthanol/ammoniaque (85:14:1, v/v). Le produit pur est isolé sous forme de sirop jaune orangé qui conduit, après traitement à l'acide chlorhydrique dans l'éther, au composé **26** (2,38 g ; 90%).

Les exemples **27** à **34** sont préparés selon la méthode décrite pour la préparation de l'exemple **3** à partir de **2A**.

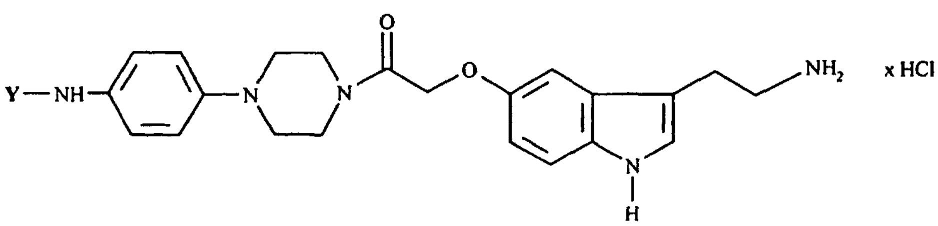

| Exemples | Y | Réactifs | Base/solvant/T°C | Rdt (%) | PtF (°C) | Formules brutes |
|---|---|---|---|---|---|---|
| 27 | 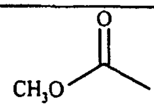 | 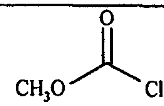 | $Et_3N/CH_2Cl_2$/50°C | 16 | 244 | $C_{24}H_{29}N_5O_4$.2HCl |
| 28 | 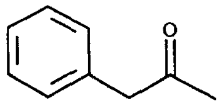 | 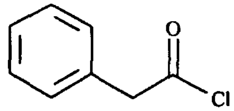 | $Et_3N/CH_2Cl_2$/25°C | 31 | 176 | $C_{30}H_{33}N_5O_3$.2HCl |
| 29 | 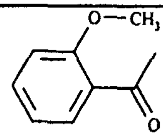 | 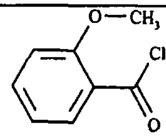 | pyridine/25°C | 46 | 192 | $C_{30}H_{33}N_5O_4$.2HCl |
| 30 | 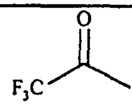 | $(CF_3CO)_2O$ | $Et_3N/CH_2Cl_2$/25°C | 20 | 183 | $C_{24}H_{26}N_5O_3F_3$.2HCl |
| 31 | $Me_2N-SO_2-$ | $Me_2N-SO_2Cl$ | pyridine/25°C | 47 | 170 | $C_{24}H_{32}N_6O_4S_1$.2HCl |
| 32 | 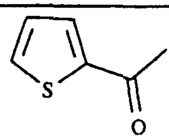 | 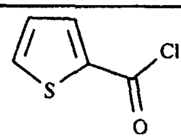 | pyridine/25°C | 51 | 234 | $C_{27}H_{29}N_5O_3S_1$.2HCl |
| 33 | 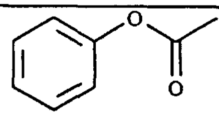 | 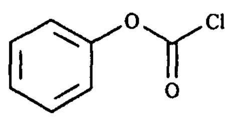 | $Et_3N/CH_2Cl_2$/25°C | 28 | 167 | $C_{29}H_{31}N_5O_4$.2HCl |
| 34 | 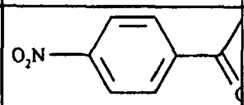 | 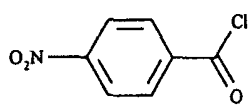 | pyridine/25°C | 45 | 190 | $C_{29}H_{30}N_6O_5$.2HCl |

**Exemple 35 :** Chlorhydrate de 3-[3-(2-amino-éthyl)-1H-indol-5-yl]-1-[4-(4-amino-phényl)-pipérazin-1-yl]-propan-1-one

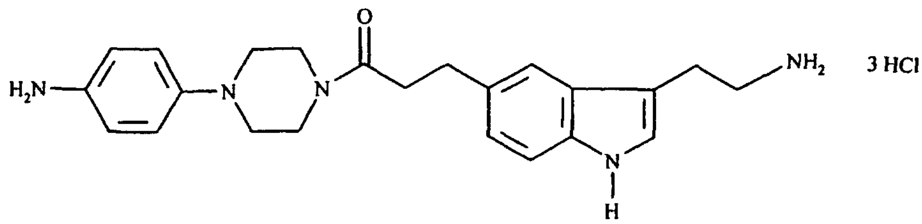

**35A** -3-[3-(2-{N-terbutoxycarbonyl}-amino-éthyl)-1H-indol-5-yl]-1-[4-(4-amino-phényl)-pipérazin-1-yl]-propan-1-one

Le composé **35A** est préparé à partir du composé **25** (5,8 g ; 11,1 mmol) dans les conditions décrites pour la préparation de l'exemple **2** à partir de **1C**. La mousse rose obtenue (5,24 g ; 96 %) est utilisée brute pour la réaction suivante.

**35 -** Chlorhydrate de 3-[3-(2-amino-éthyl)-1H-indol-5-yl]-1-[4-(4-amino-phényl)-pipérazin-1-yl]-propan-1-one

Le produit **35A** (575 mg ; 1,17 mmol) est ensuite déprotégé selon la méthode décrite pour la préparation de l'exemple **2** à partir de **2A.**

La purification du produit, sous forme de base, est effectuée par chromatographie sur colonne de gel de silice éluée par un mélange chloroforme/méthanol/ammoniaque (80:18:2 ; v/v). Le produit pur est isolé sous forme de sirop incolore qui conduit, après traitement à l'acide chlorhydrique dans l'éther, au composé **35** sous forme de poudre rosée (330 mg ; 72 %).

Analyse élémentaire ($C_{23}H_{32}Cl_3N_5O$, $H_2O$ ), % calculés: C 53,24 ; H 6,60 ; N 13,50 , Cl 20,50; % trouvés : C 53,40 ; H 6,76 ; N 13,53 ; Cl 18,84

RMN 1H, DMSO-d6 (ppm) : 2,70 t, 2H ; 2,88-3,13 m, 10H ; 3,63 s, 4H ; 4,70 large s, 3H ; 6,97-7,42 m, 8H ; 8,15 s, 3H ; 10,90 s, 1H

Point de fusion : 189°C

**Exemple 36:** Chlorhydrate de la N-[4-(4-{3-[3-(2-amino-éthyl)-1H-indol-5-yl]-propionyl}-pipérazin-1-yl)-phényl]-méthanesulfonamide.

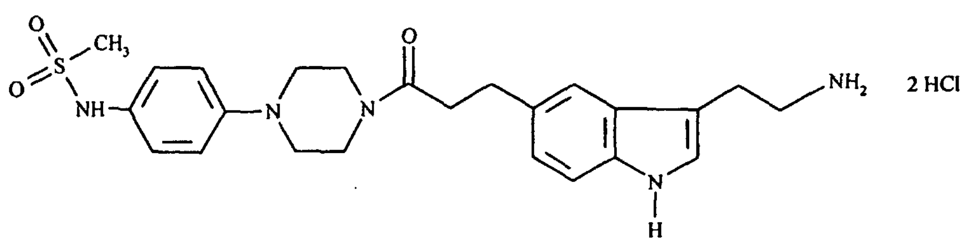

Le composé **36** est préparé à partir du composé **35A** (1,2 g ; 2,43 mmol) et de chlorure de mésyle (0,188 ml ; 2,43 mmol) selon la méthode décrite pour la préparation de l'exemple **3.**

La purification du produit, sous forme de base, est effectuée par chromatographie sur colonne de gel de silice éluée par un mélange chloroforme/méthanol/ammoniaque (80:18:2 ; v/v). Le produit pur est isolé sous forme de sirop jaune pâle qui conduit, après traitement à l'acide chlorhydrique dans l'éther, au composé **36** sous forme de poudre beige (1,06 g; 77 %).

Analyse élémentaire ($C_{24}H_{33}Cl_2N_5O_3S_1$, $H_2O$ ), % calculés : C 51,43 ; H 6,29 ; N 12,49 , Cl 12,65 % trouvés : C 51,66 ; H 6,29 ; N 12,38 ; Cl 10,59

RMN 1H, DMSO-d6 (ppm) : 2,70 t, 2H ; 2,87-3,15 m, 13H ; 3,73 s, 4H ; 6,99 d, 1H ; 7,18-7,42 m, 7H ; 8,13 s, 3H ; 9,70 s, 1H ; 10,89 s, 1H

Point de fusion : 179°C
Spectre de masse ($DCI/NH_3$) : m/z 470 (M + H)

**Exemple 37 :** Chlorhydrate de la N-[4-(4-{3-[3-(2-amino-éthyl)-1H-indol-5-yl]-propionyl}-pipérazin-1-yl)-phényl]-N-diméthylsulfonurée

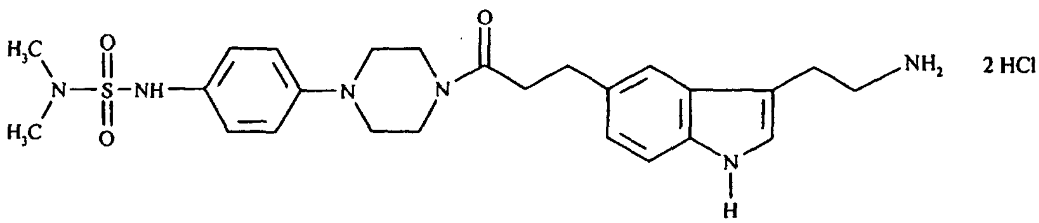

Le composé **37** est préparé à partir du composé **35A** (1,2 g ; 2,43 mmol) et de chlorure de diméthylsulfamoyle (0,39 ml ; 3,64 mmol) selon la méthode décrite pour la préparation de l'exemple **3**.

La purification du produit, sous forme de base, est effectuée par chromatographie sur colonne de gel de silice éluée par un mélange dichlorométhane/méthanol/ ammoniaque (85 ; 14 ; 1 ; v/v). Le produit pur est isolé sous forme de sirop incolore qui conduit, après traitement à l'acide chlorhydrique dans l'éther, au composé **37** sous forme de poudre beige (580 mg; 60 %).
Analyse élémentaire ($C_{25}H_{36}Cl_2N_6O_3S_1$,1,1$H_2O$ ), % calculés : C 50,77 ; H 6,51 ; N 14,21 , Cl 11,99 ; % trouvés : C 50,74 ; H 6,60 ; N 13,89 ; Cl 10,61
RMN 1H, DMSO-d6 (ppm) : 2,66-2,75 m, 8H ; 2,88-3,16 m, 10H ; 3,76 large s, 4H ; 7,00 d, 1H ; 7,15-7,29 m, 6H ; 7,43 s, 1H ; 8,11 s, 3H ; 9,91 s, 1H ; 10,89 s, 1H
Point de fusion : 166°C

**Exemple 38 :** Chlorhydrate de la N-[4-(4-{3-[3-(2-amino-éthyl)-1H-indol-5-yl]-propionyl}-pipérazin-1-yl)-phényl]-4-nitro-benzènesulfonamide.

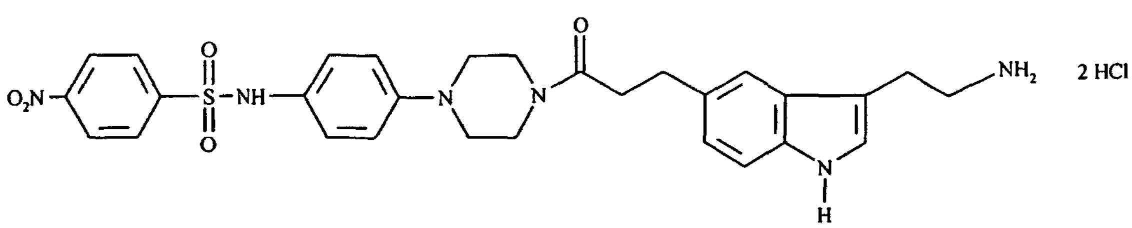

Le composé **38** est préparé à partir du composé **35A** (906 mg ; 1,84 mmol) et de chlorure de 4-nitrobenzènesulfonyle (613 mg ; 2,76 mmol) selon la méthode décrite pour la préparation de l'exemple **3**.

La purification du produit, sous forme de base, est effectuée par chromatographie sur colonne de gel de silice éluée par un mélange dichlorométhane/méthanol/ammoniaque (85:14:1 ; v/v). Le produit pur est isolé sous forme de sirop orange qui conduit, après traitement à l'acide chlorhydrique dans l'éther, au composé **38** sous forme de poudre orange (896 mg; 71 %).
Analyse élémentaire ($C_{29}H_{34}Cl_2N_6O_5S$, 2$H_2O$ ), % calculés: C 50,80 ; H 5,59 ; N 12,26, Cl 10,34 % trouvés : C 50,83 ; H 5,50 ; N 12,11 ; Cl 10,33
RMN 1H, DMSO-d6 (ppm) : 2,78 t, 2H ; 2,90 t, 2H ; 3,00-3,16 m, 8H ; 3,66 large s, 4H ; 6,96-7,41 m, 8H ; 7,97 d, 2H ; 8,09 large s, 3H ; 8,37 d, 2H ; 10,56 s, 1H ; 10,88 s, 1H
Point de fusion : 184°C

**Exemple 39 :** Chlorhydrate de la N-[4-(4-{3-[3-(2-amino-éthyl)-1H-indol-5-yl]-propionyl}-pipérazin-1-yl)-phényl]-benzamide.

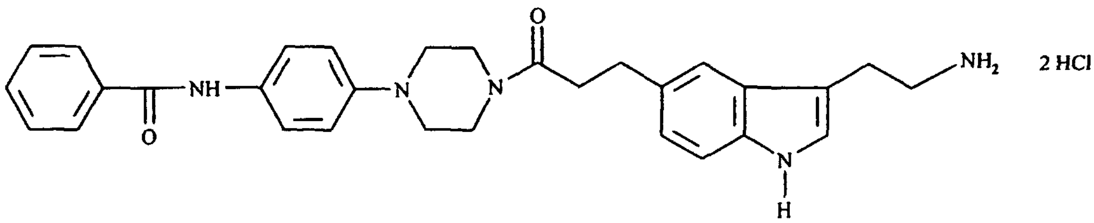

Le composé **39** est préparé à partir du composé **35A** (1,08 g ; 2,18 mmol) et de chlorure de benzoyle (0,25 ml ; 2,18 mmol) selon la méthode décrite pour la préparation de l'exemple **3.**
La purification du produit, sous forme de base, est effectuée par chromatographie sur colonne de gel de silice éluée par un mélange dichlorométhane/méthanol/ammoniaque (85:14:1; v/v). Le produit pur est isolé sous forme de sirop incolore qui conduit, après traitement à l'acide chlorhydrique dans l'éther, au composé **39** sous forme de poudre blanche (869 mg; 68 %).
Analyse élémentaire ($C_{30}H_{35}Cl_2N_5O_2$, $H_2O$ ), % calculés: C 61,43 ; H 6,36 ; N 11,94 , Cl 12,09 % trouvés : C 61,54 ; H 6,17 ; N 11,84; Cl 11,79
RMN 1H, DMSO-d6 (ppm) : 2,72 t, 2H ; 2,93-3,01 m, 6H ; 3,21 m, 4H ; 3,81 large s, 4H ; 7,00 d, 1H ; 7,19-7,55 m, 8H ; 7,81 d, 2H ; 7,97 d, 2H ; 8,14 large s, 3H ; 10,39 s, 1H ; 10,90 s, 1H
Point de fusion : 209°C

**Exemple 40 :** Chlorhydrate du 4-(4-{2-[3-(2-amino-éthyl)-1H-indol-5-yloxy]-éthyl}-pipérazin-1-yl)-phénylamine

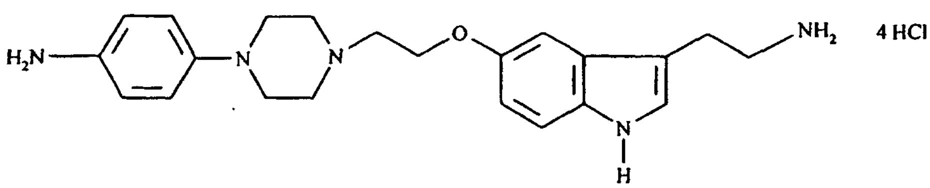

**40A** -4-(4-{2-[3-(2-{N-terbutoxycarbonyl]-amino-éthyl)-1H-indol-5-yloxy]-éthyl}-pipérazin-1-yl)-phénylamine

Le composé **40A** est préparé à partir du composé **23A** (7,1 g ; 13,9 mmol) selon les conditions décrites pour la préparation de l'exemple **2** à partir de **1C**.
Le sirop brun obtenu est chromatographié sur colonne de gel de silice éluée par un mélange dichlorométhane/acétone (1:1 , v/v). Le produit pur est isolé sous forme de mousse rose (6,45 g ; 97 %).
RMN 1H, DMSO-d6 (ppm) : 1,38 s, 9H ; 2,07 d, 2H ; 2,65 m, 4H ; 2,75 m, 4H; 2,93 m, 2H ; 3,09-3,17 m, 4H ; 4,11 t, 2H ; 4,56 s, 1H ; 6,47-6,58 m, 2H ; 6,68-6,75 m, 2H; 6,86-6,90 m, 2H ; 7,06-7,08 m, 2H ; 7,21 d, 1H ; 10,64 s, 1H

**40 -** Chlorhydrate du 4-(4-{2-[3-(2-amino-éthyl)-1H-indol-5-yloxy]-éthyl}-pipérazin-1-yl)-phénylamine

Le produit **40A** (700 mg ; 1,46 mmol) en solution dans le dichlorométhane (12 ml) est traité par une solution (~1,5 M) d'acide chlorhydrique dans l'éther (16 ml). Après 1 heure d'agitation, le mélange est évaporé à sec et le produit formé est désalifié. Le sirop obtenu est chromatographié sur colonne de gel de silice éluée par un mélange dichlorométhane/acétate d'éthyle (80:18,5:1,5 ; v/v). Le produit pur est isolé sous forme de sirop incolore qui conduit, après traitement à l'acide chlorhydrique dans l'éther, au composé **40** sous forme de poudre rose pâle (669 mg; 87 %).
Analyse élémentaire ($C_{22}H_{33}Cl_4N_5O$, $2H_2O$ ), % calculés: C 48,31 ; H 6,52 ; N 12,80, Cl 25,93 % trouvés : C 48,31 ; H 6,65 ; N 12,55 ; Cl 24,95
RMN 1H, DMSO-d6 (ppm) : 3,02 m, 4H ; 3,30 m, 4H ; 3,65 m, 4H ; 3,88 d, 2H; 4,51 large s, 2H ; 6,83 dd, 1H ; 7,09-7,33 m, 7H ; 8,21 large s, 3H ; 10,35 large s, 3H; 10,95 s, 1H ; 11,59 s, 1H

Point de fusion : 200°C (décomposition)

**Exemple 41 :** Chlorhydrate de la N-[4-(4-{2-[3-(2-amino-éthyl)-1H-indol-5-yloxy]-éthyl}-pipérazin-1-yl)-phényl]-méthanesulfonamide.

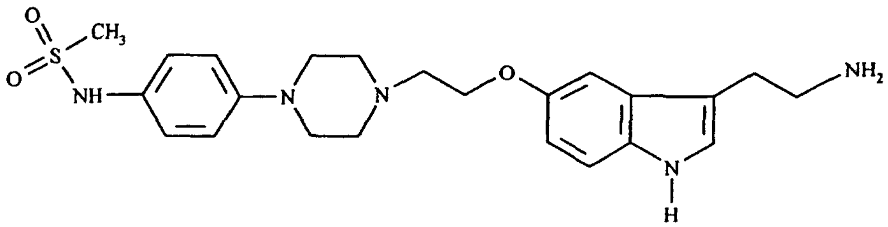

2 HCl

Le composé 41 est préparé à partir du produit **40A** (1 g ; 2,08 mmol) et de chlorure de méthanesulfonyle (0,194 ml ; 2,50 mmol) selon la méthode décrite pour la préparation de l'exemple **3.**

La purification du produit, sous forme de base, est effectuée par chromatographie sur colonne de gel de silice éluée par un mélange dichlorométhane/méthanol/ammoniaque (80:19:1; v/v). Le produit pur est isolé sous forme de sirop jaune pâle qui conduit, après traitement à l'acide chlorhydrique dans l'éther, au composé **41** sous forme de poudre blanche (1,01 g; 92 %).
Analyse élémentaire ($C_{23}H_{33}Cl_2N_5O_3S$, $H_2O$ ), % calculés: C 50,36 ; H 6,43 ; N 12,77 , Cl 12,93 ; % trouvés : C 50,34 ; H 6,16 ; N 12,46 ; Cl 14,31
RMN 1H, DMSO-d6 (ppm) : 2,86 s, 3H ; 3,00 large s, 4H ; 3,15-3,38 m, 4H ; 3,60-3,80 m, 6H ; 4,48 t, 2H ; 6,80 dd, 1H ; 6,97 d, 2H ; 7,12 d, 2H ; 7,20-7,30 m, 3H ; 8,17 large s, 3H ; 9,36 s, 1H ; 10,91 s, 1H ; 11,49 s, 1H
Point de fusion : 230°C (décomposition)

**Exemple 42 :** Chlorhydrate de la N-[4-(4-{2-[3-(2-amino-éthyl)-1H-indol-5-yloxy]-acétyl}-pipérazin-1-yl)-phényl]-méthylcarbamate

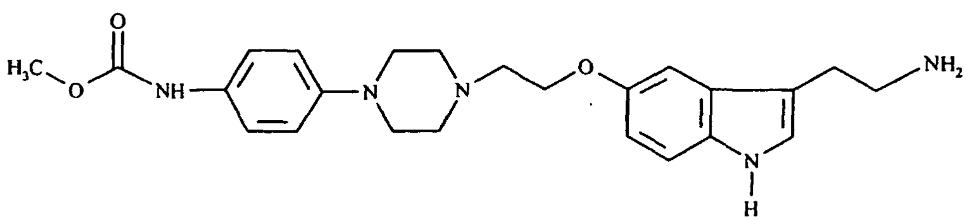

3 HCl

Le composé **42** est préparé à partir du produit **40A** (1,5 g ; 3,13 mmol) et de chloroformate d'éthyle (0,27 ml ; 3,44 mmol) selon la méthode décrite pour la préparation de l'exemple **6.**

La purification du produit, sous forme de base, est effectuée par chromatographie sur colonne de gel de silice éluée par un mélange dichlorométhane/méthanol/ammoniaque (80:18,5:1,5 ; v/v). Le produit pur est isolé sous forme de sirop violet qui conduit, après traitement à l'acide chlorhydrique dans l'éther, au composé **42** sous forme de poudre mauve (612 mg; 36 %).
Analyse élémentaire ($C_{24}H_{34}Cl_3N_5O_3$, $0,8H_2O$ ), % calculés: C 51,35 ; H 6,35 ; N 12,48 , Cl 18,95 ; % trouvés : C 51,30 ; H 6,37 ; N 12,13 ; Cl 19,02
RMN 1H, DMSO-d6 (ppm) : 3,00-3,30 m, 6H ; 3,61 s, 3H ; 3,68-3,90 m, 8H ; 4,48 t 2H ; 6,80 dd, 1H ; 6,94 d, 2H ; 7,21-7,35 m, 5H ; 8,17 large s, 3H ; 9,44 s, 1H ; 10,91 s, 1H; 11,41 s, 1H
Point de fusion : 166°C (décomposition)

**Exemple 43:** Chlorhydrate de la 2-(5-{2-[4-(2-méthoxy-4-nitro-phényl)-pipérazin-1-yl)-éthoxy}-1-H-indol-3-yl)-éthylamine.

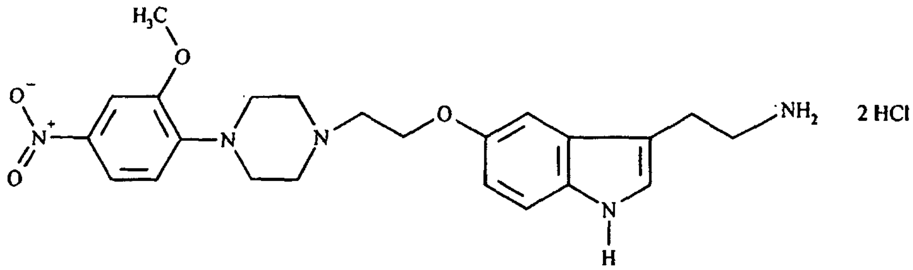

Le composé **43** est préparé à partir du composé **23A** (650 mg ; 1,92 mmol) et de 2-méthoxy-4-nitrophényl-pipérazine (545 mg ; 2,3 mmol) selon les conditions décrites pour la préparation de l'exemple **23.**

La purification du produit, sous forme de base, est effectuée par chromatographie sur colonne de gel de silice éluée par un mélange dichlorométhane/méthanol/ammoniaque (85:14:1; v/v). Le produit pur est isolé sous forme de sirop jaune qui conduit, après traitement à l'acide chlorhydrique dans l'éther, au composé **43** sous forme de poudre jaune (777 mg; 79 %).

Analyse élémentaire ($C_{23}H_{31}Cl_2N_5O_4$, $1H_2O$ ), % calculés: C 52,08 ; H 6,27 ; N 13,20, Cl 13,37 % trouvés : C 52,14 ; H 6,14 ; N 13,05 ; Cl 13,43

RMN 1H, DMSO-d6 (ppm) : 3,03 s, 4H ; 3,36 m, 4H ; 3,63 m, 4H ; 3,83 m, 2H; 3,94 s, 3H ; 4,50 t, 2H ; 6,84 dd, 1H ; 7,13 d, 1H ; 7,24-7,32 m, 3H ; 7,74 d, 1H ; 7,86 dd, 1H ; 8,15 large s, 3H; 10,93 s, 1H ; 11,60 s, 1H.

Point de fusion : 210°C (décomposition)

**Exemple 44:** Chlorhydrate du 4-(4-{2-[3-(2-amino-éthyl)-1H-indol-5-yloxy]-éthyl]-pipérazin-1-yl)-benzonitrile.

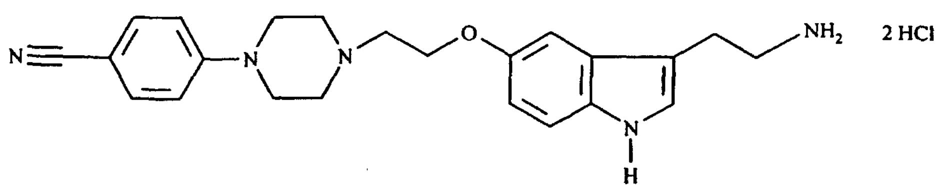

**44A** -4-(4-{2-[3-(2-{N-terbutoxylcarbonyl}-amino-éthyl)-1H-indol-5-yloxy]-éthyl]-pipérazin-1-yl)-benzonitrile.

Le composé **44A** est préparé à partir du produit **23A** (2,8 g; 8,19 mmol) et de 4-cyanophényle-pipérazine (3,06 g; 8,19 mmol) selon la méthode décrite pour la préparation de l'exemple **23.**

Le sirop obtenu est chromatographié sur colonne de gel de silice éluée par un mélange dichlorométhane/acétate d'éthyle (5:1, v/v). Le produit pur est isolé sous forme de sirop incolore (2,03 g; 50 %).

RMN 1H, $CDCl_3$ (ppm) : 1,42 s, 9H ; 2,74-2,94 m, 8H ; 3,36 m, 6H ; 4,20 t, 2H; 4,62 large s, 1H ; 6,83 m, 3H ; 7,04 dd, 2H ; 7,24 m, 1H ; 7,50 m, 2H ; 8,11 s, 1H.

**44 -** Chlorhydrate du 4-(4-{2-[3-(2-amino-éthyl)-1H-indol-5-yloxy]-éthyl]-pipérazin-1yl)-benzonitrile.

Le produit **44A** (707 mg; 1,44 mmol) est ensuite déprotégé selon la méthode décrite pour la préparation de l'exemple **2** à partir de **2A.**

La purification du produit, sous forme de base, est effectuée par chromatographie sur colonne de gel de silice éluée par un mélange dichlorométhane/méthanol/ammoniaque (80:19:1; v/v). Le produit pur est isolé sous forme de sirop incolore qui conduit, après traitement à l'acide chlorhydrique dans l'éther, au composé **44** sous forme de poudre blanche (496 mg ; 76 %).

Analyse élémentaire ($C_{23}H_{27}N_5O_1$.2,3HCl, $H_2O$), % calculés: C 56,22; H 6,42; N 14,25; Cl 16,59; % trouvés : C 56,84;

H 6,40; N 14,04; Cl 16,72.
RMN 1H, DMSO-d6 (ppm) : 3,02 s, 4H; 3,37 m, 4H; 3,62 m, 4H; 4,11 m, 2H; 4,49 t, 2H; 6,84 dd, 1H; 7,13 d, 2H; 7,23-7,32 m, 3H; 7,67 d, 2H; 8,14 large s, 3H; 10,93 s, 1H; 11,58 large s, 1H.
Point de fusion : 160°C

**Exemple 45 :** Chlorhydrate de la [2-(5-{2-[4-(4-aminométhyl-phényl)-pipérazin-1-yl]-éthoxy}-1H-indol-3-yl)-éthyl]-méthyl-amine.

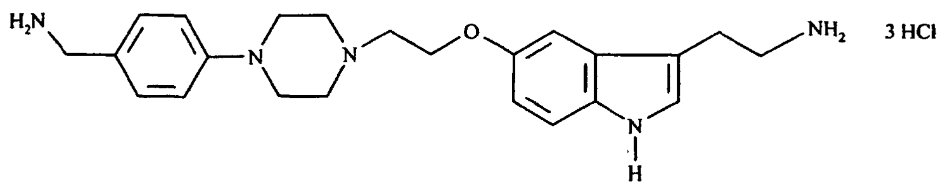

Le composé **44A** (600 mg ; 1,23 mmol) en solution dans le tétrahydrofurane anhydre (10 ml) est traité, sous azote et à 0°C, par une solution molaire d'hydrure de lithium aluminium dans le tétrahydrofurane (2,5 ml; 2,46 mmol). Le mélange est ensuite agité à 80°C pendant 12 h. Le milieu est alors traité par du sulfate de sodium/eau puis filtré sur célite.
La purification du produit, sous forme de base, est effectuée par chromatographie sur colonne de gel de silice éluée par un mélange dichlorométhane/méthanol/ammoniaque (80:18:2; v/v). Le produit pur est isolé sous forme de sirop jaune pâle qui conduit, après traitement à l'acide chlorhydrique dans l'éther, au composé **45** sous forme de poudre jaune (281 mg; 44 %).
Analyse élémentaire ($C_{24}H_{36}Cl_3N_5O_1$, 1,3$H_2O$), % calculés: C 53,35 ; H 7,20; N 12,96; Cl 19,68; % trouvés : C 53,37 ; H 7,19 ; N 12,70 ; Cl 19,66.
RMN 1H, DMSO-d6 (ppm) : 3,09-3,28 m, 8H; 3,49-3,93 m, 8H; 4,53 s, 2H; 6,84 dd, 1H; 7,05 d, 2H; 7,23-7,42 m, 5H; 8,39 large s, 3H; 9,16 large s, 2H; 10,94 s, 1H; 11,55 large s, 1H.
Point de fusion : 84°C (décomposition)

**Exemple 46 :** Chlorhydrate du 2-(4-{2-[3-(2-amino-éthyl)-1H-indol-5-yloxy]-acétyl}-pipérazin-1-yl)-benzonitrile.

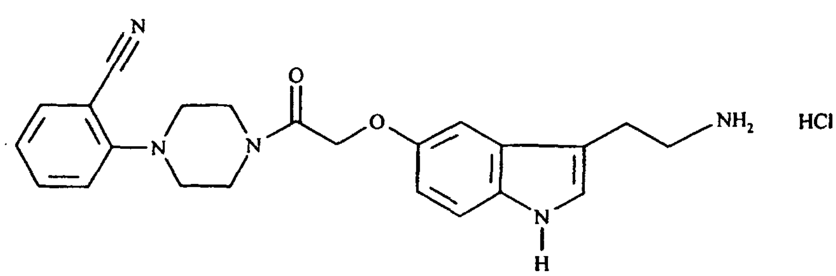

**46A** -2-(4-{2-[3-(2-{N-terbutoxycarbonyl}-amino-éthyl)-1H-indol-5-yloxy]-acétyl}-pipérazin-1-yl)-benzonitrile.

Le composé **46A** est préparé à partir de la 2-cyanophényle-pipérazine (1 g; 5,34 mmol), de chlorure de chloracétyle (0,43 ml; 5,34 mmol) et du composé **1A** (660 mg ; 2,39 mmol) selon la procédure décrite pour la préparation du composé **1B**.
Le sirop obtenu est chromatographié sur colonne de gel de silice éluée par un mélange dichlorométhane/acétone (5:1, v/v). Le produit pur est isolé sous forme de sirop incolore (1,01 g; 84 %).
RMN 1H, $CDCl_3$ (ppm) : 1,40 s, 9H ; 2,88 t, 2H ; 3,16 m, 4H ; 3,41 t, 2H ; 3,84 t, 2H ; 4,60 large s, 1H ; 4,75 s, 2H ; 6,86 dd, 1H ; 6,94-7,09 m, 4H ; 7,23 m, 1H ; 7,43-7,58 m, 2H ; 8,10 large s, 1H.

**46 -** Chlorhydrate du 2-(4-{2-[3-(2-amino-éthyl)-1H-indol-5-yloxy]-acétyl}-pipérazin-1-yl)-benzonitrile.

Le produit **46A** (741 mg ; 1,47 mmol) est ensuite déprotégé selon la méthode décrite pour la préparation de l'exemple **2** à partir de **2A.**
La purification du produit, sous forme de base, est effectuée par chromatographie sur colonne de gel de silice éluée par un mélange dichlorométhane/méthanol/ammoniaque (80:19:1; v/v). Le produit pur est isolé sous forme de sirop incolore qui conduit, après traitement à l'acide chlorhydrique dans l'éther, au composé **46** sous forme de poudre blanche (388 mg ; 60 %).
Analyse élémentaire ($C_{23}H_{26}ClN_5O_2$, 1,4$H_2O$), % calculés: C 59,39; H 6,24; N 15,06; Cl 7,62; % trouvés : C 59,72; H 5,85; N 14,79; Cl 8,14.
RMN 1H, DMSO-d6 (ppm) : 3,00-3,20 m, 8H ; 3,69 m, 4H ; 4,83 s, 2H ; 6,79 dd, 1H ; 7,10-7,29 m, 5H ; 7,58-7,76 m, 2H ; 8,08 large s, 3H ; 10,88 s, 1H.
Point de fusion : 107°C (décomposition)

**Exemple 47 :** Chlorhydrate du 2-[3-(2-amino-éthyl)-1H-indol-5-yloxy]-1-[4-(2-aminométhyl-phényl)-pipérazin-1-yl]-éthanone.

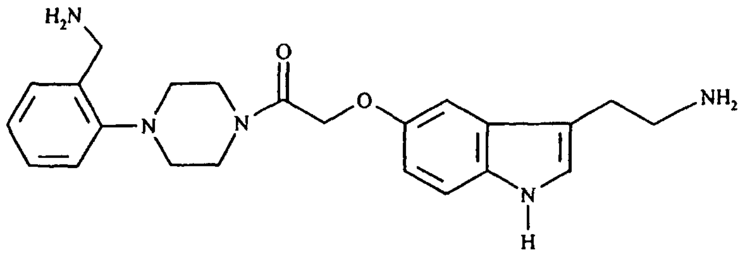

3 HCl

**47A** -2-[3-(2-{N-terbutoxycarbonyl}-amino-éthyl)-1H-indol-5-yloxy]-1-[4-(2-aminométhyl-phényl)-pipérazin-1-yl]-éthanone.

Le composé **46A** (3,4 g; 6,8 mmol) en solution dans le tétrahydrofurane (114 ml), en présence de Nickel de Raney (~200 mg; catalytique), est soumis à une pression atmosphérique d'hydrogène pendant 48 heures, à température ambiante. Le mélange est filtré sur célite et évaporé à sec.
Le sirop obtenu est chromatographié sur colonne de gel de silice éluée par un mélange dichlorométhane/méthanol/ammoniaque (90:9:1, v/v). Le produit pur est isolé sous forme de sirop incolore (2,97 g; 86 %).
RMN 1H, DMSO-d6 (ppm) : 1,36 s, 9H ; 2,70-2,88 m, 6H; 3,17 m, 4H; 3,62 large s, 4H ; 3,77 s, 2H ; 4,77 s, 2H ; 6,75 dd, 1H; 6,87 t, 1H ; 7,00-7,24 m, 6H; 7,43 dd, 1H ; 10,65 s, 1H.

**47 -** Chlorhydrate du 2-[3-(2-amino-éthyl)-1H-indol-5-yloxy]-1-[4-(2-aminométhylphényl)-pipérazin-1yl]-éthanone.

Le composé 47A (600mg; 1,18 mmol) est ensuite déprotégé selon la méthode décrite pour la préparation de l'exemple **40**.
La purification du produit, sous forme de base, est effectuée par chromatographie sur colonne de gel de silice éluée par un mélange dichlorométhane/méthanol/ammoniaque (80:18:2; v/v). Le produit pur est isolé sous forme de sirop incolore qui conduit, après traitement à l'acide chlorhydrique dans l'éther, au composé **47** sous forme de poudre blanche (506 mg; 83 %).
Analyse élémentaire ($C_{23}H_{32}Cl_3N_5O_2$, 1,6$H_2O$), % calculés: C 50,62 ; H 6,50; N 12,83; Cl 19,49; % trouvés : C 50,85; H 6,36; N 12,64; Cl 18,00.
RMN 1H, DMSO-d6 (ppm) : 2,80-2,99 m, 8H ; 3,69 large s, 4H ; 4,11 s, 6H ; 4,82 s, 2H ; 6,77 dd, 1H ; 7,15-7,54 m, 7H ; 8,16 large s, 3H ; 8,45 large s, 3H ; 10,86 s, 1H.
Point de fusion : 198°C (décomposition)

**Exemple 48:** Chlorhydrate de la N-[2-[4-{2-[3-(2-amino-éthyl)-1H-indol-5-yloxy]-acétyl }-pipérazin-1-yl)-benzyl-méthanesulfonamide.

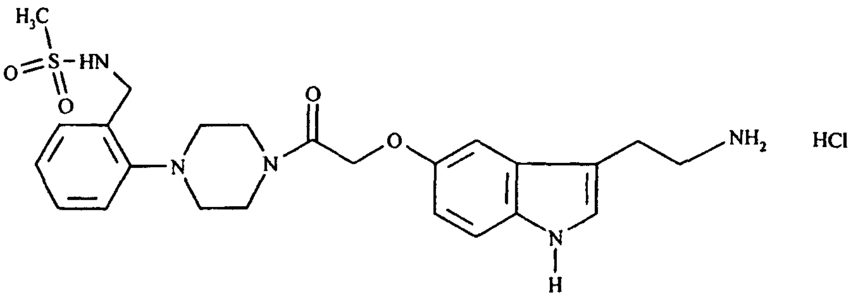

Le composé **48** est préparé à partir du composé 47A (800 mg ; 1,58 mmol) et du chlorure de mésyle (0,27 ml ; 3,16 mmol) selon la méthode décrite pour la préparation de l'exemple **3**.

La purification du produit, sous forme de base, est effectuée par chromatographie sur colonne de gel de silice éluée par un mélange dichlorométhane/méthanol/ammoniaque (83:16:1; v/v). Le produit pur est isolé sous forme de sirop incolore qui conduit, après traitement à l'acide chlorhydrique dans l'éther, au composé **48** sous forme de poudre blanche (585 mg; 71 %).
Analyse élémentaire ($C_{24}H_{31}N_5O_4S$ .1,5HCl, $1H_2O$), % calculés: C 51,63 ; H 6,23 ; N 12,54 ; Cl 9,52 ; % trouvés : C 51,98 ; H 6,11 ; N 12,45 ; Cl 8,94.
RMN 1H, DMSO-d6 (ppm) : 2,80-3,00 m, 11H ; 3,67 large s, 4H ; 4,26 d, 2H ; 4,82 s, 2H ; 6,79 dd, 1H ; 7,09-7,47 m, 7H ; 8,01 large s, 3H ; 10,85 s, 1H.
Point de fusion : 250°C.

**Exemple 49 :** Chlorhydrate de la N-[2-(4-{2-[3-(2-amino-éthyl)-1H-indol-5-yloxy]-acétyl}-pipérazin-1-yl]-benzenesulfonamide.

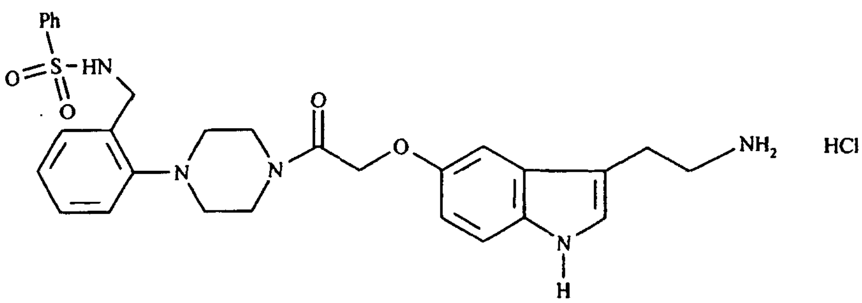

Le composé **49** est préparé à partir du composé **47A** (800 mg ; 1,58 mmol) et du chlorure de benzènesulfonyle (0,22 ml ; 1,70 mmol) selon la méthode décrite pour la préparation de l'exemple **3.**

La purification du produit, sous forme de base, est effectuée par chromatographie sur colonne de gel de silice éluée par un mélange dichlorométhane/méthanol/ammoniaque (80:19,5:0,5; v/v). Le produit pur est isolé sous forme de sirop incolore qui conduit, après traitement à l'acide chlorhydrique dans l'éther, au composé **49** sous forme de poudre blanche (674 mg ; 73 %).
Analyse élémentaire ($C_{29}H_{34}ClN_5O_4S$), % calculés: C 59,63 ; H 5,87 ; N 11,99; Cl 6,07 ; % trouvés : C 59,34 ; H 5,85 ; N 11,81 ; Cl 6,21.
RMN 1H, DMSO-d6 (ppm) : 2,75 m, 4H ; 3,00 m, 4H ; 3,50 large s, 4H ; 4,04 s, 2H ; 4,78 s, 2H ; 6,77 dd, 1H ; 7,01-7,33 m, 7H ; 7,55 m, 3H ; 7,80 m, 2H ; 7,99 large s, 4H ; 10,84 s, 1H.
Point de fusion : 240°C.

**Exemple 50 :** Chlorhydrate du 4-(4-{2-[3-(2-amino-éthyl)-1H-indol-5-yloxy]-acétyl}-pipérazin-1-yl)-benzonitrile.

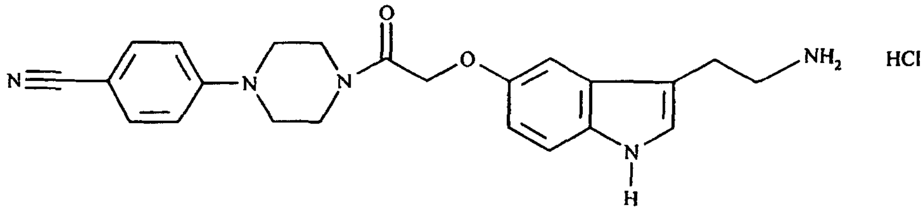

**50A** -4-(4-{2-[3-(2-{N-terbutoxycarbonyl}-amino-éthyl)-1H-indol-5-yloxy]-acétyl}-pipérazin-1-yl)-benzonitrile.

Le composé **50A** est préparé à partir de la 4-cyanophényle-pipérazine (30,1 g ; 160,9 mmol), de chlorure de chloracétyle (12,8 ml ; 160,9 mmol) et du composé **1A** (7,2 g; 26,06 mmol) selon la procédure décrite pour la préparation du composé **1B**.

Le sirop obtenu est chromatographié sur colonne de gel de silice éluée par un mélange acétate d'éthyle/dichlorométhane (2:1, v/v). Le produit pur est isolé sous forme de poudre blanche (10,9 g; 83 %).
Analyse élémentaire ($C_{28}H_{33}N_5O_4$), % calculés: C 66,78 ; H 6,61 ; N 13,91 ; % trouvés : C 66,92 ; H 6,73 ; N 13,64.
RMN 1H, CDCl3 (ppm) : 1,36 s, 9H ; 2,83 t, 2H ; 3,25-3,40 m, 6H ; 3,75 m, 4H; 4,71 s, 2H ; 6,83 m, 3H ; 6,97 s, 1H ; 7,03 d, 1H ; 7,22 d, 1H ; 7,45 m, 2H; 8,75 large s, 1H.

**50 -** Chlorhydrate du 4-(4-{2-[3-(2-amino-éthyl)-1H-indol-5-yloxy]-acétyl}-pipérazin-1-yl)-benzonitrile.

Le produit **50A** (1g; 1,98 mmol) est ensutie déprotégé selon la méthode décrite pour la préparation de l'exemple **2** à partir de **2A.**
La purification du produit, sous forme de base, est effectuée par chromatographie sur colonne de gel de silice éluée par un mélange dichlorométhane/méthanol/ammoniaque (80:19:1; v/v). Le produit pur est isolé sous forme de sirop incolore qui conduit, après traitement à l'acide chlorhydrique dans l'éther, au composé **50** sous forme de poudre blanche (728 mg; 88 %).

Analyse élémentaire ($C_{23}H_{25}N_5O_2$, 1,3HCl, $0{,}8H_2O$), % calculés: C 59,37 ; H 6,04 ; N 15,05 ; Cl 9,91 ; % trouvés : C 59,46 ; H 5,92 ; N 14,87 ; Cl 9,77.
RMN 1H, DMSO-d6 (ppm): 2,99 m; 4H ; 3,45 m, 4H ; 3,68 m, 4H ; 4,83 s, 2H ; 6,78 dd, 1H ; 7,03 d, 2H ; 7,19 dd, 2H ; 7,26 d, 1H ; 7,61 d, 2H ; 8,03 large s, 3H ; 10,86 s, 1H.
Point de fusion : 224-226°C.

**Exemple 51 :** Chlorhydrate de la 2-[3-(2-amino-éthyl)-1H-indol-5-yloxy]-1-[4-(4-aminométhyl-phényl)-pipérazin-1-yl]-éthanone.

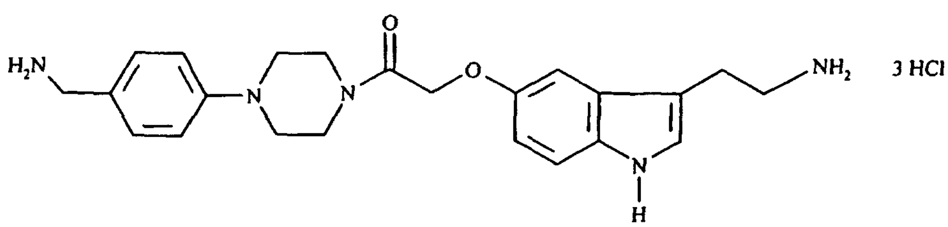

**51A** -2-[3-(2-{N-terbutoxycarbonyl}-amino-éthyl)-1H-indol-5-yloxy]-1-[4-(4-aminométhyl-phényl)-pipérazin-1-yl]-éthanone.

Le composé **50A** (800 mg; 1,59 mmol) est hydrogéné dans les conditions décrites pour la préparation du produit **47A** à partir de **46A.**

Le sirop obtenu est chromatographié sur colonne de gel de silice éluée par un mélange dichlorométhane/méthanol/ammoniaque (80:19:1, v/v). Le produit pur est isolé sous forme de sirop incolore (800 mg; 99 %).
RMN 1H, $CDCl_3$ (ppm) : 1,44 s, 9H; 2,91 t, 2H; 3,17 m, 4H; 3,46 m, 2H; 3,80 m, 6H; 4,66 large s, 1H; 4,78 s, 2H; 6,90 m, 3H; 7,02 d, 1H; 7,11 d, 1H; 7,21-7,29 m, 4H; 8,14 large s, 1H.

**51** - Chlorhydrate de la 2-[3-(2-amino-éthyl)-1H-indol-5-yloxy]-1-[4-(4-aminométhyl-phényl)-pipérazin-1-yl]-éthanone

Le composé **51A** (500 mg ; 0,98 mmol) est ensuite déprotégé dans les conditions décrites pour la préparation de l'exemple **2** à partir de **2A.**

La purification du produit, sous forme de base, est effectuée par chromatographie sur colonne de gel de silice éluée par un mélange dichlorométhane/méthanol/ammoniaque (75:20:5; v/v). Le produit pur est isolé sous forme de solide beige qui conduit, après traitement à l'acide chlorhydrique dans l'éther, au composé **51** sous forme de poudre jaune pâle (304 mg; 60 %).
Analyse élémentaire ($C_{23}H_{32}Cl_3N_5O_2$, 1,5$H_2O$), % calculés: C 50,79 ; H 6,49 ; N 12,88 ; Cl 19,55 ; % trouvés : C 50,68; H 6,46; N 12,71; Cl 20,09.
RMN 1H, DMSO-d6 (ppm) : 2,98 large s, 4H ; 3,20-3,37 m, 4H ; 3,69 m, 4H ; 3,90 m, 2H ; 4,82 s, 2H ; 6,77 dd, 1H ; 7,10 d, 2H ; 7,18 d, 2H ; 7,25 d, 1H ; 7,38 d, 2H ; 8,13 large s, 3H ; 8,35 large s, 3H ; 10,85 s, 1H.
Point de fusion : 185°C (décomposition)

**Exemple 52 :** Chlorhydrate de la N-[4-(4-{2-[3-(2-amino-éthyl)-1H-indol-5-yloxy]-acétyl}-pipérazin-1-yl)-benzyl]-méthanesulfonamide.

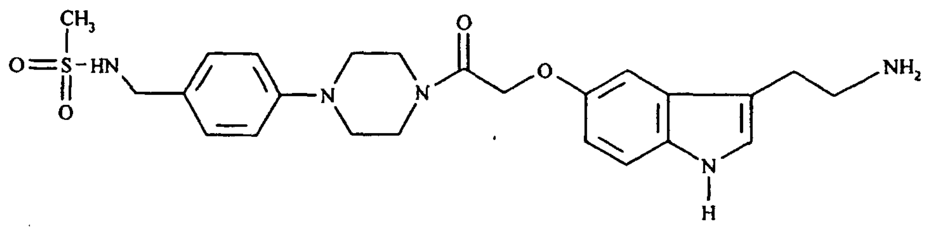

2 HCl

Le composé **52** est préparé à partir du produit **51A** (900 mg ; 1,77 mmol) et de chlorure de mésyle (0,14 ml ; 1,77 mmol) dans les conditions décrites pour la préparation de l'exemple **3**.

La purification du produit, sous forme de base, est effectuée par chromatographie sur colonne de gel de silice éluée par un mélange dichlorométhane/méthanol/ammoniaque (80:19:1; v/v). Le produit pur est isolé sous forme de solide beige qui conduit, après traitement à l'acide chlorhydrique dans l'éther, au composé **52** sous forme de poudre blanche (277 mg; 28 %).
Analyse élémentaire ($C_{24}H_{33}Cl_2N_5O_4S$, 0,3$H_2O$), % calculés: C 51,12 ; H 6,01; N 12,42 ; Cl 12,57 ; % touvés: C 51,12 ; H 6,15 ; N 12,26 ; Cl 11,78.
RMN 1H, DMSO-d6 (ppm) : 2,80 s, 3H ; 2,97 large s, 4H ; 3,25 m, 4H ; 3,74 m, 4H; 4,05 s, 2H ; 4,81 s, 2H ; 6,75 dd, 1H ; 7,17-7,29 m, 7H ; 7,49 large s, 1H ; 8,05 large s, 3H ; 10,83 s, 1H.
Point dé fusion : 154°C (décomposition)

**Exemple 53 :** Chlorhydrate de la 2-[3-(2-amino-éthyl)-1H-indol-5-yloxy]-1-[4-(4-amino-2-méthoxy-phényl)-pipérazin-1-yl]-éthanone.

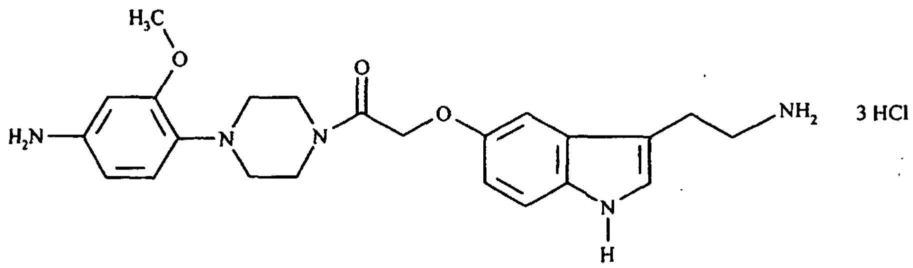

**53A** -2-[3-(2-{N-terbutoxycarbonyl}-amino-éthyl)-1H-indol-5-yloxy]-1-[4-(4-amino-2-méthoxy-phényl)-pipérazin-1-yl]-éthanone.

Le composé 22, sous sa forme protégée (NHBOC), (3,06 g ; 5,53 mmol) est traité dans les conditions décrites pour la préparation de l'exemple **2** à partir de **1C**.

Le sirop obtenu est chromatographié sur colonne de gel de silice éluée par un mélange dichlorométhane/acétone (2:1, v/v). Le produit pur est isolé sous forme de mousse beige (2,3 g; 81 %).
RMN 1H, DMSO-d6 (ppm) : 1,38 s, 9H ; 2,72 m, 6H ; 3,18 m, 2H ; 3,60-3,75 m, 7H ; 4,77 s, 2H ; 6,05-6,31 m, 2H ; 6,75-6,89 m, 2H ; 7,07 dd, 2H ; 7,23 d, 1H ; 10,67 s, 1H.

**53 -** Chlorhydrate de la 2-[3-(2-amino-éthyl)-1H-indol-5-yloxy]-1-[4-(4-amino-2-méthoxy-phényl)-pipérazin-1-yl]-éthanone.

Le composé **53A** (600 mg ; 1,14 mmol) est ensuite déprotégé selon la méthode décrite pour la préparation de l'exemple **40** à partir de **40A**.

La purification du produit, sous forme de base, est effectuée par chromatographie sur colonne de gel de silice éluée par un mélange dichlorométhane/méthanol/ammoniaque (80:18:2; v/v). Le produit pur est isolé sous forme de sirop incolore qui conduit, après traitement à l'acide chlorhydrique dans l'éther, au composé **53** sous forme de poudre blanche (441 mg; 73 %).
Analyse élémentaire ($C_{23}H_{32}Cl_3N_5O_3$, $2{,}2H_2O$), % calculés: C 48,25; H 6,41 ; N 12,23 ; Cl 18,58 ; % trouvés : C 48,03 ; H 6,19 ; N 12,06 ; Cl 18,72.
RMN 1H, DMSO-d6 (ppm) : 3,00-3,16 m, 8H ; 3,71 m, 4H ; 3,81 s, 3H ; 4,83 s, 2H ; 6,77-6,96 m, 3H ; 7,10-7,29 m, 4H ; 8,14 large s, 3H ; 10,87 s, 1H.
Point de fusion : ~ 190°C

**Exemple 54 :** Chlorhydrate de la N-[4-(4-{2-[3-(2-amino-éthyl)-1H-indol-5-yloxy]-acétyl}-pipérazin-1-yl)-3-méthoxy-phényl]-méthanesulfonamide.

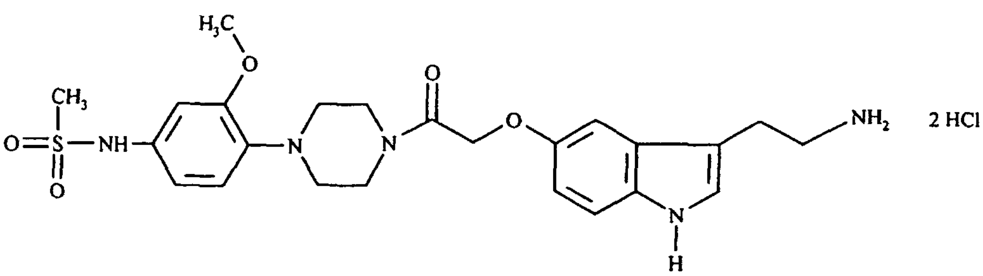

Le composé **54** est préparé à partir du produit **53A** (1,0 g ; 1,91 mmol) et de chlorure de mésyle (0,16 ml ;

2,10 mmol) selon la méthode décrite pour la préparation de l'exemple **3.**

La purification du produit, sous forme de base, est effectuée par chromatographie sur colonne de gel de silice éluée par un mélange dichlorométhane/méthanol/ ammoniaque (80:18:2; v/v). Le produit pur est isolé sous forme de solide beige qui conduit, après traitement à l'acide chlorhydrique dans l'éther, au composé **54** sous forme de poudre beige (745 mg; 68 %).

Analyse élémentaire ($C_{24}H_{33}Cl_2N_5O_5S$, $2H_2O$), % calculés: C 47,21 ; H 6,11 ; N 11,47 ; Cl 11,61 ; % trouvés : C 47,49 ; H 5,82 ; N 11,29 ; Cl 11,76.

RMN 1H, DMSO-d6 (ppm) : 2,97-3,16 m, 11H ; 3,79 m, 7H ; 4,82 s, 2H ; 6,80 dd, 1H ; 6,85 d, 2H ; 7,10-7,28 m, 4H ; 8,04 large s, 3H ; 9,68 s, 1H ; 10,85 s, 1H.

Point de fusion : 195°C (décomposition)

**Exemple 55 :** Chlorhydrate de la 2-[3-(2-amino-éthyl)-1H-indol-5-yloxy]-1-[4-(4-nitro-napthtalèn-1-yl)-pipérazin-1-yl]-éthanone.

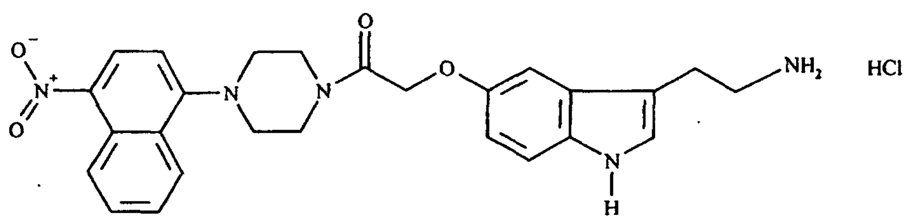

Le composé **55** est préparé à partir du 4-nitro-naphtalényl-pipérazine (2,47 g ; 9,56 mmol), de chlorure de chloracétyle (1,37 ml ; 17,2 mmol) et du composé **1A** (350 mg ; 1,26 mmol) selon la méthode décrite pour la préparation de l'exemple **1**.

La purification du produit, sous forme de base, est effectuée par chromatographie sur colonne de gel de silice éluée par un mélange dichlorométhane/méthanol/ammoniaque (85:14:1; v/v). Le produit pur est isolé sous forme de cristaux jaunes qui conduit, après traitement à l'acide chlorhydrique dans l'éther, au composé **55** sous forme de poudre orange (340 mg; 49 %).

Analyse élémentaire ($C_{26}H_{28}ClN_5O_4$, $2H_2O$), % calculés: C 57,19 ; H 5,91; N 12,83 ; Cl 6,49 ; % trouvés : C 57,59 ; H 5,52 ; N 12,69 ; Cl 7,02.

RMN 1H, DMSO-d6 (ppm) : 2,97 m, 4H ; 3,13-3,21 m, 4H ; 3,82 m, 4H ; 4,84 s, 2H ; 6,78 dd, 1H ; 7,17-7,27 m, 4H ; 7,65-7,82 m, 2H ; 8,01 large s, 3H ; 8,26-8,35 m, 2H ; 8,51 d, 1H ; 10,84 s, 1H.

Point de fusion : 183°C.

**Exemple 56 :** Chlorhydrate du méthanesulfonate du 4-(4-{2-[3-(2-amino-éthyl)-1H-indol-5-yloxy]-acétyl}-pipérazin-1-yl)-phényle.

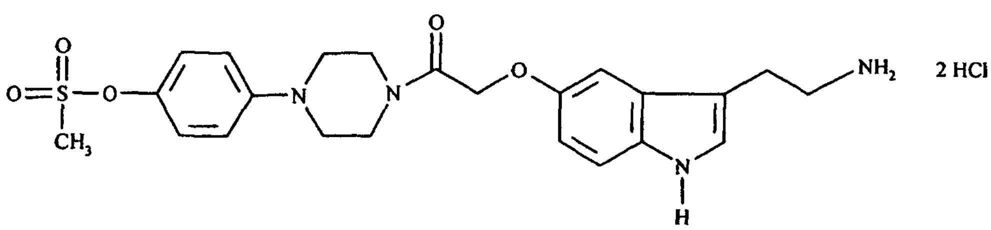

Le composé **56** est préparé à partir du méthanesulfonate du 4-(pipérazin-1-yl)-phényle (1,62 g ; 6,3 mmol), de chlorure de chloracétyle (0,60 ml ; 7,54 mmol) et du composé **1A** (1,16 g ; 4,2 mmol) selon la méthode décrite pour la préparation de l'exemple **1**.

La purification du produit, sous forme de base, est effectuée par chromatographie sur colonne de gel de silice éluée par un mélange dichlorométhane/méthanol/ammoniaque (85:14:1; v/v). Le produit pur est isolé sous forme de sirop incolore qui conduit, après traitement à l'acide chlorhydrique dans l'éther, au composé **56** sous forme de poudre

beige (504 mg; 22 %).
Analyse élémentaire ($C_{23}H_{30}Cl_2N_4O_5S$, $1H_2O$), % calculés: C 49,18 ; H 5,71 ; N 9,97 ; Cl 12,62 ; % trouvés : C 49,13 ; H 5,67 ; N 9,82 ; Cl 11,39.
RMN 1H, DMSO-d6 (ppm) : 2,97 m, 4H ; 3,22 s, 4H ; 3,29 s, 3H ; 3,65 m, 4H ; 4,80 s, 2H ; 6,77 dd, 1H ; 7,02-7,26 m, 7H ; 8,00 large s, 3H ; 10,84 s, 1H.
Point de fusion : 238-240°C (décomposition)

**Exemple 57 :** Chlorhydrate du 6-[3-(2-amino-éthyl)-1H-indol-5-yloxy]-1-[4-(4-nitro-phényl)-pipérazin- 1-yl]-hexan-1-one.

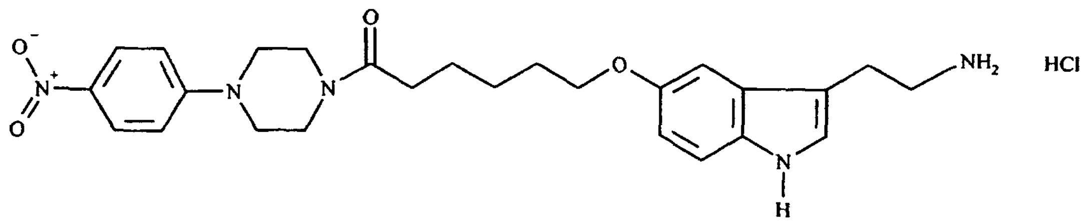

**57A** -6-chloro-1-[4-(4-nitro-phényl)-pipérazin-1-yl]-hexan-1-one

La 4-nitro-phényl-pipérazine (3,0 g ; 14,48 mmol) en solution dans la méthyléthylcétone (105 ml), en présence de carbonate de potassium (6 g ; 43,4 mmol) est traité, sous azote et à 0°C, par le chlorure de 6-chloro-hexanoique (10,2 g ; 58 mmol). Aprés 2 heures d'agitation de 0°C à température ambiante le milieu est dilué à l'acétate d'éthyle et lavé à la soude (2N) puis à l'eau. La phase organique est séchée sur sulfate de sodium, filtrée et évaporée à sec.
Le sirop obtenu est chromatographié sur colonne de gel de silice éluée par un mélange dichlorométhane/acétone (30:1 puis 10:1, v/v). Le produit **57A** est obtenu sous forme de cristaux jaunes (4,1 g; 84 %).

**57B** -6-[3-(2-{N-terbutoxycarbonyl}-amino-éthyl)-1H-indol-5-yloxy]-1-[4-(4-nitro-phényl)-pipérazin-1-yl]-hexan-1-one.

Un mélange du composé **1A** (1,87 g ; 6,77 mmol) et du composé **57A** (4,1 g; 12,2 mmol) dans le diméthylformamide (11 ml), en présence de carbonate de césium (3,3 g ; 10,1 mmol) est chauffé à 70°C pendant 24 h. Le milieu est dilué à l'acétate d'éthyle et lavé à l'eau. La phase organique est séchée sur sulfate de sodium, filtrée et évaporée à sec.
Le sirop obtenu est chromatographié sur colonne de gel de silice éluée par un mélange dichlorométhane/acétate d'éthyle (2:1 puis 1:1, v/v). Le produit pur est isolé sous forme de sirop jaune (2,5 g; 63 %).
RMN 1H, DMSO-d6 (ppm) : 1,34 s, 9H ; 1,44-1,72 m, 6H ; 2,36 t, 2H ; 2,70 t, 2H ; 3,12 m, 2H ; 3,46 m, 4H ; 3,56 m, 4H ; 3,92 t, 2H ; 6,66 dd, 1H ; 6,84 t, 1H ; 6,96 m, 3H ; 7,04 d, 1H ; 7,17 d, 1H ; 8,04 d, 2H ; 10,58 s, 1H.

**57 -** Chlorhydrate du 6-[3-(2-amino-éthyl)-1H-indol-5-yloxy]-1-[4-(4-nitro-phényl)-pipérazin-1-yl]-hexan-1-one.

Le composé **57B** (500 mg ; 0,86 mmol) est ensuite déprotégé selon la méthode décrite pour la préparation de l'exemple **1** à partir de **1C**.
La purification du produit, sous forme de base, est effectuée par chromatographie sur colonne de gel de silice éluée par un mélange dichlorométhane/méthanol/ammoniaque (85:14:1; v/v). Le produit pur est isolé sous forme de sirop jaune qui conduit, après traitement à l'acide chlorhydrique dans l'éther, au composé **57** sous forme de poudre jaune (315 mg; 71 %).
Analyse élémentaire ($C_{26}H_{34}ClN_5O_4$, $1H_2O$), % calculés: C 58,08 ; H 6,77 ; N 13,02 ; Cl 7,25 ; % trouvés: C 57,96 ; H 6,47 ; N 12,73 ; Cl 7,48.
RMN 1H, DMSO-d6 (ppm): 1,44-1,76 m, 6H ; 2,37 t, 2H ; 2,96 m, 4H ; 3,54 m, 8H; 3,94 t, 2H ; 6,70 dd, 1H; 6,95-7,04 m, 3H; 7,15-7,23 m, 2H; 8,02 m, 5H; 10,77 s, 1H.
Point de fusion : 100°C

**Exemple 58 :** Chlorhydrate du 6-[3-(2-amino-éthyl)-1H-indol-5-yloxy]-1-[4-(4-amino-phényl)-pipérazin-1-yl]-hexan-1-one

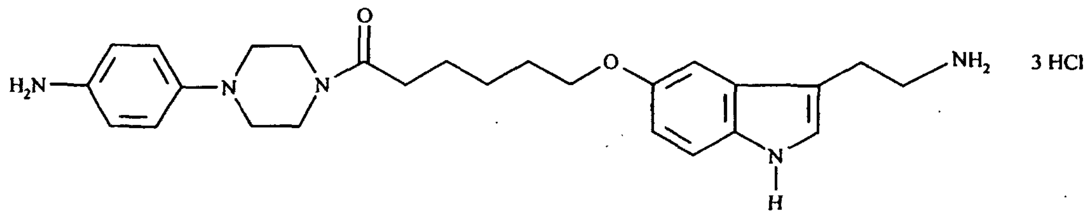

**58A**-6-[3-(2-{N-terbutoxycarbonyl}-amino-éthyl)-1H-indol-5-yloxy]-1-[4-(4-amino-phényl)-pipérazin-1-yl]-hexan-1-one

Le composé **57B** (2,01 g ; 3,46 mmol) est hydrogéné dans les conditions décrites pour la préparation de l'exemple **2A**.

Le sirop obtenu est chromatographié sur colonne de gel de silice éluée par un mélange dichlorométhane/acétone (3:1, v/v). Le produit pur est isolé sous forme de sirop beige (1,89 g; 99 %).
RMN 1H, DMSO-d6 (ppm) : 1,35 s, 9H ; 1,40-1,72 m, 6H ; 2,34 t, 2H ; 2,68-2,86 m, 6H ; 3,12 m, 2H ; 3,52 large s, 4H ; 3,92 t, 2H ; 4,59 s, 2H ; 6,46 d, 2H; 6,67 m, 3H ; 6,84 t, 1H ; 7,00 dd, 2H ; 7,17 d, 1H.

**58 -** Chlorhydrate du 6-[3-(2-amino-éthyl)-1H-indol-5-yloxy]-1-[4-(4-amino-phényl)-pipérazin-1-yl]-hexan-1-one

Le composé **58A** (500 mg ; 0,91 mmol) est ensuite déprotégé dans les conditions décrites pour la préparation de l'exemple **1** à partir de **1C**.

La purification du produit, sous forme de base, est effectuée par chromatographie sur colonne de gel de silice éluée par un mélange dichlorométhane/méthanol/ammoniaque (80:18:2; v/v). Le produit pur est isolé sous forme de sirop jaune pâle qui conduit, après traitement à l'acide chlorhydrique dans l'éther, au composé **58** sous forme de poudre rose pâle (270 mg; 60 %).
Analyse élémentaire ($C_{26}H_{38}Cl_3N_5O_2$, 1,4$H_2O$), % calculés: C 53,46 ; H 7,04 ; N 11,99 ; Cl 18,21; % trouvés : C 53,44 ; H 6,96 ; N 11,80 ; Cl 17,13.
RMN 1H, DMSO-d6 (ppm) : 1,40-1,80 m, 6H ; 2,40 t, 2H ; 2,99 large s, 4H ; 3,18 large s, 4H ; 3,64 m, 4H ; 3,97 t, 2H ; 6,70 dd, 1H ; 7,07-7,28 m, 7H ; 8,09 large s, 3H; 10,82 s, 1H.
Point de fusion : 150°C

**Exemple 59 :** Chlorhydrate de la N-[4-(4-{6-[3-(2-amino-éthyl)-1H-indol-5-yloxy]-hexanoyl}-pipérazin-1-yl)-phényl]-méthanesulfonamide.

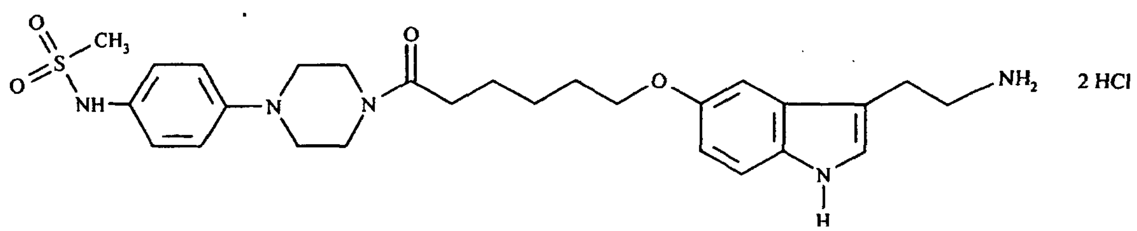

Le composé **59** est préparé à partir du produit **58A** (690 mg; 1,25 mmol) et du chlorure de mésyle (107 µl ; 1,37 mmol) dans les conditions décrites pour la préparation de l'exemple **3.**

La purification du produit, sous forme de base, est effectuée par chromatographie sur colonne de gel de silice éluée par un mélange dichlorométhane/méthanol/ammoniaque (80:19:1; v/v). Le produit pur est isolé sous forme de sirop jaune pâle qui conduit, après traitement à l'acide chlorhydrique dans l'éther, au composé **59** sous forme de poudre beige (658 mg; 92 %).
Analyse élémentaire ($C_{27}H_{39}Cl_2N_5O_4S$, 0,5 $H_2O$, 0,3EtOH), % calculés: C 53,20 ; H 6,61 ; N 11,49 ; Cl 11,63 ; % trouvés: C 53,24 ; H 6,96 ; N 11,10 ; Cl 10,12.

RMN 1H, DMSO-d6 (ppm) : 1,53-1,75 m, 6H ; 2,41 t, 2H ; 2,93 s, 3H ; 2,99 large s, 4H ; 3,23 large s, 4H ; 3,72 large s, 4H ; 3,97 t, 2H ; 6,75 d, 1H ; 7,07-7,25 m, 7H ; 8,04 large s, 3H ; 9,62 s, 1H ; 10,81 s, 1H.
Point de fusion : 141°C.

**Exemple 60:** Chlorhydrate de la N-[4-(4-{6-[3-(2-amino-éthyl)-1H-indol-5-yloxy]-hexanol}-pipérazin-1-yl)-benzyl]-benzènesulfonamide

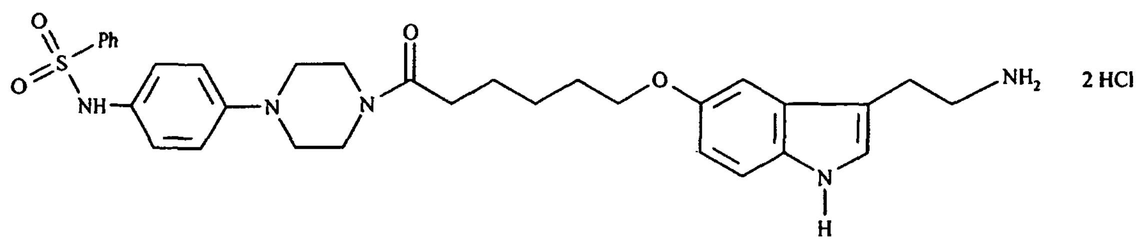

Le composé **60** est préparé à partir du produit **58A** (690 mg ; 1,25 mmol) et du chlorure de benzènesulfonyle (176 µl; 1,37 mmol) dans les conditions décrites pour la préparation de l'exemple **3.**

La purification du produit, sous forme de base, est effectuée par chromatographie sur colonne de gel de silice éluée par un mélange dichlorométhane/méthanol/ammoniaque (80:19:1; v/v). Le produit pur est isolé sous forme de sirop beige qui conduit, après traitement à l'acide chlorhydrique dans l'éther, au composé **60** sous forme de poudre beige (550 mg; 67 %).
Analyse élémentaire ($C_{32}H_{41}Cl_2N_5O_4S$, $0,5H_2O$), % calculés: C 57,22 ; H 6,30; N 10,43 ; Cl 10,56 ; % trouvés: C 57,31 ; H 6,20 ; N 10,18 ; Cl 9,41.
RMN 1H, DMSO-d6 (ppm) : 1,52-1,75 m, 6H ; 2,34 t, 2H ; 2,99-3,08 m, 8H ; 3,61 large s, 4H ; 3,96 t, 2H ; 6,73 dd, 1H ; 6,97-7,26 m, 7H ; 7,53-7,74 m, 5H ; 8,05 large s, 3H ; 10,05 s, 1H ; 10,82 s, 1H.
Point de fusion : 132°C

**Exemple 61 :** Chlorhydrate de la 5-[3-(2-amino-éthyl)-1H-indol-5-yloxy]-1-[4-(4-nitrophényl)-pipérazin-1-yl]-pentan-1-one.

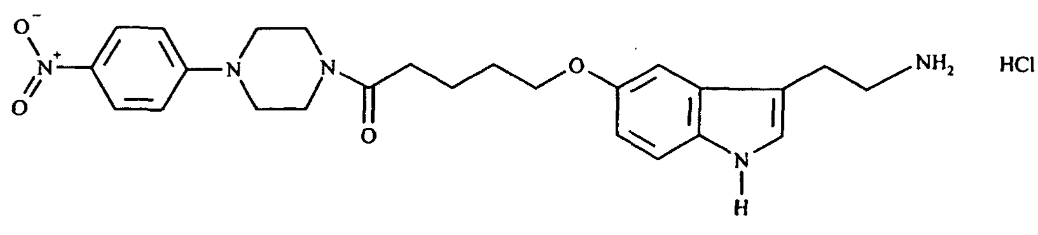

**61A** -5-[3-(2-{N-terbutoxycarbonyl}-amino-éthyl)-1H-indol-5-yloxy]-1-[4-(4-nitrophényl)-pipérazin-1-yl]-pentan-1-one

Le composé **61A** est préparé à partir de la 4-nitro-phényl-pipérazine (1,1 g ; 5,20 mmol), de chlorure de 5-chloro-pentanoyle (2,6 ml; 20,8 mmol) et du composé **1A** (800 mg; 2,89 mmol) dans les conditions décrites pour la préparation du composé **57B**.

Le sirop obtenu est chromatographié sur colonne de gel de silice éluée par un mélange dichlorométhane/acétone (10:1, v/v). Le produit pur est isolé sous forme de sirop jaune (685 mg; 42 %).
RMN 1H, DMSO-d6 (ppm) : 1,35 s, 9H ; 1,71 m, 4H ; 2,46 t, 2H ; 2,70 t, 2H ; 3,13 m, 2H ; 3,47 m, 4H ; 3,58 large s, 4H ; 3,95 t, 2H ; 6,69 dd, 1H ; 6,84 t, 1H ; 6,95-7,19 m, 5H ; 8,05 d, 2H ; 10,58 s, 1H.

**61 -** Chlorhydrate de la 5-[3-(2-amino-éthyl)-1H-indol-5-yloxy]-1-[4-(4-nitrophényl)-pipérazin-1-yl]-pentan-1-one.

Le produit **61A** (300 mg ; 0,530 mmol) est ensuite déprotégé dans les conditons décrites pour la préparation de l'exemple **1** à partir de **1C**.

La purification du produit, sous forme de base, est effectuée par chromatographie sur colonne de gel de silice éluée par un mélange dichlorométhane/méthanol/ammoniaque (80:18:2; v/v). Le produit pur est isolé sous forme de sirop jaune qui conduit, après traitement à l'acide chlorhydrique dans l'éther, au composé **61** sous forme de poudre jaune (211 mg; 75 %).
Analyse élémentaire ($C_{25}H_{32}ClN_5O_4$, 1,7$H_2O$), % calculés: C 56,38 ; H 6,70 ; N 13,15 ; Cl 6,66 ; % trouvés: C 56,33 ; H 6,58 ; N 12,88 ; Cl 7,34.
RMN 1H, DMSO-d6 (ppm) : 1,75 m, 4H ; 2,46 t, 2H ; 2,99 m, 4H ; 3,51 m, 4H; 3,62 large s, 4H ; 4,00 t, 2H ; 6,73 dd, 1H ; 6,99-7,27 m, 5H ; 7,99 large s, 3H ; 8,08 d, 2H ; 10,80 s, 1H.
Point de fusion : 120°C

**Exemple 62 :** Chlorhydrate de la 5-[3-(2-amino-éthyl)-1H-indol-5-yloxy]-1-[4-(amino-phényl)-pipérazin-1-yl]-pentan-1-one.

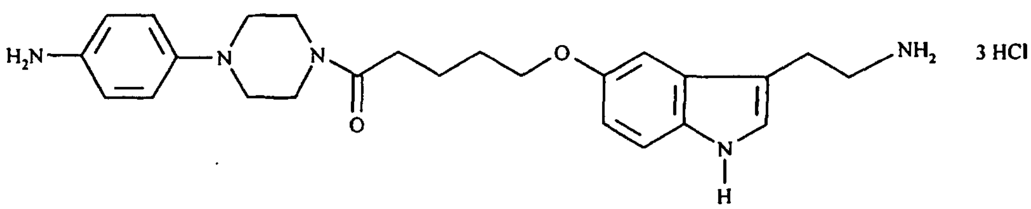

Le composé **62** est préparé à partir du produit **61A** (560 mg ; 0,99 mmol) dans les conditions décrites pour la préparation de l'exemple **2.**
La purification du produit, sous forme de base, est effectuée par chromatographie sur colonne de gel de silice éluée par un mélange dichlorométhane/méthanol/ammoniaque (80:18,5:1,5; v/v). Le produit pur est isolé sous forme de sirop incolore qui conduit, après traitement à l'acide chlorhydrique dans l'éther, au composé **62** sous forme de poudre rose (270 mg; 50%).
Analyse élémentaire ($C_{25}H_{36}Cl_3N_5O_2$, 1,5$H_2O$), % calculés: C 52,50 ; H 6,87 ; N 12,24 ; Cl 18,59 ; % trouvés: C 52,42 ; H 6,94 ; N 12,01 ; Cl 17,36.
RMN 1H, DMSO-d6 (ppm) : 1,72 large s, 4H ; 2,43 t, 2H ; 2,97 large s, 4H ; 3,14 m, 4H ; 3,62 large s, 4H ; 3,97 t, 2H ; 6,71 dd, 1H ; 7,06-7,24 m, 7H ; 8,03 large s, 3H ; 10,79 s, 1H.
Point de fusion : 170°C

**Exemple 63:** Chlorhydrate de la 4-[3-(2-amino-éthyl)-1H-indol-5-yloxy]-1-[4-(4-nitro-phényl)-pipérazin-1-yl]-butan-1-one.

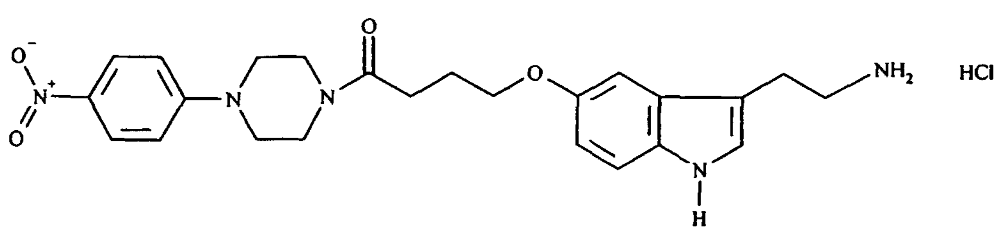

Le composé **63** est préparé à partir de la 4-nitro-phényl-pipérazine (2,0 g ; 9,65 mmol), du chlorure de 4-chloro-butanoyle (4,3 ml ; 38,6 mmol) et du composé **1A** (1,48 g; 5,36 mmol) dans les conditions décrites pour la préparation de l'exemple **57.**
La purification du produit, sous forme de base, est effectuée par chromatographie sur colonne de gel de silice éluée par un mélange dichlorométhane/méthanol/ammoniaque (80:19:1; v/v). Le produit pur est isolé sous forme de sirop jaune qui conduit, après traitement à l'acide chlorhydrique dans l'éther, au composé **63** sous forme de poudre jaune (919 mg; 35 %).
Analyse élémentaire ($C_{24}H_{30}ClN_5O_4$, $H_2O$), % calculés: C 56,97 ; H 6,37 ; N 13,84 ; Cl 7,01 ; % trouvés : C 56,88 ; H

6,12 ; N 13,68 ; Cl 8,89.
RMN 1H, DMSO-d6 (ppm) : 1,96 t, 2H ; 2,54 t, 2H ; 2,97 large s, 4H ; 3,49 m, 4H ; 3,61 m, 4H ; 4,00 t, 2H ; 6,72 dd, 1H ; 6,95-7,25 m, 5H ; 8,05 m, 5H ; 10,82 s, 1H.
Point de fusion : 120°C

**Exemple 64 :** Chlorhydrate de la N-[4-(4-{2-[3-(2-amino)-1H-indol-5-yloxy]-acétyl }-pipérazin-1-yl)-phényl]-4-cyano-phénylsulfonamide.

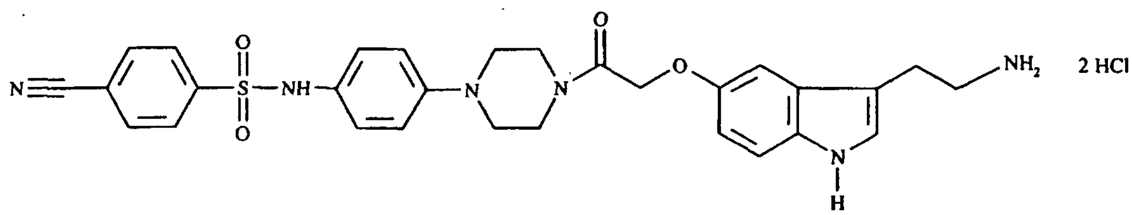

**64A** - N-[4-(4-terbutoxycarbonyl-pipérazin-1-yl-phényl)- 4-cyano-benzènesulfonamide

Le composé **26A** (3,0 g ; 10,81 mmol) en solution dans le dichlorométhane (80 ml) en présence de triéthylamine (2,21 ml ; 11,89 mmol) est traité à 0°C par le chlorure de 4-cyano-benzènesulfonyle (2,18 g ; 10,81 mmol). Après 4h d'agitation de 0°C à température ambiante du chlorure de 4-cyano-benzènesulfonyle (0,64 g ; 3,24 mmol) est à nouveau additionné. Après 2h00 la réaction est diluée au dichlorométhane, lavée à l'eau, séchée sur sulfate de sodium, filtrée et évaporée à sec. Le sirop vert foncé obtenu est chromatographié sur colonne de gel de silice éluée par un mélange dichlorométhane/acétone (25:1, v/v). Le produit pur est isolé sous forme de sirop jaune (3,28 g; 69 %).
Analyse élémentaire ($C_{22}H_{26}N_4O_4S_1$, 0,6$H_2O$), % calculés: C 58,29 ; H 6,07 ; N 12,36 ; % trouvés : C 58,17 ; H 5,76 ; N 12,03.
RMN 1H, DMSO-d6 (ppm) : 1,37 s, 9H ; 2,97 t, 4H ; 3,35 m, 4H ; 6,83 q, 4H ; 7,79 d, 2H ; 8,00 d, 2H ; 10,08 s, 1H.

**64:** Chlorhydrate de la N-[4-(4-{2-[3-(2-amino)-1H-indol-5-yloxy]-acétyl}-pipérazin-1-yl)-phényl]-4-cyano-phénylsulfonamide.

Le composé **64** est préparé à partir du produit **64A** (3,2 g ; 7,23 mmol) dans les conditions décrites pour la préparation de l'exemple **26** à partir de **26B**.
La purification du produit, sous forme de base, est effectuée par chromatographie sur colonne de gel de silice éluée par un mélange dichlorométhane/méthanol/ammoniaque (85:14:1; v/v). Le produit pur est isolé sous forme de sirop qui conduit, après traitement à l'acide chlorhydrique dans l'éther, au composé **64** (1,1 g).

**Exemple 65:** Chlorhydrate de la N-[4-(4-{2-[3-(2-amino)-1H-indol-5-yloxy]-acétyl}-pipérazin-1-yl)-phényl]-4-trifluoro-méthane-phénylsulfonamide.

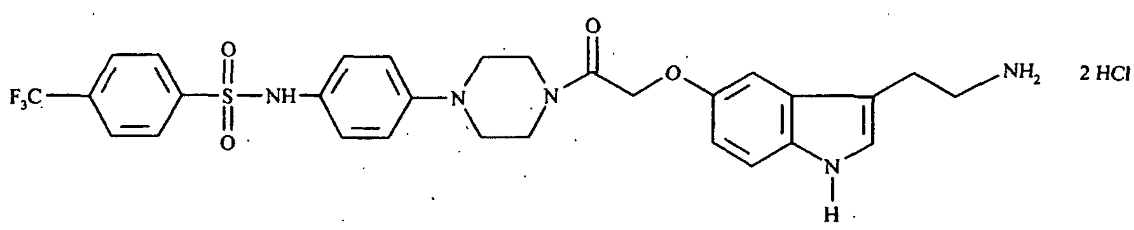

**65A** - N-[4-(4-terbutoxycarbonyl-pipérazin-1-yl-phényl)-4-trifluorométhane-benzènesulfonamide

Le composé **26A** (3,0 g ; 10,81 mmol) en solution dans le dichlorométhane (80 ml) en présence de triéthylamine (2,21 ml ; 11,89 mmol) est traité à 0°C par le chlorure de 4-trifluorométhane-benzènesulfonyle (2,64 g ; 10,81 mmol). Après 4h d'agitation de 0°C à température ambiante du chlorure de 4-cyano-benzènesulfonyle (0,75 g ; 3,24

mmol) est à nouveau additionné. Après 2h00 la réaction est diluée au dichlorométhane, lavée à l'eau, séchée sur sulfate de sodium, filtrée et évaporée à sec.

Le sirop obtenu est chromatographié sur colonne de gel de silice éluée par un mélange dichlorométhane/acétone (30:1, v/v). Le produit pur est isolé sous forme de sirop jaune (3,50 g; 67 %).

Analyse élémentaire ($C_{22}H_{26}N_3O_4S_1F_3$, 0,4$H_2O$), % calculés: C 53,63 ; H 5,48; N 8,53 ; % trouvés : C 53,61 ; H 5,61 ; N 8,40.

RMN 1H, DMSO-d6 (ppm) : 1,41 s, 9H ; 3,00 t, 4H ; 3,38 m, 4H ; 6,87 q, 4H ; 7,91 q, 4H ; 10,13 large s, 1H.

**65:** Chlorhydrate de la N-[4-(4-{2-[3-(2-amino)-1H-indol-5-yloxy]-acétyl}-pipérazin-1-yl)-phényl]-4-trifluorométhane-phénylsulfonamide.

Le composé **65** est préparé à partir du produit **65A** (3,5 g ; 7,20 mmol) dans les conditions décrites pour la préparation de l'exemple **26** à partir de **26B**.

La purification du produit, sous forme de base, est effectuée par chromatographie sur colonne de gel de silice éluée par un mélange dichlorométhane/méthanol/ammoniaque (85:14:1; v/v). Le produit pur est isolé sous forme de sirop qui conduit, après traitement à l'acide chlorhydrique dans l'éther, au composé **65** (0,97 g).

**Exemple 66:** Chlorhydrate de la N-[4-(4-{2-[3-(2-amino)-1H-indol-5-yloxy]-acétyl}-pipérazin- 1-yl)-phényl]-4-méthoxy-phénylsulfonamide.

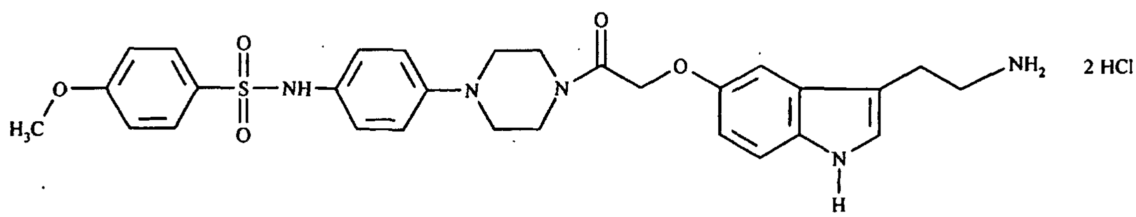

**66A-** N-[4-(4-terbutoxycarbonyl-pipérazin-1-yl-phényl)-4-méthoxy-benzènesulfonamide

Le composé **26A** (3,0 g ; 10,81 mmol) en solution dans le dichlorométhane (80 ml) en présence de triéthylamine (2,21 ml ; 11,89 mmol) est traité à 0°C par le chlorure de 4-méthoxy-benzènesulfonyle (2,23 g ; 10,81 mmol). Après 4h d'agitation de 0°C à température ambiante du chlorure de 4-méthoxy-benzènesulfonyle (0,67 g ; 3,24 mmol) est à nouveau additionné. Après 2h00 la réaction est diluée au dichlorométhane, lavée à l'eau, séchée sur sulfate de sodium, filtrée et évaporée à sec.

Le sirop obtenu est chromatographié sur colonne de gel de silice éluée par un mélange dichlorométhane/acétone (30:1, v/v). Le produit pur est isolé sous forme de sirop jaune (4,1g; 84 %).

Analyse élémentaire ($C_{22}H_{29}N_3O_5S_1$, 0,3$H_2O$), % calculés: C 58,34 ; H 6,59 ; N 9,28 ; % trouvés : C 58,38 ; H 6,49 ; N 9,08.

RMN 1H, DMSO-d6 (ppm) : 1,38 s, 9H ; 2,96 t, 4H ; 3,38 t, 4H ; 3,77 s, 3H ; 6,83 q, 4H ; 7,01 d, 2H ; 7,58 d, 2H ; 9,69 s, 1H.

**66:** Chlorhydrate de la N-[4-(4-{2-[3-(2-amino)-1H-indol-5-yloxy]-acétyl}-pipérazin-1-yl)-phényl]-4-méthoxy-phénylsulfonamide.

Le composé **66** est préparé à partir du produit **66A** (4,1 g ; 9,1 mmol) dans les conditions décrites pour la préparation de l'exemple **26** à partir de **26B**.

La purification du produit, sous forme de base, est effectuée par chromatographie sur colonne de gel de silice éluée par un mélange dichlorométhane/méthanol/ammoniaque (85:14:1; v/v). Le produit pur est isolé sous forme de sirop qui conduit, après traitement à l'acide chlorhydrique dans l'éther, au composé **66** (1,6 g).

**Exemple 67:** Chlorhydrate de la N-[4-(4-{2-[3-(2-amino)-1H-indol-5-yloxy]-acétyl}-pipérazin-1-yl)-phényl]-4-fluoro-phénylsulfonamide.

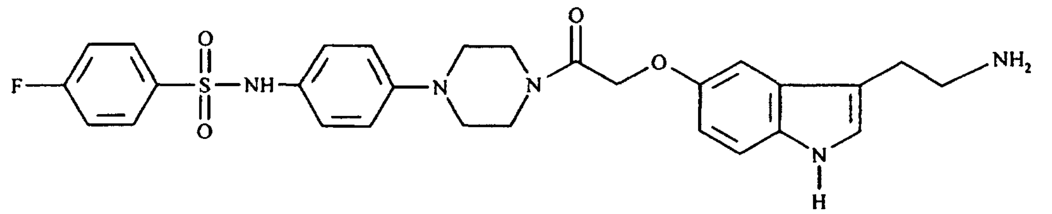

2 HCl

**67A-** N-[4-(4-terbutoxycarbonyl-pipérazin-1-yl-phényl)-4-fluoro-benzènesulfonamide

Le composé **26A** (3,0 g ; 10,81 mmol) en solution dans le dichlorométhane (80 ml) en présence de triéthylamine (2,21 ml ; 11,89 mmol) est traité à 0°C par le chlorure de 4-fluoro-benzènesulfonyle (2,1 g ; 10,81 mmol). Après 4h d'agitation de 0°C à température ambiante du chlorure de 4-fluoro-benzènesulfonyle (0,63 g ; 3,24 mmol) est à nouveau additionné. Après 2h00 la réaction est diluée au dichlorométhane, lavée à l'eau, séchée sur sulfate de sodium, filtrée et évaporée à sec. Le sirop obtenu est chromatographié sur colonne de gel de silice éluée par un mélange dichlorométhane/acétone (30:1, v/v). Le produit pur est isolé sous forme de mousse jaune (3,6 g; 76 %).
Analyse élémentaire ($C_{21}H_{26}N_3O_4S_1F_1$, 0,3$H_2O$), % calculés: C 57,21 ; H 6,08; N 9,53 ; % trouvés : C 57,36 ; H 6,01 ; N 9,45.
RMN 1H, DMSO-d6 (ppm) : 1,39 s, 9H ; 2,98 t, 4H ; 3,39 t, 4H ; 6,84 q, 4H ; 7,36 m, 2H ; 7,71 m, 2H ; 9,86 s, 1H.

**67:** Chlorhydrate de la N-[4-(4-{2-[3-(2-amino)-1H-indol-5-yloxy]-acétyl}-pipérazin-1-yl)-phényl]-4-fluoro-phénylsulfonamide.

Le composé **67** est préparé à partir du produit **67A** (3,6 g ; 8,26 mmol) dans les conditions décrites pour la préparation de l'exemple **26** à partir de **26B**.
La purification du produit, sous forme de base, est effectuée par chromatographie sur colonne de gel de silice éluée par un mélange dichlorométhane/méthanol/ammoniaque (85:**14**:1; v/v). Le produit pur est isolé sous forme de sirop qui conduit, après traitement à l'acide chlorhydrique dans l'éther, au composé **67** (1,2 g).

**Exemple 68:** Chlorhydrate de la N-[4-(4-{2-[3-(2-amino)-1H-indol-5-yloxy]-acétyl}-pipérazin-1-yl)-phényl]-4-acétamido-phénylsulfonamide.

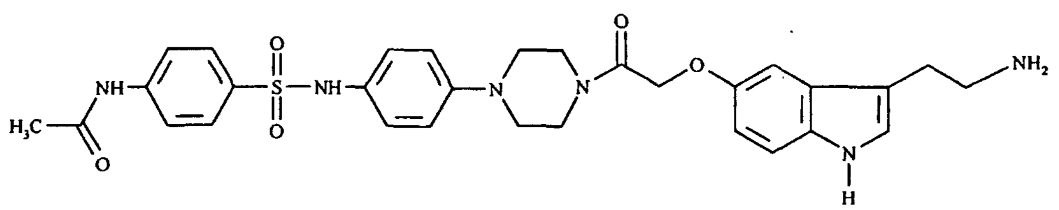

2 HCl

**68A** - N-[4-(4-terbutoxycarbonyl-pipérazin-1-yl-phényl)-4-acétamido-benzènesulfonamide

Le composé **26A** (3,0 g ; 10,81 mmol) en solution dans le dichlorométhane (80 ml) en présence de triéthylamine (2,21 ml ; 11,89 mmol) est traité à 0°C par le chlorure de 4-acétamido-benzènesulfonyle (2,5 g ; 10,81 mmol). Après 4h d'agitation de 0°C à température ambiante du chlorure de 4-acétamido-benzènesulfonyle (0,76 g ; 3,24 mmol) est à nouveau additionné. Après 1h00 la réaction est diluée au dichlorométhane, lavée à l'eau, séchée sur sulfate de sodium, filtrée et évaporée à sec.
Le sirop obtenu est chromatographié sur colonne de gel de silice éluée par un mélange dichlorométhane/acétone (6:1, v/v). Le produit pur est isolé sous forme de mousse violette (3,5 g; 69 %).
Analyse élémentaire ($C_{23}H_{30}N_4O_5S_1$, 1$H_2O$), % calculés: C 56,08 ; H 6,55 ; N 11,37 ; % trouvés : C 56,16 ; H 6,29 ;

N 11,00.
RMN 1H, DMSO-d6 (ppm) : 1,38 s, 9H ; 2,03 s, 3H ; 2,95 t, 4H ; 3,37 t, 4H ; 6,82 q, 4H ; 7,60 q, 4H ; 9,70 s, 1H ; 10,26 s, 1H.

**68:** Chlorhydrate de la N-[4-(4-{2-[3-(2-amino)-1H-indol-5-yloxy]-acétyl}-pipérazin-1-yl)-phényl]-4-acétamido-phénylsulfonamide.

Le composé **68** est préparé à partir du produit **68A** (3,6 g ; 8,26 mmol) dans les conditions décrites pour la préparation de l'exemple **26** à partir de **26B**.
La purification du produit, sous forme de base, est effectuée par chromatographie sur colonne de gel de silice éluée par un mélange dichlorométhane/méthanol/ammoniaque (80:18:2; v/v). Le produit pur est isolé sous forme de sirop qui conduit, après traitement à l'acide chlorhydrique dans l'éther, au composé **68** (1,1 g).

**Exemple 69:** Chlorhydrate de la N-[4-(4-{2-[3-(2-amino)-1H-indol-5-yloxy]-acétyl}-pipérazin-1-yl)-phényl]-4-amino-phénylsulfonamide.

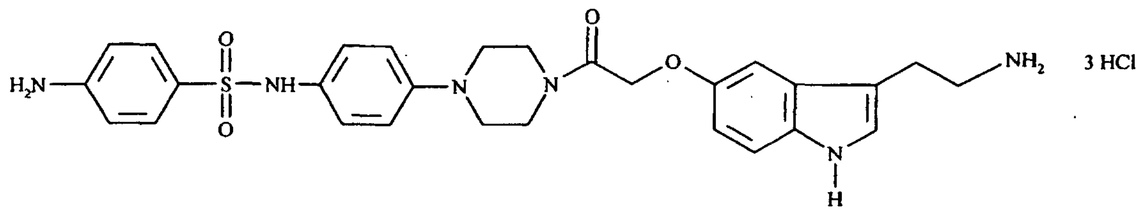

**69A** - Chlorhydrate de la N-[4-(4-{2-[3-(2-{N-terbutoxycarbonyl}-amino)-1H-indol-5-yloxy]-acétyl}-pipérazin-1-yl)-phényl]-4-amino-phénylsulfonamide.

Le composé 69A est préparé à partir du composé 26C (1,3 g ; 1.96 mmol) selon la méthode décrite pour la préparation de l'exemple 2A à partir de 1 C.
Le sirop obtenu est chromatographié sur colonne de gel de silice éluée par un mélange dichlorométhane/méthanol/ammoniaque (95:4:1; v/v). Le produit pur est isolé sous forme de mousse orange (1,2 g; 95 %).
RMN 1H, DMSO-d6 (ppm) : 1,37 s, 9H ; 2,73 t, 2H ; 3,03-3,16 m, 6H ; 3,60 large s, 4H ; 4,77 s, 2H ; 5,91 s, 2H ; 6,50 d, 2H ; 6,74-6,93 m, 6H ; 7,06 dd, 2H ; 7,20-7,33 m, 4H ; 9,41 s, 1H ; 10,66 s, 1H.

**69 -** Chlorhydrate de la N-[4-(4-{2-[3-(2-amino)-1H-indol-5-yloxy]-acétyl}-pipérazin-1-yl)-phényl]-4-amino-phénylsulfonamide.

Le composé **69A** (560 mg ; 0,86 mmol) est ensuite déprotégé dans les conditions décrites pour la préparation de l'exemple **1** à partir de **1C**.
La purification du produit, sous forme de base, est effectuée par chromatographie sur colonne de gel de silice éluée par un mélange dichlorométhane/méthanol/ammoniaque (80:18:2; v/v). Le produit pur est isolé sous forme de sirop incolore qui conduit, après traitement à l'acide chlorhydrique dans l'éther, au composé **69** sous forme de poudre blanche (407 mg; 72 %).
Analyse élémentaire ($C_{28}H_{35}Cl_3N_6O_4$, 3,2$H_2O$), % calculés: C 46,99 ; H 5,83 ; N 11,74 ; Cl 14,86; % trouvés: C 46,95 ; H 5,82 ; N 11,42 ; Cl 14,37.
RMN 1H, DMSO-d6 (ppm): 2,96 large s, 4H; 3,23 large d, 4H; 3,71 m, 4H; 4,79 s, 2H; 6,56 d, 2H; 6,75 dd, 1H; 6,92-7,38 m, 9H; 8,01 large s, 3H; 9,75 s, 1H; 10,82s, 1H.
Point de fusion : 186°C

**Exemple 70:** Chlorhydrate de la N-[4-(4-{2-[3-(2-amino)-1H-indol-5-yloxy]-acétyl}-pipérazin-1-yl)-phényl]-4-méthane-sulfonylamino-phénylsulfonamide.

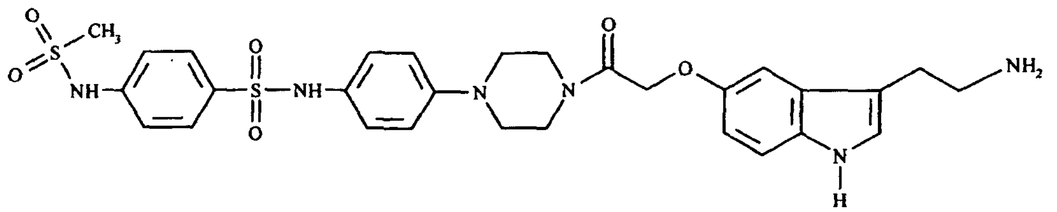

2 HCl

Le composé **70** est préparé à partir du composé **69A** (300 mg ; 0,46 mmol) et de chlorure de méthanesulfonyle (36 µl ; 1,46 mmol) selon la méthode décrite pour la préparation de l'exemple **3.**

Le sirop obtenu est chromatographié sur colonne de gel de silice éluée par un mélange dichlorométhane/méthanol/ammoniaque (80:18:2 ; v/v). Le produit pur est isolé sous forme de sirop qui conduit, après traitement à l'acide chlorhydrique dans l'éther, au composé **70** (30 mg; 10 %).

## ETUDE D'AFFINITE POUR LES RECEPTEURS 5-$HT_{1D}$

Cette étude est réalisée selon la technique décrite par Pauwells et al. (Biochem. Pharmacol. 46, 535-538, 1993).

### Préparation des membranes

Des cerveaux de mouton sont prélevés à l'abattoir local et transportés dans la glace. Les noyaux caudés sont prélevés, pesés et homogénéisés au Polytron pendant 20 sec (vitesse 6-7) dans 20 volumes de Tris-HCl 50 mM, pH 7,7. L'homogénat est centrifugé 10 mn à 48000 g avec une centrifugeuse L5 50E (Beckman).

Le culot repris par 20 volumes de Tris-HCl 50 mM, pH 7,7 est placé dans un bain-marie à 37°C pendant 10 mn, puis recentrifugé 10 mn à 48000 g. Le culot alors obtenu est aussitôt congelé en fractions de 0,5 g de tissu.

### Affinités

Le culot est décongelé et homogénéisé au Dounce dans 80 volumes de Tris-HCl, 50 mM pH 7,7 contenant 4 mM $CaCl_2$, 10 µM de pargyline et 0,1 % d'acide ascorbique. L'affinité est réalisée à 25°C en incubant pendant 30 mn :

- 0,1 ml de tampon ou 10 µM en concentration finale de sumatriptan pour obtenir le binding non spécifique
- 0,8 ml de membrane
- 0,1 ml de 3H-5HT (15 à 30 Ci:mM, New England Nuclear France)

L'incubation est terminée par la filtration rapide sur filtres GF/B et rinçage avec 3 fois 3 ml de tampon glacé, à l'aide d'un harvester de fabrication Brandel permettant de filtrer 48 échantillons. Les filtres sont introduits dans des fioles contenant 4 ml de liquide scintillant emulsifier-safe (Packard) et la radioactivité mesurée avec un compteur Tri-carb 4640 (Packard).L'$IC_{50}$ (concentration qui inhibe de 50 % l'affinité spécifique) est déterminée graphiquement.

Les affinités des différents produits faisant partie de cette invention pour récepteurs $5HT_{1A}$ et $5HT_{1B}$ ont été mesurées selon les techniques décrites dans :

- Peroutka S.J. Pharmacological differentiation and characterization of $5HT_{1A}$, $5HT_{1B}$ and $5HT_{1C}$ binding sites in rat frontal cortex. J. Neurochem., 45, 529-540, 1986.

| Exemples du profil d'affinité de quelques molécules ($CI_{50}$ x 10-9M) | | | |
|---|---|---|---|
| Exemple * | 5-$HT_{1D}$ | 5-$HT_{1A}$ | Ratio # |
| | Moyenne | Moyenne | 1A/1D |
| 4 comparatif | 1 | 75 | 75 |
| 6 comparatif | 4,3 | 79 | 18 |
| 7 | 1,6 | 44 | 28 |
| 8 | 1,2 | 26 | 22 |
| 9 comparatif | 5 | 38 | 8 |
| 10 comparatif | 1 | 20 | 20 |
| 11 comparatif | 2,4 | 105 | 44 |
| 14 | 1 | 9,5 | 10 |

* Les exemples sont ceux décrits dans le texte pour illustrer l'invention

# Rapport des $CI_{50}$ de chaque produit pour les récepteurs 5-$HT_{1A}$ et 5-$HT_{1D}$

ETUDE DE L'ACTIVITE AGONISTE AU NIVEAU DES RECEPTEURS 5-$HT_{1B}$

Les mesures ont été effectuées au niveau de l'inhibition de la formation de la cAMP stimiulée par la forskoline, médiée par une récepteur 5-$HT_{1B}$, dans une lignée cellulaire OK épithéliale de rein comme décrit par ailleurs (P.J. Pauwels, C. Palmier, Neuropharmacology, sous presse).

Les études effectuées montrent que la plupart des produits de l'invention, comme la sérotonine, sont capables de bloquer efficacement la formation de cAMP dans ce test. C'est ainsi que les exemples 1, 2, 3 (comparatif) et 5 possèdent une $CE_{50}$ comprise entre 0.3 et 1.2 nM.

<u>Etude de l'affinité et de l'activité agoniste au niveau des récepteurs humains</u>

Les récepteurs humains $5HT_{1Da}$ et $5HT_{1Db}$ ont été clonés selon les séquences publiées par M. Hamblin et M. Metcalf, Mol. Pharmacol., **<u>40</u>**,143 (1991) et Weinshenk et coll., Proc. Natl. Acad. Sci. **<u>89</u>**,3630 (1992).

La transfection transitoire et la transfection permanente des gènes de ces récepteurs a été réalisée dans des lignées cellulaires Cos-7 et CHO-$K_1$ en utilisant un électroporateur.

La lignée cellulaire HeLa HA7 exprimant le récepteur $5HT_{1A}$ humain a été obtenue de Tulco (Duke Univ., Durham, N.C., USA) et cultivée selon la méthode de Fargin et coll., J. Biol. Chem. **<u>264</u>**,14848 (1989).

L'étude de la liaison des dérivés de la présente invention avec les récepteurs $5HT_{1Da}$ , $5HT_{1Db}$ et $5HT_{1A}$ humains a été réalisée selon la méthode décrite par P. Pauwels et C. Palmier (Neuropharmacology, **<u>33</u>**,67,1994).

Les milieux d'incubation pour ces mesures de liaison comprennent 0.4 ml de préparation de membrane cellulaire, 0.05ml d'un ligand tritié [[3H]-5CT (concentration finale : 2nM) pour les récepteurs $5HT_{1Da}$ et $5HT_{1Db}$ et [3H]-8OH-DPAT (concentration finale: 1 nM) pour le récepteur $5HT_{1A}$] et 0.05 ml de la molécule à tester (concentrations finales de 0.1 nM à 1000 nM) ou 10 μM (concentration finale) de sérotonine ($5HT_{1Da}$ et $5HT_{1Db}$) ou 1 μM (concentration finale) de spiroxatrine ($5HT_{1A}$).

L'étude de l'inhibition de la formation d'AMP cyclique (stimulée par la forskoline) médiée par le récepteur $5HT_{1Db}$ humain a été réalisée dans les cellules CHO-K1 transfectées par le récepteur selon la technique décrite préalablement pour le récepteur $5HT_{1B}$ (P. Pauwels et C. Palmier, Neuropharmacology, **<u>33</u>**,67,1994).

Illustration du profil de quelques molécules de la présente invention

| | Ki(nM) | | | |
|---|---|---|---|---|
| Exemple | $5HT_{1Da}$ | $5HT_{1Db}$ | $5HT_{1A}$ | $EC_{50}$*(nM) |
| 1 | 0.5 | 1 | 25 | 1.2 |
| 3 comparatif | 1.7 | 3.3 | 78.6 | 20 |
| 5 | 0.7 | 3.2 | 73 | 6.5 |
| 26 comparatif | 0.25 | 0.5 | 16.5 | 6.5 |
| 33 comparatif | 2.8 | 2 | 39 | 9 |
| 41 invention | 1.1 | 0.7 | 28 | 2.6 |
| 59 invention | 1.3 | 1.3 | 9.3 | 0.52 |

*Inhibition de la cyclase $5HT_{1Db}$ stimulée par la forskoline

Les quelques exemples décrits ci-dessus montrent clairement que les produits de la présente invention sont d'excellents ligands pour les récepteurs humains $5HT_{1Da}$ et $5HT_{1Db}$ et sont d'excellents agonistes au niveau du récepteur $5HT_{1Db}$ humain.

Les nouveaux composés indoliques dérivés d'arylpipérazines faisant partie de cette invention sont des ligands ayant une affinité exceptionnelle pour les récepteurs 5 $HT_{1D}$ et 5 $HT_{IB}$ comme le démontrent les exemples décrits cidessus. De nombreux composés, faisant partie intégrante de la présente invention, présentent en outre l'avantage d'être particulièrement sélectifs pour le récepteur 5 $HT_{1D}$ par rapport aux récepteurs 5 $HT_{1A}$, 5 $HT_{1C}$, 5 $HT_2$, $\alpha_1$, $\alpha_2$, $D_2$. La sélectivité des composés de la présente invention et en particulier leur affinité préférentielle pour le récepteur 5 $HT_{1D}$ par rapport au récepteur 5 $HT_{1A}$ représente un avantage très important par rapport aux ligands du récepteur 5 $HT_{1D}$ connus jusqu'ici (cf. Annual Reports in Medicinal Chemistry, vol 27, chapitre 3, p.25 ; Academic Press, 1992).

En thérapeutique humaine, les composés de formule générale (I) selon l'invention sont particulièrement utiles pour le traitement et la prévention des désordres liés à la sérotonine au niveau du système nerveux central et du système vasculaire. Ces composés peuvent donc être utilisés dans le traitement et la prévention de la dépression, des désordres compulsifs obsessionnels, des désordres alimentaires tels que la boulimie et l'anorexie, de l'agressivité, de l'alcoolisme, du tabagisme, de l'hypertension, de la nausée, du dysfonctionnement sexuel, du comportement asocial, de l'anxiété, de la migraine, de la maladie d'Alzheimer et des troubles de la mémoire.

La présente invention concerne également les médicaments constitués par au moins un composé de formule (I) à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semisynthétiques ou des polyéthylèneglycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée; elles sont généralement comprises entre 0,001 g et 1 g (de préférence comprises entre 0,005 g et 0,25 g) par jour de préférence par voie orale pour un adulte avec des doses unitaires allant de 0,1 mg à 500 mg de substance active, de préférence de 1 mg à 50 mg.

D'une façon générale, le médecin déterminera la posologie appropriée en fonction de l'âge, du poids et de tous les autres facteurs propres au sujet à traiter. Les exemples suivants illustrent des compositions selon l'invention [dans ces exemples, le terme "composant actif" désigne un ou plusieurs (généralement un) des composés de formule (I) selon la présente invention]:

Comprimés

On peut les préparer par compression directe ou en passant par une granulation au mouillé. Le mode opératoire par compression directe est préféré mais il peut ne pas convenir dans tous les cas selon les doses et les propriétés physiques du composant actif.

| A - Par compression directe | |
|---|---|
| | mg pour 1 comprimé |
| composant actif | 10,0 |
| cellulose microcristalline B.P.C. | 89,5 |
| stéarate de magnésium | 0,5 |
| | 100,0 |

On passe le composant actif au travers d'un tamis à ouverture de maille de 250 μm de côté, on mélange avec les excipients et on comprime à l'aide de poinçons de 6,0 mm.

On peut préparer des comprimés présentant d'autres résistances mécaniques en modifiant le poids de compression avec utilisation de poinçons appropriés.

| B - Granulation au mouillé | |
|---|---|
| | mg pour un comprimé |
| composant actif | 10,0 |
| lactose Codex | 74,5 |
| amidon Codex | 10,0 |
| amidon de maïs prégélatinisé Codex | 5,0 |
| stéarate de magnésium | 0,5 |
| Poids à la compression | 100,0 |

On fait passer le composant actif au travers d'un tamis à ouverture de maille de 250 μm et on mélange avec le lactose, l'amidon et l'amidon prégélatinisé. On humidifie les poudres mélangées par de l'eau purifiée, on met à l'état de granulés, on sèche, on tamise et on mélange avec le stéarate de magnésium. Les granulés lubrifiés sont mis en comprimés comme pour les formules par compression directe. On peut appliquer sur les comprimés une pellicule de revêtement au moyen de matières filmogènes appropriées, par exemple la méthylcellulose ou l'hydroxy-propyl-méthylcellulose, selon des techniques classiques. On peut également revêtir les comprimés de sucre.

| Capsules | |
|---|---|
| | mg pour une capsule |
| composant actif | 10,0 |

(suite)

| Capsules | |
|---|---|
| | mg pour une capsule |
| *amidon 1500 | 89,5 |
| stéarate de magnésium Codex | 0,5 |
| Poids de remplissage | 100,0 |

*une forme d'amidon directement compressible provenant de la firme Colorcon Ltd, Orpington, Kent, Royaume Uni.

On fait passer le composant actif au travers d'un tamis à ouverture de maille de 250 µm et on mélange avec les autres substances. On introduit le mélange dans des capsules de gélatine dure n°2 sur une machine à remplir appropriée. On peut préparer d'autres unités de dosage en modifiant le poids de remplissage et, lorsque c'est nécessaire, en changeant la dimension de la capsule.

Sirop

| | | mg par dose de 5 ml |
|---|---|---|
| composant actif | | 10,0 |
| saccharose Codex | | 2750,0 |
| glycérine Codex | | 500,0 |
| tampon | ) | |
| arôme | ) | |
| colorant | ) | q.s. |
| préservateur | ) | |
| eau distillée | | 5,0 |

On dissout le composant actif, le tampon, l'arôme, le colorant et le préservateur dans une partie de l'eau et on ajoute la glycérine. On chauffe le restant de l'eau à 80°C et on y dissout le saccharose puis on refroidit. On combine les deux solutions, on règle le volume et on mélange. Le sirop obtenu est clarifié par filtration.

| Suppositoires | | |
|---|---|---|
| Composant actif | | 10,0 mg |
| * Witepsol H15 | complément à | 1,0 g |

* Marque commercialisée pour Adeps Solidus de la Pharmacopée Européenne.

On prépare une suspension du composant actif dans le Witepsol H15 et on l'introduit dans une machine appropriée avec moules à suppositoires de 1 g.

| Liquide pour administration par injection intraveineuse | | |
|---|---|---|
| | | g/l |
| composant actif | | 2,0 |
| eau pour injection Codex | complément à | 1000,0 |

On peut ajouter du chlorure de sodium pour régler la tonicité de la solution et régler le pH à la stabilité maximale et/ou pour faciliter la dissolution du composant actif au moyen d'un acide ou d'un alcali dilué ou en ajoutant des sels tampons appropriés. On prépare la solution, on la clarifie et on l'introduit dans des ampoules de dimension appropriée

qu'on scelle par fusion du verre. On peut également stériliser le liquide pour injection par chauffage à l'autoclave selon l'un des cycles acceptables. On peut également stériliser la solution par filtration et introduire en ampoule stérile dans des conditions aseptiques. La solution peut être introduite dans les ampoules en atmosphère gazeuse.

| Cartouches pour inhalation | |
|---|---|
| | g/cartouche |
| composant actif mironisé | 1,0 |
| lactose Codex | 39,0 |

Le composant actif est micronisé dans un broyeur à énergie de fluide et mis à l'état de fines particules avant mélange avec du lactose pour comprimés dans un mélangeur à haute énergie. Le mélange pulvérulent est introduit en capsules de gélatine dure n°3 sur une machine à encapsuler appropriée. Le contenu des cartouches est administré à l'aide d'un inhalateur à poudre.

| Aérosol sous pression à valve doseuse | | |
|---|---|---|
| | mg/dose | pour 1 boîte |
| composant actif micronisé | 0,500 | 120 mg |
| acide oléique Codex | 0,050 | 12 mg |
| trichloroofluorométhane pour usage pharmaceutique | 22,25 | 5,34 g |
| dichlorodifluorométhane pour usage pharmaceutique | 60,90 | 14,62 g |

Le composant actif est micronisé dans un broyeur à énergie de fluide et mis à l'état de fines particules. On mélange l'acide oléique avec le trochlorofluorométhane à une température de 10-15°C et on introduit dans la solution à l'aide d'un mélangeur à haut effet de cisaillement le médicament micronisé. La suspension est introduite en quantité mesurée dans des boîtes aérosol en aluminium sur lesquelles on fixe des valves doseuses appropriées délivrant une dose de 85 mg de la suspension ; le dichlorodifluorométhane est introduit dans les boîtes par injection au travers des valves.

## Revendications

1. Composés répondant à la formule générale (I)

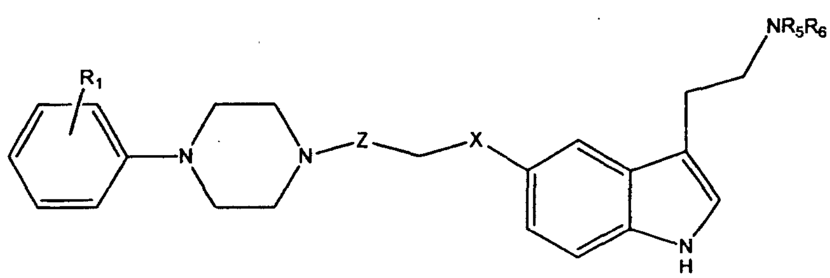

(I)

dans laquelle :

$R_1$ représente un radical $NH_2$, $NO_2$, $NR_5R_6$, $NHSO_2R_7$ ou CN, pouvant être en position o, m ou p sur le cycle aromatique
Z représente C=O, $(CH_2)_n$ ou encore $CO(CH_2)_n$; n étant compris entre 1 et 5
X représente $CH_2$ ou O
$R_5$ et $R_6$ identiques ou différents représentent chacun un atome d'hydrogène, un radical alkyle linéaire ou ramifié, comprenant de 1 à 6 atomes de carbone, de préférence un méthyle, éthyle, n-propyle, isopropyle, n-butyle, s-butyle, ou t-butyle

$R_7$ représente un radical alkyle linéaire ou ramifié comprenant de 1 à 5 atomes de carbone, ou un hétérocycle à 5 atomes contenant un ou plusieurs atome(s) de soufre

ou $R_7$ représente -$NMe_2$ quand $R_5$ et $R_6$ représentent un atome d'hydrogène et Z représente C=O, leurs sels et solvats acceptables pour l'usage thérapeutique.

**2.** Un composé de formule (I) selon la revendication 1, sélectionné parmi:

2-[3-(2-amino-éthyl)-1-H-indol-5-yloxy]-1-[4-(4-nitro-phényl)-pipérazin-1-yl]-éthanone.
2-[3-(2-amino-éthyl)-1H-indol-5-yloxy]-1-[4-(4-amino-phényl)-pipérazin-1-yl)-éthanone.
N-[4-(4-{2-[3-(2-amino-éthyl)-1H-indol-5-yloxy]-acétyl}-pipérazin-1-yl)phényl]-méthanesulfonamide.
thiophène-2-{N-[4-(4-{2-[3-(2-amino-éthyl)-1H-indol-5-yloxy]-acétyl}-pipérazin-1-yl)phényl]-sulfonamide.
3-[3-(2-amino-éthyl)-1H-indol-5-yl]-1-[4-(4-nitro-phényl)-pipérazin-1-yl]-propan-1-one.
3-[3-(2-amino-éthyl)-1H-indol-5-yl]-1-[4-(4-amino-phényl)-pipérazin-1-yl]-propan-1-one.
N-[4-(4- {3-[3-(2-amino-éthyl)-1H-indol-5-yl]-propionyl}-pipérazin-1-yl)-phényl]-N-diméthylsulfonurée.
4-(4-{2-[3-(2-amino-éthyl)-1H-indol-5-yloxy]-éthyl]}-pipérazin-1-yl)-phénylamine.
N-[4-(4-{2-[3-(2-amino-éthyl)-1H-indol-5-yloxy]-éthyl}-pipérazin-1-yl)-phényl]-méthanesulfonamide.
4-(4- {2-[3-(2-amino-éthyl)-1H-indol-5-yloxy]-éthyl}-pipérazin-1-yl)-benzonitrile.
2-(4-{2-[3-(2-amino-éthyl)-1H-indol-5-yloxy]-acétyl}-pipérazin-1-yl)-benzonitrile.
4-(4-{2-[3-(2-amino-éthyl)-1H-indol-5-yloxy]-acétyl}-pipérazin-1-yl)-benzonitrile.
6-[3-(2-amino-éthyl)-1H-indol-5-yloxy]-1-[4-(4-amino-phényl)-pipérazin-1-yl]-hexan-1-one.
N-[4-(4- {6-[3-(2-amino-éthyl)-1H-indol-5-yloxy]-hexanoyl}-pipérazin-1-yl)-phényl]-méthanesulfonamide.
5-[3-(2-amino-éthyl)-1H-indol-5-yloxy]-1-[4-(4-amino-phényl)-pipérazin-1-yl]-pentan-1-one.

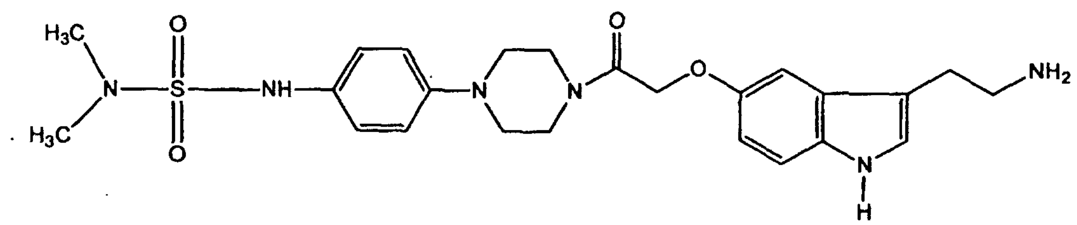

leurs sels et solvats acceptables pour l'usage thérapeutique.

**3.** Procédé de préparation des composés de formule (I) selon la revendication 1 pour lesquels $R_6$ représente un hydrogène, **caractérisé en ce que** l'on transforme un carbonate de formule (II)

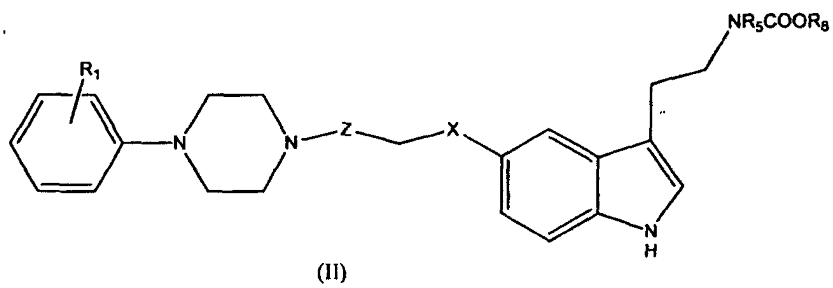

(II)

dans laquelle $R_1$, Z, X et $R_5$ sont définis comme dans la revendication 1, et $R_8$ représente un radical t-butyle ou benzyle.

**4.** Procédé de préparation des composés de formule (I) selon la revendication 1, **caractérisé en ce que** l'on fait réagir une pipérazine de formule générale (III)

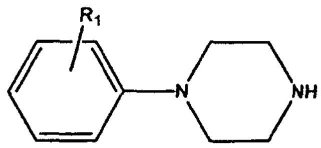

(III)

dans laquelle $R_1$ est défini comme dans la revendication 1 avec un intermédiaire indolique de formule générale (IV)

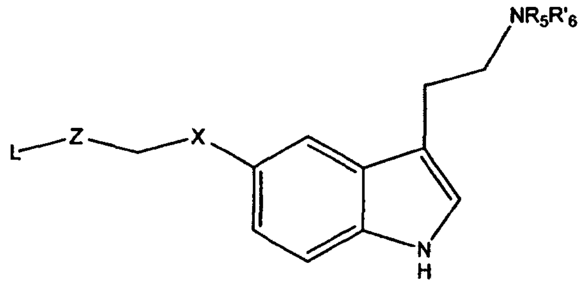

(IV)

dans laquelle Z, X, $R_5$ sont définis comme dans la revendication 1, $R'_6$ représente $R_6$, $R_6$ étant défini comme dans la revendication 1, L représente un groupe partant avantageusement choisi dans le groupe constitué par un halogène (chlore, brome ou iode) ou un radical O-mésyle, O-trifluorométhane sulfonyle ou O-tosyle.

5. Procédé de préparation des composés de formule (I) selon la revendication 1, **caractérisé en ce que** l'on fait réagir une pipérazine de formule générale (III) dans laquelle $R_1$ est défini comme dans la revendication 1 avec un intermédiaire indolique de formule générale (IV) dans laquelle Z, X, $R_5$ sont définis comme dans la revendication 1, $R'_6$ représente $CO_2R_8$, $R_8$ étant défini comme dans la revendication 3, L représente un groupe partant avantageusement choisi dans le groupe constitué par un halogène (chlore, brome ou iode) ou un radical O-mésyle, O-trifluorométhane sulfonyle ou O-tosyle, et on obtient un composé de formule (II) que l'on transforme en composé de formule (I) par le procédé selon la revendication 3.

6. Procédé de préparation des composés de formule (I) selon la revendication 1, **caractérisé en ce que** l'on fait réagir un intermédiaire de formule générale (V)

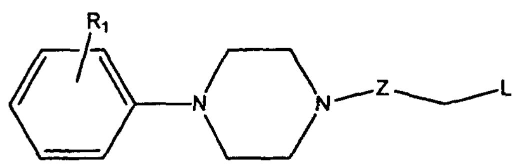

(V)

dans laquelle $R_1$ et Z sont définis comme dans la revendication 1, et L représente un groupe partant avantageusement choisi dans le groupe constitué par un halogène (de préférence un atome de brome, d'iode ou de chlore), un O-mésylate, O-triflate ou O-tosylate,
avec un dérivé de la sérotonine de formule générale (VI)

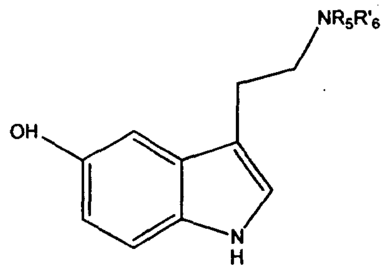

(VI)

dans laquelle $R'_6$ représente $R_6$, $R_6$ étant défini comme dans la revendication 1, et $R_5$ est défini comme dans la revendication 1.

**7.** Procédé de préparation des composés de formule (I) selon la revendication 1, **caractérisé en ce que** l'on fait réagir un intermédiaire de formule générale (V) dans laquelle $R_1$ et Z sont définis comme dans la revendication 1, et L représente un groupe partant avantageusement choisi dans le groupe constitué par un un halogène (de préférence un atome de brome, d'iode ou de chlore), un O-mésylate, O-triflate ou O-tosylate,
avec un dérivé de la sérotonine de formule générale (VI) dans laquelle $R'_6$ représente $CO_2R_8$, $R_8$ étant défini comme dans la revendication 3, et $R_5$ est défini comme dans la revendication 1, et on obtient un composé de formule (II) que l'on transforme en composé de formule (I) par le procédé selon la revendication 3.

**8.** Procédé de préparation des composés de formule (I) selon la revendication 1 pour lesquels Z représente CO et X représente $CH_2$ qui consiste à condenser un acide $\alpha$, $\beta$ insaturé de structure générale (VII)

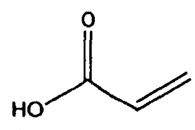

(VII)

successivement avec un dérivé 5-bromoindolique de structure générale (VIII)

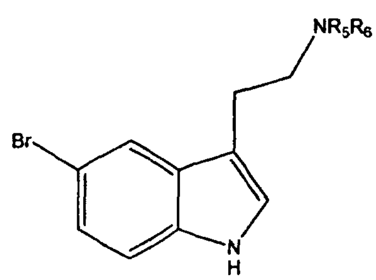

(VIII)

tel que $R_5$ est défini comme dans la revendication 1, et $R_6$ est défini comme dans la revendication **1**
et après activation de la fonction acide en chlorure d'acide ou ester activé, avec un dérivé de pipérazine de structure générale (III), suivi de la réduction de la double liaison par hydrogénation sur palladium/charbon.

**9.** Procédé de préparation des composés de formule (I) selon la revendication 1 pour lesquels $R_1$ représente $NH_2$ qui consiste soit à réduire un composé de formule (I) dans laquelle $R_1$ représente $NO_2$, soit à réduire un composé de formule (II) dans laquelle $R_1$ représente $NO_2$ puis à transformer le composé obtenu en un composé de formule (I) par le procédé selon la revendication 3.

**10.** Procédé de préparation des composés de formule (I) selon la revendication 1 pour lesquels $R_1$ représente $NR_5R_6$, $NHSO_2R_7$, $R_5$, $R_6$ et $R_7$ étant définis comme dans la revendication 1, qui consiste soit à fonctionnaliser un dérivé

correspondant de formule (I) dans laquelle $R_1$ représente $NH_2$, soit à fonctionnaliser un dérivé correspondant de formule (II) dans laquelle $R_1$ représente $NH_2$ puis à transformer le composé obtenu en un composé de formule (I) par le procédé selon la revendication 3.

**11.** Procédé de préparation des composés de formule (I) selon la revendication 1 **caractérisé en ce que** l'on convertit un composé de formule générale (I) selon la revendication 1 ou un sel ou un dérivé comportant un groupe protecteur d'un tel composé, en un autre composé de formule générale (I).

**12.** Compositions pharmaceutiques contenant, à titre d'ingrédients actifs, un composé selon l'une des revendications 1 et 2, en combinaison avec un véhicule pharmaceutique acceptable, pour le traitement tant curatif que préventif des désordres liés à la sérotonine.

**13.** Compositions pharmaceutiques contenant, à titre d'ingrédients actifs, un composé selon l'une des revendications 1 et 2, en combinaison avec un véhicule pharmaceutique acceptable, pour le traitement tant curatif que préventif des crises de migraine, de l'algie vasculaire de la face et des céphalées chroniques vasculaires.

**14.** Compositions pharmaceutiques contenant, à titre d'ingrédients actifs, un composé selon l'une des revendications 1 et 2, en combinaison avec un véhicule pharmaceutique acceptable, pour le traitement tant curatif que préventif de la dépression, des désordres compulsifs obsessionnels, de l'anxiété et des attaques de panique, de la schizophrénie, de l'agressivité et/ou de l'alcoolisme et/ou du comportement asocial, des désordres alimentaires tels que la boulimie et l'anorexie, des dysfonctionnements sexuels, et des maladies neurodégénératives telles que par exemple la maladie de Parkinson ou la maladie d'Alzheimer.

## Patentansprüche

**1.** Verbindungen, welche der allgemeinen Formel (I) entsprechen

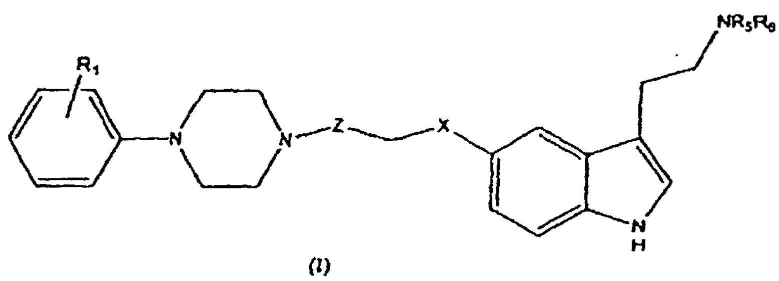

(I)

in welcher:

$R_1$ für einen Rest $NH_2$, $NO_2$, $NR_5R_6$, $NHSO_2R_7$ oder CN, welcher sich in o-, m- oder p-Position an dem aromatischen Ring befinden kann, steht,
Z für C=O, $(CH_2)_n$ oder ferner $CO(CH_2)_n$ steht; wobei n zwischen 1 und 5 beträgt;
X für $CH_2$ oder O steht,
$R_5$ und $R_6$, die gleich oder verschieden sind, jeweils für ein Wasserstoffatom, einen linearen oder verzweigten Alkylrest, welcher 1 bis 6 Kohlenstoffatome umfasst, vorzugsweise einen Methyl-, Ethyl-, n-Propyl-, Isopropyl-, n-Butyl-, sek.-Butyloder tert.-Butylrest stehen,
$R_7$ für einen linearen oder verzweigten Alkylrest, welcher 1 bis 5 Kohlenstoffatome umfasst, oder einen Heterocyclus mit 5 Atomen, welcher ein oder mehrere Schwefelatom(e) enthält, steht oder $R_7$ für $-NMe_2$ steht, wenn $R_5$ und $R_6$ für ein Wasserstoffatom stehen und Z für C=O steht,

deren Salze und Solvate, welche für eine therapeutische Verwendung annehmbar sind.

**2.** Verbindung der Formel (I) gemäß Anspruch 1, ausgewählt unter:

2-[3-(2-Aminoethyl)-1-H-indol-5-yloxy]-1-[4-(4-nitrophenyl)-piperazin-1-yl]-ethanon.
2-[3-(2-Aminoethyl)-1H-indol-5-yloxy]-1-[4-(4-aminophenyl)-piperazin-1-yl]-ethanon.

N-[4-(4-{2-[3-(2-Aminoethyl)-1H-indol-5-yloxy]-acetyl}piperazin-1-yl)phenyl]-methansulfonamid.
Thiophen-2-{N-[4-(4-{2-[3-(2-Aminoethyl)-1H-indol-5-yloxy]-acetyl}-piperazin-1-yl)phenyl]-sulfonamid.
3-[3-(2-Aminoethyl)-1H-indol-5-yl]-1-[4-(4-nitrophenyl)-piperazin-1-yl]-propan-1-on.
3-[3-(2-Aminoethyl)-1H-indol-5-yl]-1-[4-(4-aminophenyl)-piperazin-1-yl]-propan-1-on.
N-[4-(4-{3-[3-(2-Aminoethyl)-1H-indol-5-yl]-propionyl}piperazin-1-yl)-phenyl]-N-dimethylsulfonharnstoff.
4-(4-{2-[3-(2-Aminoethyl)-1H-indol-5-yloxy]-ethyl}-piperazin-1-yl)-phenylamin.
N-[4-(4-{2-[3-(2-Aminoethyl)-1H-indol-5-yloxy]-ethyl}piperazin-1-yl)-phenyl]-methansulfonamid.
4-(4-{2-[3-(2-Aminoethyl)-1H-indol-5-yloxy]-ethyl}-piperazin-1-yl)-benzonitril.
2-(4-{2-[3-(2-Aminoethyl)-1H-indol-5-yloxy]-acetyl}-piperazin-1-yl)-benzonitril.
4-(4-{2-[3-(2-Aminoethyl)-1H-indol-5-yloxy]-acetyl}-piperazin-1-yl)-benzonitril.
6-[3-(2-Aminoethyl)-1H-indol-5-yloxy]-1-[4-(4-aminophenyl)-piperazin-1-yl]-hexan-1-on.
N-[4-(4-{6-[3-(2-Aminoethyl)-1H-indol-5-yloxy]-hexanoyl}piperazin-1-yl)-phenyl]-methansulfonamid.
5-[3-(2-Aminoethyl)-1H-indol-5-yloxy]-1-[4-(4-aminophenyl)-piperazin-1-yl]-pentan-2-on.

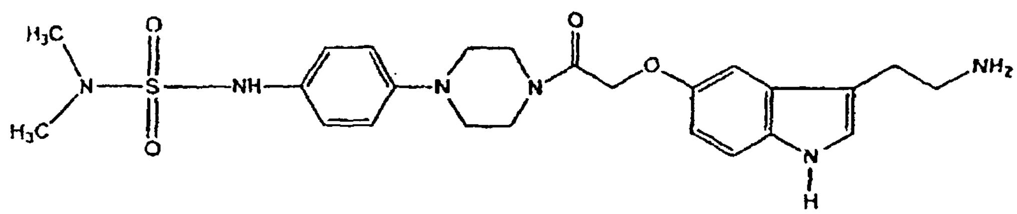

deren Salzen und Solvaten, welche für eine therapeutische Verwendung annehmbar sind.

**3.** Verfahren zur Herstellung der Verbindungen der Formel (I) gemäß Anspruch 1, für die $R_6$ für ein Wasserstoffatom steht, **dadurch gekennzeichnet, dass** man ein Carbonat der Formel (II)

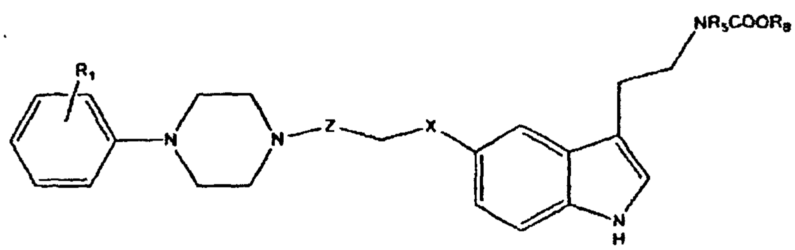

(II)

in welcher $R_1$, Z, X und $R_5$ wie in Anspruch 1 definiert sind und $R_8$ für einen tert.-Butyl- oder Benzylrest steht, umwandelt.

**4.** Verfahren zur Herstellung der Verbindungen der Formel (I) gemäß Anspruch 1, **dadurch gekennzeichnet, dass** man ein Piperazin der allgemeinen Formel (III)

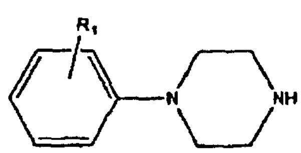

(III)

in welcher $R_1$ wie in Anspruch 1 definiert ist, mit einem indolischen Zwischenprodukt der allgemeinen Formel (IV)

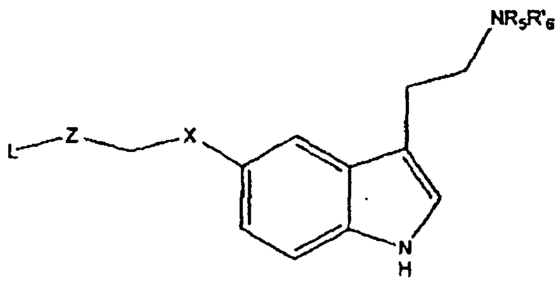

(IV)

in welcher Z, X, $R_5$ wie in Anspruch 1 definiert sind, $R'_6$ für $R_6$ steht, wobei $R_6$ wie in Anspruch 1 definiert ist, L für eine austretende Gruppe steht, welche vorteilhafterweise in der Gruppe, welche aus einem Halogen (Chlor, Brom oder Iod) oder einem O-Mesyl-, O-Trifluormethansulfonyl- oder O-Tosylrest gebildet wird, ausgewählt wird, reagieren lässt.

5. Verfahren zur Herstellung der Verbindungen der Formel (I) gemäß Anspruch 1, **dadurch gekennzeichnet, dass** man ein Piperazin der allgemeinen Formel (III), in welcher $R_1$ wie in Anspruch 1 definiert ist, mit einem indolischen Zwischenprodukt der allgemeinen Formel (IV), in welcher Z, X, $R_5$ wie in Anspruch 1 definiert sind, $R'_6$ für $CO_2R_8$ steht, wobei $R_8$ wie in Anspruch 3 definiert ist, L für eine austretende Gruppe steht, welche vorteilhafterweise in der Gruppe, welche aus einem Halogen (Chlor, Brom oder Iod) oder einem O-Mesyl-, O-Trifluormethansulfonyl- oder O-Tosylrest gebildet wird, ausgewählt wird, reagieren lässt und man eine Verbindung der Formel (II) erhält, welche man in eine Verbindung der Formel (I) durch das Verfahren gemäß Anspruch 3 umwandelt.

6. Verfahren zur Herstellung der Verbindungen der Formel (I) gemäß Anspruch 1, **dadurch gekennzeichnet, dass** man ein Zwischenprodukt der allgemeinen Formel (V)

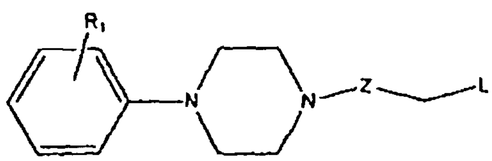

(V)

in welcher $R_1$ und Z wie in Anspruch 1 definiert sind und L für eine austretende Gruppe steht, welche vorteilhafterweise in der Gruppe, welche aus einem Halogen (vorzugsweise einem Brom-, Iod- oder Chloratom), einem O-Mesylat, O-Triflat oder O-Tosylat gebildet wird, ausgewählt wird, mit einem Serotonin-Derivat der allgemeinen Formel (VI)

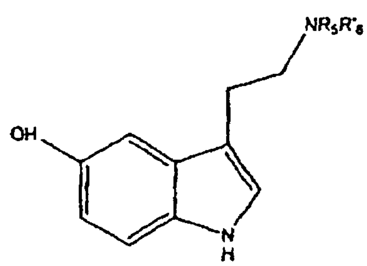

(VI)

in welcher $R'_6$ für $R_6$ steht, wobei $R_6$ wie in Anspruch 1 definiert ist, und $R_5$ wie in Anspruch 1 definiert ist, reagieren lässt.

**7.** Verfahren zur Herstellung der Verbindungen der Formel (I) gemäß Anspruch 1, **dadurch gekennzeichnet, dass** man ein Zwischenprodukt der allgemeinen Formel (V), in welcher $R_1$ und Z wie in Anspruch 1 definiert sind und L für eine austretende Gruppe steht, welche vorteilhafterweise in der Gruppe, welche aus einem Halogen (vorzugsweise einem Brom-, Iod- oder Chloratom), einem O-Mesylat, O-Triflat oder O-Tosylat gebildet wird, ausgewählt wird,
mit einem Serotonin-Derivat der allgemeinen Formel (VI), in welcher $R'_6$ für $CO_2R_8$ steht, wobei $R_8$ wie in Anspruch 3 definiert ist, und $R_5$ wie in Anspruch 1 definiert ist, reagieren lässt und man eine Verbindung der Formel (II) erhält, welche man in eine Verbindung der Formel (I) durch das Verfahren gemäß Anspruch 3 umwandelt.

**8.** Verfahren zur Herstellung der Verbindungen der Formel (I) gemäß Anspruch 1, für welche Z für CO steht und X für $CH_2$ steht, welches darin besteht, eine $\alpha,\beta$-ungesättigte Säure der allgemeinen Struktur (VII)

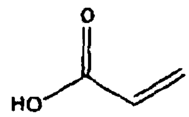

(VII)

nacheinander mit einem 5-Bromindolderivat der allgemeinen Struktur (VIII)

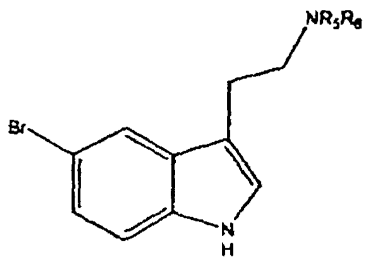

(VIII)

welches derart ist, dass $R_5$ wie in Anspruch 1 definiert ist und $R_6$ wie in Anspruch 1 definiert ist,
und nach Aktivierung der Säurefunktion zu einem Säurechlorid oder aktivierten Ester mit einem Piperazinderivat der allgemeinen Struktur (III) zu kondensieren, gefolgt von der Reduktion der Doppelbindung durch Hydrierung auf Palladium/Kohlenstoff.

**9.** Verfahren zur Herstellung der Verbindungen der Formel (I) gemäß Anspruch 1, für welche $R_1$ für $NH_2$ steht, welches darin besteht, entweder eine Verbindung der Formel (I), in welcher $R_1$ für $NO_2$ steht, zu reduzieren oder eine Verbindung der Formel (II), in welcher $R_1$ für $NO_2$ steht, zu reduzieren, dann die erhaltene Verbindung in eine Verbindung der Formel (I) durch das Verfahren gemäß Anspruch 3 umzuwandeln.

**10.** Verfahren zur Herstellung der Verbindungen der Formel (I) gemäß Anspruch 1, für welche $R_1$ für $NR_5R_6$, $NHSO_2R_7$ steht, wobei $R_5$, $R_6$ und $R_7$ wie in Anspruch 1 definiert sind, welches darin besteht, entweder ein der Formel (I), in welcher $R_1$ für $NH_2$ steht, entsprechendes Derivat zu funktionalisieren oder ein Formel (II), in welcher $R_1$ für $NH_2$ steht, entsprechendes Derivat zu funktionalisieren, dann die erhaltene Verbindung in eine Verbindung der Formel (I) durch das Verfahren gemäß Anspruch 3 umzuwandeln.

**11.** Verfahren zur Herstellung der Verbindungen der Formel (I) gemäß Anspruch 1, **dadurch gekennzeichnet, dass** man eine Verbindung der allgemeinen Formel (I) gemäß Anspruch 1 oder ein Salz oder ein Derivat, welches eine Schutzgruppe einer solchen Verbindung umfasst, in eine andere Verbindung der allgemeinen Formel (I) umwandelt.

**12.** Pharmazeutische Zusammensetzungen, welche als Wirkstoffe eine Verbindung nach einem der Ansprüche 1 und 2 in Kombination mit einem pharmazeutisch annehmbaren Träger umfassen, für die sowohl heilende als auch präventive Behandlung von mit Serotonin in Verbindung stehenden Erkrankungen.

**13.** Pharmazeutische Zusammensetzungen, welche als Wirkstoffe eine Verbindung nach einem der Ansprüche 1 und 2 in Kombination mit einem pharmazeutisch annehmbaren Träger umfassen, für die sowohl heilende als auch präventive Behandlung von Migräne-Krisen, vaskulären Algien des Gesichts und chronischen vaskulären Kopfschmerzen.

**14.** Pharmazeutische Zusammensetzungen, welche als Wirkstoffe eine Verbindung nach einem der Ansprüche 1 und 2 in Kombination mit einem pharmazeutisch annehmbaren Träger umfassen, für die sowohl heilende als auch präventive Behandlung von Depressionen, obsessiv-kompulsiven Erkrankungen oder Störungen, Angst und Panikattacken, Schizophrenie, Aggressivität und/oder Alkoholismus und/oder asozialem Verhalten, Ernährungsstörungen oder -krankheiten, wie Bulimie und Anorexie, sexuellen Dysfunktionen und neurodegenerativen Erkrankungen, wie beispielsweise Parkinson'-Krankheit oder Alzheimer'-Krankheit.

**Claims**

**1.** Compound corresponding to the general formula (I)

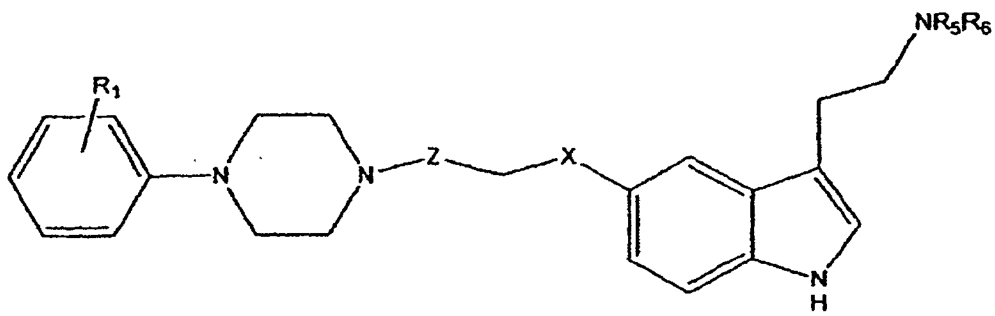

(I)

in which:

$R_1$ represents an $NH_2$, $NO_2$, $NR_5R_6$, $NHSO_2R_7$ or CN radical which can be in the o, m or p position on the aromatic ring
Z represents C=O, $(CH_2)_n$ or alternatively $CO(CH_2)_n$; n being between 1 and 5
X represents $CH_2$ or O
$R_5$ and $R_6$, which are identical or different, each represent a hydrogen atom or a linear or branched alkyl radical comprising from 1 to 6 carbon atoms, preferably a methyl, ethyl, n-propyl, isopropyl, n-butyl, s-butyl or t-butyl
$R_7$ represents a linear or branched alkyl radical comprising from 1 to 5 carbon atoms or a 5-membered heterocycle comprising one or more sulphur atoms

or $R_7$ represents $-NMe_2$ when $R_5$ and $R_6$ represent a hydrogen atom and Z represents C=O,
its salts and solvates which are acceptable for therapeutic use.

**2.** Compound of formula (I) according to Claim 1, selected from:

2-[3-(2-Aminoethyl)-1H-indol-5-yloxy]-1-[4-(4-nitro-phenyl)piperazin-1-yl]ethanone
2-[3-(2-Aminoethyl)-1H-indol-5-yloxy]-1-[4-(4-aminophenyl)piperazin-1-yl]ethanone
N-[4-(4-{2-[3-(2-Aminoethyl)-1H-indol-5-yloxy]acetyl}-piperazin-1-yl)phenyl]methanesulphonamide
Thiophene-2-{N-[4-(4-{2-[3-(2-aminoethyl)-1H-indol-5-yloxy]acetyl}piperazin-1-yl)phenyl]sulphonamide}
3-[3-(2-Aminoethyl)-1H-indol-5-yl]-1-[4-(4-nitro-phenyl)piperazin-1-yl]propan-1-one
3-[3-(2-Aminoethyl)-1H-indol-5-yl]-1-[4-(4-aminophenyl)piperazin-1-yl]propan-1-one
N-[4-(4-{3-[3-(2-Aminoethyl)-1H-indol-5-yl]propionyl}-piperazin-1-yl)phenyl]-N',N'-dimethylsulphamide
4-(4-{2-[3-(2-Aminoethyl)-1H-indol-5-yloxy]-ethyl}piperazin-1-yl)aniline
N-[4-(4-{2-[3-(2-Aminoethyl)-1H-indol-5-yloxy]ethyl}piperazin-1-yl)phenyl]methanesulphonamide
4-(4-{2-[3-(2-Aminoethyl)-1H-indol-5-yloxy]ethyl}-piperazin-1-yl)benzonitrile
2-(4-{2-[3-(2-Aminoethyl)-1H-indol-5-yloxy]-acetyl}piperazin-1-yl)benzonitrile
4-(4-{2-[3-(2-Aminoethyl)-1H-indol-5-yloxy]acetyl}-piperazin-1-yl)benzonitrile

6-[3-(2-Aminoethyl)-1H-indol-5-yloxyl-1-[4-(4-amino-phenyl)piperazin-1-yl]hexan-1-one
N-[4-(4-{6-[3-(2-Aminoethyl)-1H-indol-5-yloxy]hexanoyl}-piperazin-1-yl)phenyl]methanesulphonamide
5-[3-(2-Aminoethyl)-1H-indol-5-yloxy]-1-[4-(4-aminophenyl)piperazin-1-yl]pentan-1-one

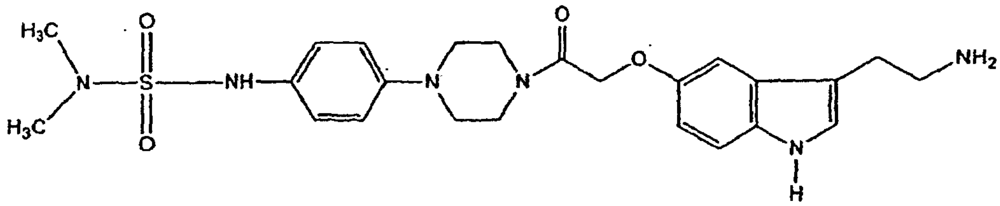

its salts and solvates which are acceptable for therapeutic use.

**3.** Process for the preparation of the compounds of formula (I) according to Claim 1 for which $R_6$ represents a hydrogen, **characterized in that** a carbonate of formula (II)

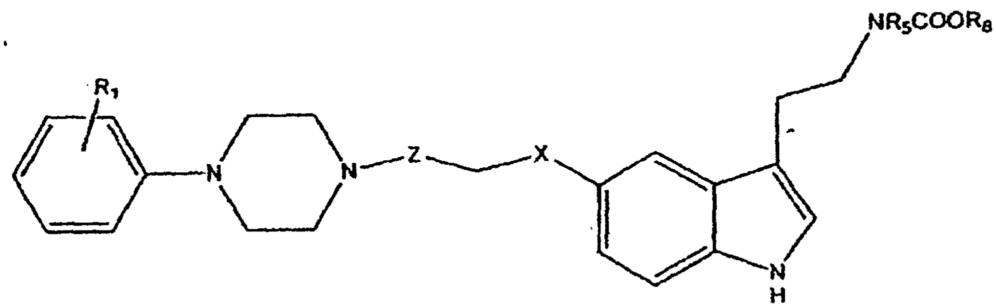

(II)

in which $R_1$, Z, X and $R_5$ are defined as in Claim 1 and $R_8$ represents a t-butyl or benzyl radical, is converted.

**4.** Process for the preparation of the compounds of formula (I) according to Claim 1, **characterized in that** a piperazine of general formula (III)

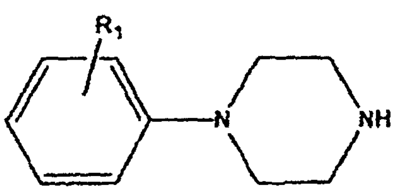

(III)

in which $R_1$ is defined as in Claim 1, is reacted with an indole intermediate of general formula (IV)

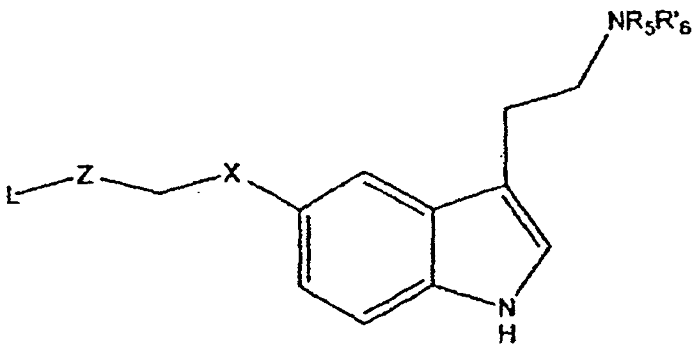

(IV)

in which Z, X and $R_5$ are defined as in Claim 1, $R'_6$ represents $R_6$, $R_6$ being defined as in Claim 1, and L represents a leaving group advantageously chosen from the group consisting of a halogen (chlorine, bromine or iodine) and an O-mesyl, O-trifluoromethylsulphonyl and O-tosyl radical.

**5.** Process for the preparation of the compounds of formula (I) according to Claim 1, **characterized in that** a piperazine of general formula (III) in which $R_1$ is defined as in Claim 1 is reacted with an indole intermediate of general formula (IV) in which Z, X, $R_5$ are defined as in Claim 1, $R'_6$ represents $CO_2R_8$, $R_8$ being defined as in Claim 3, and L represents a leaving group advantageously chosen from the group consisting of a halogen (chlorine, bromine or iodine) and an O-mesyl, O-trifluoromethylsulphonyl and O-tosyl radical, and a compound of formula (II) is obtained which is converted to the compound of formula (I) by the process according to Claim 3.

**6.** Process for the preparation of the compounds of formula (I) according to Claim 1, **characterized in that** an intermediate of general formula (V)

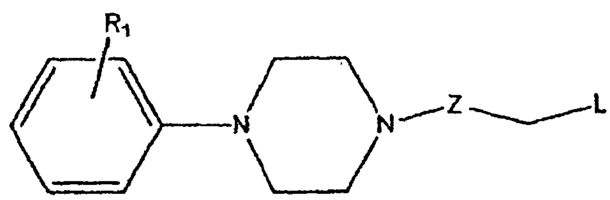

(V)

in which $R_1$ and Z are defined as in Claim 1 and L represents a leaving group advantageously chosen from the group consisting of a halogen (preferably a bromine, iodine or chlorine atom) and an O-mesylate, O-triflate and O-tosylate,
is reacted with a derivative of serotonin of general formula (VI)

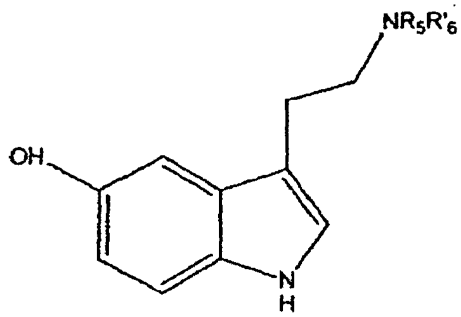

(VI)

in which $R'_6$ represents $R_6$, $R_6$ being defined as in Claim 1, and $R_5$ is defined as in Claim 1.

**7.** Process for the preparation of the compounds of formula (I) according to Claim 1, **characterized in that** an intermediate of general formula (V) in which $R_1$ and Z are defined as in Claim 1 and L represents a leaving group advantageously chosen from the group consisting of a halogen (preferably a bromine, iodine or chlorine atom) and an O-mesylate, O-triflate and O-tosylate
is reacted with a derivative of serotonin of general formula (VI) in which $R'_6$ represents $CO_2R_8$, $R_8$ being defined as in Claim 3, and $R_5$ is defined as in Claim 1, and a compound of formula (II) is obtained which is converted to the compound of formula (I) by the process according to Claim 3.

**8.** Process for the preparation of the compounds of formula (I) according to Claim 1 for which Z represents CO and X represents $CH_2$, which process consists in condensing an α,β-unsaturated acid of general structure (VII)

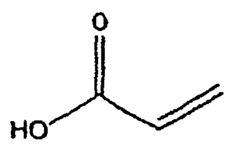

(VII)

successively with a 5-bromoindole derivative of general structure (VIII)

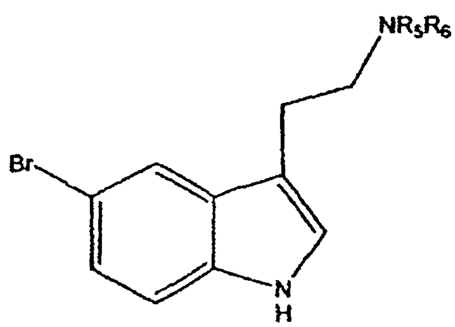

(VIII)

such that $R_5$ is defined as in Claim 1 and $R_6$ is defined as in Claim 1
and, after activation of the acid functional group to activated ester or acid chloride, with a piperazine derivative of general structure (III), followed by the reduction of the double bond by hydrogenation over palladium/charcoal.

**9.** Process for the preparation of the compounds of formula (I) according to Claim 1 for which $R_1$ represents $NH_2$,

which process consists either in reducing a compound of formula (I) in which $R_2$ represents $NO_2$ or in reducing a compound of formula (II) in which $R_1$ represents $NO_2$ and in then converting the compound obtained to a compound of formula (I) by the process according to Claim 3.

**10.** Process for the preparation of the compounds of formula (I) according to Claim 1 for which $R_1$ represents $NR_5R_6$ or $NHSO_2R_7$, $R_5$, $R_6$ and $R_7$ being defined as in Claim 1, which process consists either in functionalizing a corresponding derivative of formula (I) in which $R_1$ represents $NH_2$ or in functionalizing a corresponding derivative of formula (II) in which $R_1$ represents $NH_2$ and in then converting the compound obtained to a compound of formula (I) by the process according to Claim 3.

**11.** Process for the preparation of the compounds of formula (I) according to Claim 1, **characterized in that** a compound of general formula (I) according to Claim 1 or a salt or a derivative comprising a protective group for such a compound is converted to another compound of general formula (I).

**12.** Pharmaceutical compositions comprising, as active ingredients, a compound according to either of Claims 1 and 2 in combination with an acceptable pharmaceutical vehicle for both the curative and preventive treatment of disorders related to serotonin.

**13.** Pharmaceutical compositions comprising, as active ingredients, a compound according to either of Claims 1 and 2 in combination with an acceptable pharmaceutical vehicle for both the curative and preventive treatment of migraine attacks, facial vascular pain and chronic vascular cephalalgias.

**14.** Pharmaceutical compositions comprising, as active ingredients, a compound according to either of Claims 1 and 2 in combination with an acceptable pharmaceutical vehicle for both the curative and preventive treatment of depression, compulsive obsessional disorders, anxiety and panic attacks, schizophrenia, aggressiveness and/or alcoholism and/or asocial behavior, eating disorders, such as bulimia and anorexia, sexual disfunctionings and neurodegenerative diseases, such as, for example, Parkinson's disease or Alzheimer's disease.